# EUROPEAN PATENT APPLICATION

(11) **EP 3 673 985 A1**
(43) Date of publication of application: **01.07.2020**
(21) Application number: 17922095.9
(22) Date of filing: 05.12.2017
(51) Int. Cl.: B01J 19/00, G01N 33/53, G01N 33/68, B01L 3/00

(54) **STORAGE MEDIUM STORING REAGENT AND INSPECTION METHOD AND MODULE USING SAME**

(30) Priority: 23.08.2017 KR 20170106500
(71) Applicant: Noul Co., Ltd., Yongin-si, Gyeonggi-do 16827 (KR)
(72) Inventor: LEE, Dong Young, Yongin-si Gyeonggi-do 16923 (KR); LIM, Chan Yang, Seongnam-si Gyeonggi-do 13481 (KR); KIM, Kyung Hwan, Yongin-si Gyeonggi-do 17103 (KR)
(74) Representative: Dantz, Jan Henning
(86) International application number: PCT/KR2017/014157
(87) International publication number: WO 2019/039664

(57) **Abstract**

The present disclosure relates to an inspection method using a patch and a storage medium, the inspection method including: preparing a storage medium for storing a reagent in a non-activated state, the reagent being used to inspect whether a sample includes a target substance; preparing a patch including a network structure in which multiple microcavities are formed and a base substance that imparts an active condition to the reagent; contacting the storage medium with the patch to transfer a portion of the reagent to the patch; accommodating, in the patch, the transferred reagent put into an activated state by the base substance; and contacting the patch with the sample.

## Description

### TECHNICAL FIELD

The present disclosure relates to a storage medium, a patch for inspecting a sample, and an inspection method and an inspection module using the same, and more particularly to a storage medium, a patch for inspecting a sample, and an inspection method and an inspection module using the same, wherein the storage medium is for storing a substance for inspecting a sample.

### BACKGROUND ART

Due to rapid aging and increasing desire for quality of life, the diagnostics market, which aims at early diagnosis and early treatment, grows every year in the world including Korea. Thus, quick and simple diagnosis is becoming important. In particular, the form of diagnosis is changing to a form, such as in-vitro diagnosis (IVD) or point-of-care testing (POCT), which allows diagnosis without large-scale diagnosis equipment.

In such POCT diagnosis, various reagents are used according to the disease to be diagnosed and the method of diagnosis. These reagents are used in a solution state, and when stored in a solution state in a diagnostic kit, the solution may leak or mix with other solutions. In addition, if a reagent is stored for long periods in a solution state, the reagent may deteriorate. When a reagent is mixed with other solutions or deteriorates, the reliability of a diagnostic result is very poor. Thus, there is a need for a way of storing such reagents for a long period.

Immunochemical diagnosis, which is one of the specific diagnostic fields for performing IVD, is one of the most widely used diagnostic methods, accounting for a large portion of the IVD field. Immunochemical diagnosis refers to diagnosis through clinical immunologic analysis and chemical analysis. In immunochemical diagnosis, antigen-antibody reaction is used, and immunochemical diagnosis is used for diagnosis and tracking of various diseases, such as cancer markers or allergies. Due to its wide range of detectable diseases and the ease of detection, immunochemical diagnosis is assessed as being particularly suitable for on-site diagnosis. The demand for such immunochemical diagnosis is steadily increasing worldwide, especially in China.

### DESCRIPTION OF EMBODIMENTS

### TECHNICAL PROBLEM

In the immunochemical diagnosis described above, an antibody or a substrate is used, and a labeled substance is attached to the antibody. However, in a conventional immunochemical diagnosis environment, an antibody is stored in solution for a long period, resulting in the deterioration of the antibody or the loss of function of the labeled substance attached to the antibody.

Therefore, a method of storing a reagent for immunochemical diagnosis for a long period is required.

### SOLUTION TO PROBLEM

In the present disclosure, by using a storage medium that may store a reagent to be used in diagnosis of a specimen in a non-activated state for a long period, storage of a substance may be improved, and more accurate diagnosis may be possible.

### ADVANTAGEOUS EFFECTS OF DISCLOSURE

According to the present disclosure, a storage medium for storing a substance may be used for long-term storage of a diagnostic reagent, and storage space may be saved by storing the reagent used in the diagnosis in a more compact form.

Also, according to the present disclosure, deterioration of the needed reagent for the inspection of a sample may be prevented, and an amount of the reagent needed for inspecting the sample may be easily controlled.

The effects of present disclosure are not limited to the effects described above, and effects that are not described herein may be clearly understood by those skilled in the art in view of the description and the accompanying drawings.

### BRIEF DESCRIPTION OF DRAWINGS

FIG. 1 illustrates an embodiment of a patch according to the present disclosure;
FIG. 2 illustrates an embodiment of a patch according to the present disclosure;
FIG. 3 illustrates provision of a reaction space as an embodiment of a function of a patch according to the present disclosure;
FIG. 4 illustrates provision of a reaction space as an embodiment of a function of a patch according to the present disclosure;
FIG. 5 illustrates transfer of a substance as an embodiment of a function of a patch according to the present disclosure;
FIG. 6 illustrates transfer of a substance as an embodiment of a function of a patch according to the present disclosure;
FIG. 7 illustrates transfer of a substance as an embodiment of a function of a patch according to the present disclosure;
FIG. 8 illustrates transfer of a substance as an embodiment of a function of a patch according to the present disclosure;
FIG. 9 illustrates transfer of a substance as an embodiment of a function of a patch according to the present disclosure;
FIG. 10 illustrates transfer of a substance as an embodiment of a function of a patch according to the present disclosure;
FIG. 11 illustrates transfer of a substance as an embodiment of a function of a patch according to the present disclosure;
FIG. 12 illustrates transfer of a substance as an embodiment of a function of a patch according to the present disclosure;
FIG. 13 illustrates transfer of a substance as an embodiment of a function of a patch according to the present disclosure;
FIG. 14 illustrates absorption of a substance as an embodiment of a function of a patch according to the present disclosure;
FIG. 15 illustrates absorption of a substance as an embodiment of a function of a patch according to the present disclosure;
FIG. 16 illustrates absorption of a substance as an embodiment of a function of a patch according to the present disclosure;
FIG. 17 illustrates absorption of a substance as an embodiment of a function of a patch according to the present disclosure;
FIG. 18 illustrates absorption of a substance as an embodiment of a function of a patch according to the present disclosure;
FIG. 19 illustrates absorption of a substance as an embodiment of a function of a patch according to the present disclosure;
FIG. 20 illustrates absorption of a substance as an embodiment of a function of a patch according to the present disclosure;
FIG. 21 illustrates absorption of a substance as an embodiment of a function of a patch according to the present disclosure;
FIG. 22 illustrates absorption of a substance as an embodiment of a function of a patch according to the present disclosure;
FIG. 23 illustrates provision of an environment as an embodiment of a function of a patch according to the present disclosure;
FIG. 24 illustrates provision of an environment as an embodiment of a function of a patch according to the present disclosure;
FIG. 25 illustrates provision of an environment as an embodiment of a function of a patch according to the present disclosure;
FIG. 26 illustrates absorption and transfer of a substance as an embodiment of a patch according to the present disclosure;
FIG. 27 illustrates absorption and transfer of a substance as an embodiment of a patch according to the present disclosure;
FIG. 28 illustrates absorption and transfer of a substance as an embodiment of a patch according to the present disclosure;
FIG. 29 illustrates absorption and transfer of a substance as an embodiment of a patch according to the present disclosure;
FIG. 30 illustrates absorption and transfer of a substance as an embodiment of a patch according to the present disclosure;
FIG. 31 illustrates absorption and transfer of a substance and provision of an environment as an embodiment of a patch according to the present disclosure;
FIG. 32 illustrates absorption and transfer of a substance and provision of an environment as an embodiment of a patch according to the present disclosure;
FIG. 33 illustrates an example embodiment of a plurality of patches as an embodiment of a patch according to the present disclosure;
FIG. 34 illustrates an example embodiment of a plate having a plurality of patches and a plurality of target areas as an embodiment of a patch according to the present disclosure;
FIG. 35 illustrates an embodiment of an inspection module according to the present disclosure;
FIG. 36 illustrates an embodiment of a storage medium according to the present disclosure;
FIG. 37 illustrates an embodiment of a storage medium according to the present disclosure;
FIG. 38 illustrates an embodiment of a storage medium according to the present disclosure;
FIG. 39 illustrates a patch and a storage medium according to an embodiment of the present disclosure;
FIG. 40 illustrates a patch and a storage medium according to an embodiment of the present disclosure;
FIG. 41 illustrates a method of inspecting a sample by using a patch PA, a storage medium ME, and a plate PL according to an embodiment of the present disclosure;
FIG. 42 illustrates a method of inspecting a sample by using a patch PA, a storage medium ME, and a plate PL according to an embodiment of the present disclosure;
FIG. 43 illustrates a method of inspecting a sample by using a patch PA, a storage medium ME, and a plate PL according to an embodiment of the present disclosure;
FIG. 44 illustrates a method of inspecting a sample by using a patch PA, a storage medium ME, and a plate PL according to an embodiment of the present disclosure;
FIG. 45 illustrates a method of inspecting a sample by using a patch PA, a storage medium ME, and a plate PL according to an embodiment of the present disclosure;
FIG. 46 illustrates a method of inspecting a sample by using a patch PA, a storage medium ME, and a plate PL according to an embodiment of the present disclosure;
FIG. 47 illustrates a method of inspecting a sample by using a patch PA, a storage medium ME, and a plate PL according to an embodiment of the present disclosure;
FIG. 48 illustrates a method of inspecting a sample by using a patch PA, a storage medium ME, and a plate PL according to an embodiment of the present disclosure;
FIG. 49 illustrates a method of inspecting a sample by using a patch PA, a storage medium ME, and a plate PL according to an embodiment of the present disclosure;
FIG. 50 is a schematic view of an embodiment of transfer of a substance by using a plurality of storage media according to the present disclosure;
FIG. 51 is a schematic view of an embodiment of transfer of a substance by using a plurality of patches PA;
FIG. 52 is a schematic view of an embodiment of transfer of a substance by using a plurality of patches PA;
FIG. 53 is a schematic view of an embodiment of transfer of a substance by using a plurality of storage media and a plurality of patches according to the present disclosure;
FIG. 54 is a flowchart for illustrating a method of inspecting a sample according to an embodiment of the present disclosure;
FIG. 55 is a flowchart for illustrating a method of inspecting a sample according to an embodiment of the present disclosure;
FIG. 56 illustrates an embodiment of an inspection module according to the present disclosure;
FIG. 57 illustrates an embodiment of an inspection device according to the present disclosure;
FIG. 58 illustrates a module for inspecting the sample by using a storage medium and a patch;
FIG. 59 illustrates a module for inspecting the sample by using a storage medium and a patch;
FIG. 60 illustrates a method of performing an immunologic inspection of a sample by using an inspection module according to the present disclosure;
FIG. 61 illustrates a method of performing an immunologic inspection of a sample by using an inspection module according to the present disclosure;
FIG. 62 illustrates a method of performing an immunologic inspection of a sample by using an inspection module according to the present disclosure;
FIG. 63 illustrates a method of inspecting the sample by an indirect method;
FIG. 64 illustrates a method of inspecting the sample by an indirect method;
FIG. 65 illustrates a method of inspecting the sample by an indirect method;
FIG. 67 illustrates an inspection module that sequentially transfers a first substance and a second substance to a patch, as an embodiment of the present disclosure;
FIG. 68 illustrates an inspection module that sequentially transfers a first substance and a second substance to a patch, as an embodiment of the present disclosure;
FIG. 69 illustrates an experimental result of a first Experimental Example according to an embodiment of the present disclosure;
FIG. 70 illustrates an experimental result of a second Experimental Example according to an embodiment of the present disclosure;
FIG. 71 illustrates an experimental result of a third Experimental Example according to an embodiment of the present disclosure;
FIG. 72 illustrates an experimental result of a fourth Experimental Example according to an embodiment of the present disclosure;
FIG. 73 illustrates an experimental result of a fifth Experimental Example according to an embodiment of the present disclosure; and
FIG. 74 illustrates an experimental result of a sixth Experimental Example according to an embodiment of the present disclosure.

### BEST MODE

It is to be understood that the embodiments disclosed herein are for the purpose of clarifying the idea of present disclosure to those skilled in the art to which the present disclosure pertains. Therefore, the present disclosure is not limited by the embodiments described herein, and the scope of the present disclosure should be construed as including modifications or variations that do not depart from the spirit of the present disclosure.

Most of the terms used herein are general terms that have been widely used in the technical art to which the present disclosure pertains. However, some of the terms used herein may be created reflecting intentions of those skilled in the art, precedents, or new technologies. However, if a specific term is defined as having a meaning, the meaning of the term will be described separately. Accordingly, the specific terms used herein should be understood based on the unique meanings thereof throughout the whole context of the present disclosure.

The drawings attached hereto are intended to illustrate the present disclosure easily, and the shapes shown in the drawings may be exaggerated according to a need in order to facilitate understanding of the present disclosure. Thus, the present disclosure is not limited to the drawings.

In the description of the present disclosure, certain detailed explanations of related art are omitted when it is deemed that they may unnecessarily obscure the essence of the present disclosure.

According to an embodiment, an inspection method of a sample may be provided, wherein the inspection method may include preparing a storage medium for storing a reagent in a non-activated state, wherein the reagent is used to inspect whether the sample comprises a target substance; preparing a patch on a side of the storage medium, the patch including a network structure in which multiple microcavities are formed and a base substance is stored in the microcavities, and imparting an active condition to the reagent; contacting the storage medium with a surface of the patch on the side of the storage medium for transferring a portion of the reagent stored in the storage medium to the patch; accommodating the transferred reagent put into an activated state by the base substance in the patch; and contacting the patch with the sample to provide the reagent to a reaction area on which the sample is located.

The inspection method according to an embodiment may further include providing the reagent to a reaction area, on which the sample is located. Here, the providing of the reagent may be performed after the contacting of the patch with the sample.

The accommodating of the transferred reagent put into an activated state may be characterized by storing the reagent put into an activated state in the patch in contact with the sample. The contacting of the patch with the sample may be characterized by contacting the patch accommodating the reagent put into an activated state with the sample.

The contacting of the patch with the sample may include contacting the surface of the patch in contact with the storage medium and another surface of the patch with the sample.

The reagent may be lyophilized in the storage medium.

The activating of the reagent may include contacting the reagent with a base substance to liquefy the reagent. The activated state of the reagent may be a state in which fine particles included in the reagent have mobility. The providing of an active condition for the reagent may include contacting the reagent with the base substance to impart mobility to fine particles included in the reagent. The providing of an active condition for the reagent may include providing a pH condition for the reagent to inspect the target substance.

The reagent may include an antibody for detecting a target protein, and the antibody may be stored in a solid state in the storage medium.

The transferring of the reagent to the patch may include transferring the reagent to a base substance included in the patch.

The contacting of the patch with the sample may include indirectly contacting the patch with the sample through the storage medium in contact with the sample.

The sample may be a biological sample for diagnosing a target disease.

According to another embodiment, an inspection method of a sample may be provided, wherein the inspection method may include preparing a storage medium for storing a reagent in a non-activated state, wherein the reagent is used to inspect whether the sample comprises a target substance; preparing a patch on a side of the storage medium, the patch including a network structure in which multiple microcavities are formed and a base substance imparting an active condition to the reagent; activating the reagent by contacting the storage medium with a surface of the patch on the side of the storage medium; and contacting another surface of the storage medium on a side of the sample with the sample to provide the reagent to a reaction area on which the sample is located.

The inspection method may further include providing the reagent to a reaction area, on which the sample is located, and the providing of the reagent may include transferring the reagent to the patch in contact with the sample. The reagent may be characterized by being provided to the reaction area in an activated state. Here, the providing of the reagent to the reaction area may include providing the reagent put into an activated state by the base substance to the reaction area.

The activating of the reagent may include activating the reagent stored in the storage medium of which the other surface is in contact with the sample.

When the surface of the patch is in contact with the storage medium, a base substance included in the patch and transferred to the storage medium through the surface of the patch may induce the activated reagent to be provided to the sample.

The providing of the reagent to a reaction area, on which the sample is located, may include inducing provision of the reagent by transferring the base substance to the storage medium, upon contact of the surface of the storage medium with the patch.

The contacting of the storage medium with the sample may include contacting the storage medium, of which the surface is in contact with the patch, with the sample.

The reagent may include an antibody for detecting a target protein.

The reagent may be lyophilized in the storage medium.

According to another embodiment of the present disclosure, an inspection method of a sample using a patch and a storage medium may be provided, wherein the storage medium is contacted with the patch for storing a substance to transfer a substance stored in the storage medium to the patch, the patch provides the substance transferred to patch with an active condition, and the substance is provided to a reaction area. A method of inspecting a sample may include preparing a patch including a base substance that activates a reagent and a network structure in which multiple microcavities are formed, wherein the multiple microcavities may store the base substance, the patch being capable of absorbing a substance from an external area or providing a substance to an external area; preparing a storage medium for storing a reagent in a non-activated state, wherein the reagent is used to inspect whether the sample include a target substance; contacting the patch with the storage medium to transfer the reagent to the patch; accommodating the reagent put into an activated state by the base substance in the patch; and contacting the patch with the sample to provide the reagent to the reaction area on which the sample is located.

The inspection method may further include providing the reagent to the reaction area by the patch, and the providing of the reagent to the reaction area may include providing the reagent accommodated in an activated state by the base substance to the reaction area.

The accommodating of the reagent put into an activated state in the patch may include providing fluidity to the reagent by the base substance in the patch.

The storage medium may store the reagent that is lyophilized. The reagent may include an antibody for detecting a target protein.

According to an embodiment of the present disclosure, a gel patch for inspecting a sample may be provided. The patch may include a base substance providing a reagent with an active condition, the base substance being for identifying whether the sample includes a target substance; a network structure in which the multiple microcavities are formed, the multiple microcavities storing the base substance; and a medium contact surface that contacts a storage medium in which the reagent is stored in a non-activated state, wherein the patch receives the reagent from the storage medium, and accommodates the reagent put into an activated state by the base substance.

The patch may further include a sample contact surface that contacts the sample and provides the reagent to a reaction area on which the sample is located.

The medium contact surface may be identical to the sample contact surface. The medium contact surface may be opposite the sample contact surface. The contacting of the patch with the sample may include indirect contacting through the storage medium.

The patch may provide the base substance to the storage medium through the medium contact surface by contacting the storage medium, and the storage medium may provide the reagent put into an activated state by the base substance to a reaction area, on which the sample is located, by contacting the sample.

The accommodating of the reagent put into an activated state in the patch may include providing liquidity to the reagent by contacting of the patch with the base substance.

According to still another aspect of the present disclosure, a storage medium for inspecting a sample may be provided, wherein the inspection is performed using the storage medium and a patch, and the patch may include a base substance and a network structure in which multiple microcavities are formed, the multiple microcavities storing the base substance. The storage medium stores a reagent, which is used to inspect whether the sample includes a target substance, and includes a storing surface storing the reagent in a non-activated state and a patch contact surface that contacts the patch for activating the reagent, and the patch activates the reagent by the base substance, upon contact with storage medium, and provides the activated reagent to a reaction area on which a sample to be inspected is located.

The storage medium may store the reagent that is lyophilized. The reagent contacts the base substance through the patch contact surface and acquires liquidity due to the base substance.

The storage medium may further include a sample contact surface that contacts the sample, and the providing of the activated reagent to the reaction area by the patch is providing the activated reagent to the reaction area through the sample contact surface of the storage medium.

According to still another embodiment of the present disclosure, an inspection module for inspecting a sample may be provided. The inspection module may include a storage medium for storing a reagent in a non-activated state, wherein the reagent is used to inspect whether the sample includes a target substance; a patch including a network structure in which multiple microcavities are formed and a base substance imparting an active condition to the reagent, wherein the patch accommodates the reagent put into an activated state upon contact with the storage medium; and a plate container for containing a plate including a reaction area on which the sample is located and to which the reagent put into an activated state is provided.

The patch may contact the sample to provide the reagent put into an activated state to the reaction area. The patch may provide the reagent to the reaction area upon contact with the sample, while in contact with the storage medium. The patch may accommodate the reagent put into an activated state upon contact with the storage medium, while in contact with the sample, and may provide the reagent to the reaction area. The patch may contact the storage medium to activate the reagent and may induce transfer of the activated reagent to the plate.

The storage medium may provide the activated reagent to the reaction area upon contact with the sample, while in contact with the storage medium. The storage medium may contact the patch, while in contact with the sample.

The activated state of the reagent may be a state in which fine particles included in the reagent have mobility. The reagent may include an antibody for detecting a target protein. The sample may be a biological sample for diagnosing a target disease.

According to still another embodiment of the present disclosure, an inspection module for inspection a sample may include a gel patch, the patch including a first main surface that may receive a reagent, wherein the reagent is used to inspect whether a sample includes a target substance, and a second main surface that may contact the sample to provide the reagent to the sample; a storage medium for storing the reagent in a non-activated state, wherein the storage medium is disposed on the first main surface of the patch; and a plate container including a reaction area on which the sample to be inspected is located, providing the sample to the inspection by contacting the patch, and containing a plate disposed on the second main surface of the patch, wherein the storage medium is moved toward the patch to transfer the reagent to the patch, the patch receives the reagent and activates the reagent, and the patch is moved toward the plate to provide the activated reagent to the reaction area.

The storage medium may be spaced apart from the patch in a first direction by a predetermined distance, and the storage medium is moved in a second direction opposite to a first direction from the patch to transfer the reagent to the patch.

The inspection module may be prepared as an inspection kit for inspecting the sample.

An inspection module including a patch and a storage medium is provided in the specification. The inspection module may store a substance. The inspection module may assist a reaction of a substance. The inspection module may diagnose a biological reagent.

The storage medium may store a transferring substance. The storage medium may transfer the transferring substance to the patch. The storage medium may improve storing ability of the transferring substance. The storage medium may contact the patch. The transferring substance may be stored in the storage medium in a non-activated state.

The patch may contact the storage medium. When the patch contacts the storage medium, the transferring substance may be transferred to the patch. The patch may absorb the transferring substance from the storage medium. The transferring substance may be accommodated in the patch in an activated state. The patch may store the reaction substance. The reaction substance may react with the transferring substance. The patch may provide the transferring substance with an active condition. The patch may store an active substance. The active substance may provide the transferring substance with an active condition.

The inspection module may further include a reaction area. The reaction area may be on a plate. The inspection module may include a plate container containing a plate including the reaction area.

The patch may contact the reaction area. The patch may provide the reaction area with a reaction substance or a transferring substance. The patch may transfer the reaction substance or the transferring substance to the reaction area. The patch may provide the reaction area with a transferring substance in an activated state.

The storage medium may contact the reaction area. The storage medium may contact a sample located on the reaction area. The storage medium may transfer the transferring substance to the reaction area. The storage medium may contact the patch to provide the activated transferring substance to the reaction area.

The reaction area may contact the patch. In the reaction area, a sample to be diagnosed may be located. The sample to be diagnosed may be a biological sample such as a tissue section, blood, cell fluid, urine, or the like.

As described above, when a reaction substance is transferred to a patch using the storage medium, the quality control of the reaction substance may be facilitated. In particular, when the reaction substance is stored in the patch, the reaction substance may be deteriorated in the patch. However, upon use of the reaction substance, by storing the reaction substance in the storage medium and by transferring the reaction substance from the storage medium to the patch, the deterioration of the reaction substance may be minimized. The storage medium may store the reaction substance in a frozen and/or dried state.

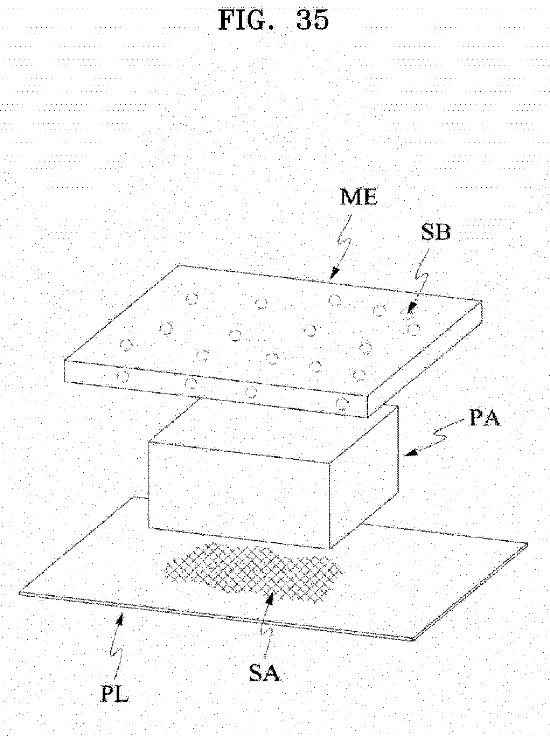

FIG. 35 illustrates an embodiment of an inspection module according to the present disclosure. As shown in FIG. 35, the inspection module according to an embodiment of the present disclosure may include a storage medium ME, a patch PA, and a plate PL. The inspection module may use the reaction substance SB stored in the storage medium to diagnose the sample SA located in the plate PL.

the inspection module according to an embodiment of the present disclosure may include the storage medium ME, and the storage medium ME may store the substance SB. The storage medium ME may store the substance SB in a non-activated state. The storage medium ME may be disposed on the patch PA. As the storage medium ME contacts the upper surface of the patch PA, the storage medium ME may transfer the substance SB to the patch. The inspection module may include a storage medium container for containing the storage medium ME.

The inspection module according to an embodiment of the present disclosure may include the patch PA, and the patch PA may accommodate the substance SB in an activated state. The patch may contact the sample SA for inspection of the sample SA by the substance SB in an activated state to provide the the substance SB in an activated state to the reaction area on which the sample SA is located.

The inspection module according to an embodiment of the present disclosure may include the plate PL, and the plate PL may include a reaction area on which the sample SA to be inspected is located. The inspection module may include a plate container containing the plate PL.

The inspection module disclosed in this specification is not limited to the foregoing description, and details of each of the inspection module and each component constituting the inspection module will be described in more detail in the following.

### Patch

### Significance of patch

The present disclosure relates to a patch for managing a liquid substance.

The liquid substance may refer to a substance in a liquid state that may flow.

The liquid substance may be a substance of a single component having liquidity. The liquid substance may be a mixture including a plurality of liquid substances.

When the liquid substance consists of a single component, the liquid substance may be a substance consisting of a single element or a mixture including a plurality of chemical elements.

When the liquid substance is a mixture, some of the plurality of substances may serve as solvents, and the others may serve as solutes. That is, the mixture may be a solution.

A substance of a plurality of components constituting the mixture may be uniformly distributed. A mixture including a substance of a plurality of components may be uniformly mixed.

The substance of a plurality of components may include a substance that is insoluble to solvents such as the solvent and uniformly distributed.

Some of the substance of a plurality of components may be nonuniformly distributed. The nonuniformly distributed substance may include a particle component that is nonuniformly distributed in the solvent.

At this time, the nonuniformly distributed particle component may be in a solid phase.

For example, the substance that may be handled using the patch may be 1) in a state of a liquid of a single component, 2) in a state of a solution, or 3) in a state of a colloid, and in some cases, 4) in a state in which solid particles are nonuniformly distributed in another liquid substance.

Hereinafter, the patch according to the present disclosure will be described in more detail.

### General nature of patch

### Feature

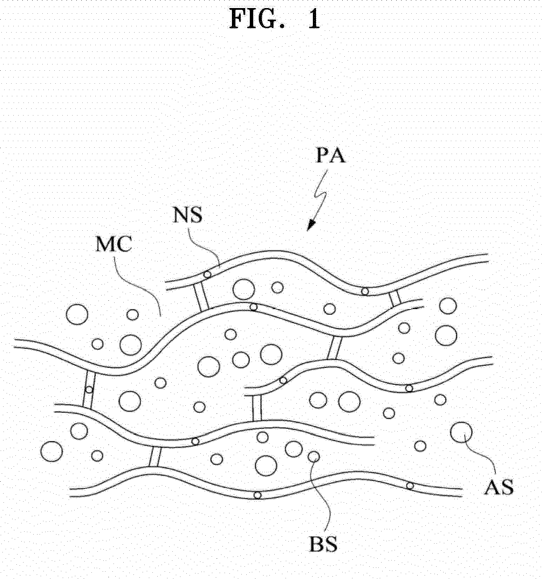

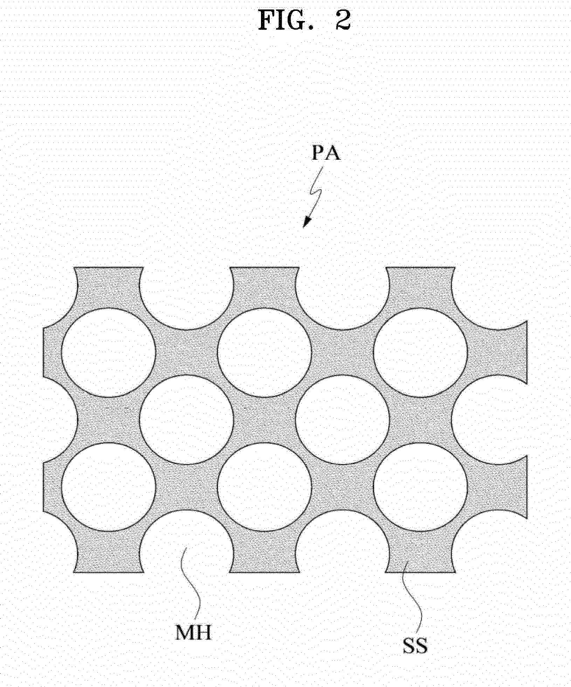

FIGS. 1 and 2 each illustrate an embodiment of a patch according to the present disclosure.

Hereinafter, referring to FIGS. 1 and 2, the patch according to the present disclosure will be described.

Referring to FIG. 1, a patch PA according to the present disclosure may include a network structure NS and a liquid substance.

Here, a liquid substance may be considered as divided into a base substance BS and an additive substance AS.

The patch PA may be in a gel phase (or a gel type). The patch PA may be realized as a gel phase structure in which colloidal molecules are bound to form a network structure.

The patch PA according to the present disclosure may include a three-dimensional network structure NS as a structure for handling the liquid substance SB. The network structure NS may be a solid structure distributed continuously. The network structure NS may have a mesh-like network structure in which a plurality of fine threads are intertwined. However, the network structure NS is not limited to a mesh-like network structure in which a plurality of fine threads are intertwined. The network structure NS but may be implemented in any three-dimensional matrix formed by connecting a plurality of microstructures.

For example, the network structure NS may be a frame that contains several microcavities. In other words, the network structure NS may form several microcavities MC.

FIG. 2 illustrates a patch in a different structure according to the present disclosure. Referring to FIG. 2, the network structure of the patch PA may have a sponge structure SS. In this case, the network structure in the sponge structure SS may include several microholes MH. Hereinafter, the microhole MH and the microcavity MC may be used interchangeably with each other, and unless otherwise stated, the microcavity MC is defined as including the concept of the microhole MH.

In addition, the network structure NS may have a regular pattern or an irregular pattern.

Furthermore, the network structure NS may include both an area having a regular pattern and an area having an irregular pattern.

The density of the network structure NS may have a value within a predetermined range. Preferably, the predetermined range may be determined to within a range such that the shape of the liquid substance SB captured in the patch PA is maintained in a form corresponding to the patch PA. The density may be defined as a degree of fineness of the network structure NS or a mass ratio, a volume ratio, or the like occupied by the network structure NS in the patch.

The patch according to the present disclosure may handle the liquid substance SB by having a three-dimensional network structure.

The patch PA according to the present disclosure may include the liquid substance SB, the liquid substance SB included in the patch PA may have limited liquidity due to the network structure NS of the patch PA.

The liquid substance SB may flow freely within the network structure NS. In other words, the liquid substance SB may be located in several microcavities formed by the network structure NS. Flow of the liquid substance SB may occur between neighboring microcavities. In this case, the liquid substance SB may be present in a form permeating the frame structure forming the net structure. In such a case, nanosize pores, through which the liquid substance SB may permeate, may be formed in the frame structure.

Further, depending on the molecular weight or particle size of the liquid substance SB captured in the patch PA, whether or not the liquid substance SB is injected into the frame structure of the network structure may be determined. A substance having a relatively large molecular weight may be captured by the microcavity and a substance having a relatively small molecular weight may be injected into the frame structure of the microcavity and/or the network structure NS and captured.

The term "captured" as used herein may be defined as meaning that the liquid substance SB is located in a plurality of microcavities and/or pores of a nanosize formed by the network structure NS. In addition, the state in which the liquid substance SB is captured by the patch PA is defined as including a state in which the liquid substance SB may flow between microcavities and/or pores of a nanosize, as described above.

The liquid substance SB may be considered as divided into a base substance BS and an additive substance AS.

The base substance BS may be the liquid substance SB having liquidity.

The additive substance AS may be a substance having liquidity by being mixed with the base substance BS. In other words, the base substance BS may be a solvent. The additive substance AS may be a solute dissolved in the solvent or a particle insoluble in the solvent.

The base substance BS may be a substance capable of flowing in the matrix formed by the network structure NS. On the other hand, the base substance BS may be uniformly distributed in the network structure NS, and may be distributed only in some areas of the network structure NS. The base substance BS may be a liquid having a single component.

The additive substance AS may be mixed with the base substance BS or be dissolved in the base substance BS. For example, the additive substance AS may serve as a solute while using the base substance BS as a solvent.

The additive substance AS may be uniformly distributed in the base substance BS.

The additive substance AS may be a fine particle that does not dissolve in the base substance BS.

For example, the additive substance AS may include fine particles such as colloidal particles, microorganisms, or the like.

The additive substance AS may include particles that are larger than the microcavities formed by the network structure NS. When a size of the microcavities is smaller than a size of the particles included in the additive substance AS, liquidity of the additive substance AS may be limited.

In addition, in some embodiments, the additive substance AS may include components that are optionally included in the patch PA.

The additive substance AS does not necessarily imply a substance that is either deficient in quantitative or functionally inferior in relation to the base substance BS described above.

Hereinafter, the characteristics of the liquid substance SB captured in the patch PA may be regarded as the characteristics of the patch PA.

That is, the characteristics of the patch PA may depend on the characteristics of the substance captured in the patch PA.

### Characteristics

The patch PA according to the present disclosure may include the network structure NS. The patch PA may handle the liquid substance SB by the network structure NS. The patch PA may allow the liquid substance SB captured in the patch PA to at least partially retain its inherent properties.

For example, substance diffusion may occur in the area of the patch PA where the liquid substance SB is distributed, and a force such as surface tension may act.

The patch PA may provide a liquid environment in which a subject substance diffuses due to thermal motion, density, or concentration difference of a substance. In general, the term 'diffusion' means that particles forming the substance due to the difference in concentration spread from a higher concentration side to a lower concentration side. This diffusion phenomenon may be understood as a result that is basically caused by the motion of a molecule (translational motion in gas or liquid, oscillation motion in solid, etc.). In the present application, the term 'diffusion' refers to a phenomenon in which particles are diffused from a higher concentration to a lower concentration due to a difference in concentration or density. Also the term 'diffusion' refers to a phenomenon of movement of particles due to irregular motion of molecules even in a uniform state. Also, the expression 'irregular motion' of particles is used in the same meaning as 'diffusion' unless otherwise stated. The substance to be diffused may be a solute dissolved in the liquid substance SB, and the solute may be provided in a solid, liquid or gaseous state.

More particularly, nonuniformly distributed substances in the liquid substance SB captured by the patch PA may diffuse in the space provided by the patch PA. In other words, the additive substance AS may diffuse in the space defined by the patch PA.

The nonuniformly distributed substance or the additive substance AS in the liquid substance SB handled by the patch PA may diffuse in microcavities provided by the network structure NS in the patch PA. In addition, an area where the nonuniformly distributed substance or the additive substance AS may diffuse may change when the patch PA contacts or connects to other substances.

Furthermore, even after the unevenly distributed substance or the additive substance AS diffuses in the patch PA or in an external area connected to the patch PA, and a concentration of the substance or the additive substance AS becomes uniform, the substance or the additive substance AS may constantly be moved by the irregular motion of molecules in the interior of the patch PA and/or in the external area connected to the patch PA.

The patch PA may be implemented to have hydrophilic or hydrophobic properties. In other words, the network structure NS of the patch PA may have hydrophilic or hydrophobic properties.

When the network structure NS and the liquid substance SB have similar properties, the network structure NS may handle the liquid substance SB more effectively.

The base substance BS may be a hydrophilic substance with polarity or a hydrophobic substance without polarity. The additive substance AS may be hydrophilic or hydrophobic.

The properties of the liquid substance SB may be related to the base substance BS and/or the additive substance AS. For example, when the base substance BS and the additive substance AS are both hydrophilic, the liquid substance SB may be hydrophilic. For example, when the base substance BS and the additive substance AS are both hydrophobic, the liquid substance SB may be hydrophobic. When the polarities of the base substance BS and the additive substance AS are different from each other, the liquid substance SB may be either hydrophilic or hydrophobic.

When the polarities of the network structure NS and the liquid substance SB are both hydrophilic or hydrophobic, attractive force between the network structure NS and the liquid substance SB may be created. When the polarity of the network structure NS is opposite to that of the liquid substance SB, for example, when the network structure NS is hydrophobic, and the liquid substance SB is hydrophilic, repulsive force between the network structure NS and the liquid substance SB may be created.

Based on the above properties, the patch PA may be used alone, in combination, or in conjunction with other media to derive a desired reaction.

Hereinafter, functional aspects of the patch PA will be described.

Hereinafter, for convenience of explanation, it is assumed that the patch PA is a gel phase in which a hydrophilic solution may be included. In other words, the network structure NS of the patch PA is assumed to have a hydrophilic property, unless otherwise stated.

However, the scope of the present disclosure should not be construed as being limited to a gel phase patch PA having hydrophilic property. In addition to the gel phase patch PA containing the hydrophobic property, the scope of the present disclosure may be extended to a patch PA without a solvent and a sol patch PA as well, when the function according to the present disclosure is possible to be implemented.

### Function of patch

The patch according to the present disclosure may have some useful functions due to the above-mentioned characteristics. In other words, the patch may occupy the liquid substance SB and be related to behavior of the liquid substance SB.

Accordingly, hereinafter, according to the behavior of the substance in relation to the patch PA, a reservoir function in which the state of the substance is defined in a predetermined area formed by the patch PA and a channeling function in which the state of the substance is defined by including an external area of the patch PA.

### Reservoir

### Significance

The patch PA according to the present disclosure may capture the liquid substance SB as described above. In other words, the patch PA may serve as a reservoir.

The patch PA may capture the liquid substance SB by the network structure NS in a plurality of microcavities formed in the network structure NS. The liquid substance SB may occupy at least a portion of the microcavities formed by the three-dimensional network structure NS of the patch PA or penetrate into pores of nanosize formed in the network structure NS.

Even if the liquid substance SB located in the patch PA is distributed in multiple microcavities, the properties of the liquid are not lost. That is, the liquid substance SB has liquidity in the patch PA, a substance may be diffused in the liquid substance SB distributed in the patch PA, and the solute suitable for the substance may be dissolved.

Hereinafter, the reservoir function of the patch PA will be described in more detail.

### Storage (Containing)

In the present disclosure, the patch PA may capture a target substance according to the above-described characteristics. The patch PA may have resistance within a certain range with respect to changes in an external environment. Thus, the patch PA may retain the substance in a captured state. The liquid substance SB to be captured may occupy the three-dimensional network structure NS.

Hereinafter, such a function of the patch PA is referred to as storage for convenience.

The meaning of the patch PA storing the liquid substance means both that the liquid substance is stored in the space formed by the network structure, and/or that the liquid substance is stored in the frame structure forming the network structure NS.

The patch PA may store the liquid substance SB. For example, due to the attractive force between the network structure NS of the patch PA and the liquid substance SB, the patch PA may store the liquid substance SB. The liquid substance SB may be bound to the network structure NS by the attractive force stronger than a predetermined level. and be stored therein.

The properties of the liquid substance SB stored in the patch PA may be classified according to the properties of the patch PA. More particularly, when the patch PA is hydrophilic, the patch PA may be combined with a hydrophilic liquid substance SB having polarity, and the hydrophilic liquid substance SB may be stored in three-dimensional microcavities. Alternatively, if the patch PA is hydrophobic, the hydrophobic liquid substance SB may be stored in three-dimensional microcavities of the network structure NS.

Also, the amount of substance that may be stored in the patch PA may be proportional to the volume of the patch PA. In other words, the amount of substance stored in the patch PA may be proportional to the amount of the three-dimensional network structure NS as a support contributing to the shape of the patch PA. However, the relationship between the amount of the substance that may be stored and the volume of the patch PA do not have a specific proportional constant, and the relationship between the amount of the substance that may be stored and the volume of the patch PA may vary depending on the design or manufacturing method of the network structure.

The amount of substance stored in the patch PA may be reduced over time by evaporation, dropout, etc. In addition, a substance may be added to the patch PA to increase or maintain the content of the substance stored in the patch PA. For example, the patch PA may contain a water retention agent for suppressing evaporation of water.

The patch PA may be realized in a form easy to store the liquid substance SB. This means that the patch PA may be implemented in order to minimize denaturation of the substance when the substance is affected by the environment such as humidity, light quantity, temperature, and the like.

For example, to prevent the patch PA from being denatured by external factors such as bacteria, the patch PA may be treated with a bacterial inhibitor or the like.

The patch PA may store the liquid substance SB having a plurality of components. In this embodiment, a substance of a plurality of components may be located in the patch PA before a reference time, or a primary substance is stored first in the patch PA, and a secondary substance is stored in the patch PA after a predetermined time elapses. For example, when the liquid substance SB of two components is stored in the patch PA, the two components may be stored in the patch PA in the time of manufacture of the patch PA, only one component is stored in the patch PA in the time of manufacture of the patch PA, and the other one may be stored therein later, or two components may be sequentially stored in the patch PA after the patch PA is manufactured.

In addition, the substance stored in the patch PA may basically exhibit liquidity as described above, and irregular motion or diffusion motion due to molecular motion may be performed in the patch PA.

The patch PA according to the present disclosure may store an antibody AB, a substrate SU, a wash solution, or a base substance BS or additive substance AS for other immunological diagnosis as described above. The substances described above, particularly the additive substance AS, may be stored in a separate medium and may be absorbed and stored in the patch PA upon diagnosis. The patch PA may absorb and store a substance such as the antibody AB or the substrate SU in a separate medium. The additive substance AS may be necessarily stored in the patch PA in advance.

In particular, this feature may be applied to the patch PA storing the antibody AB. When the antibody AB is stored in a separate medium as described above, the storage stability of the antibody AB is remarkably improved, and the quality of the antibody AB used in the diagnosis may be guaranteed to be higher than a certain level.

The patch PA according to the present disclosure may be absorbing and storing the antibody AB applied to the plate PL. The absorbing of the antibody AB applied to the plate PL may be performed by that the antibody AB is captured in a water film WF formed by contacting the patch PA with the plate PL, and the patch PA is separated from the plate PL, thus moving the water film WF with the patch PA.

The patch PA according to the present disclosure may absorb the antibody AB from the plate coated with the antibody AB, and transfer the absorbed antibody AB to the plate PL coated with the sample SA. This may be performed by bringing a bottom surface of the patch PA into contact with the plate PL coated with the sample SA, while the plate PL coated with the antibody AB is in contact with an upper surface of the patch PA.

The separate medium described above will be described in more detail in the following storage medium part.

### Provision of reaction space

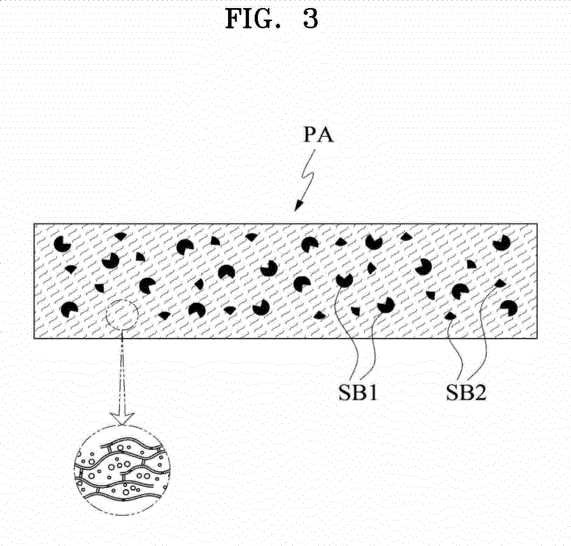

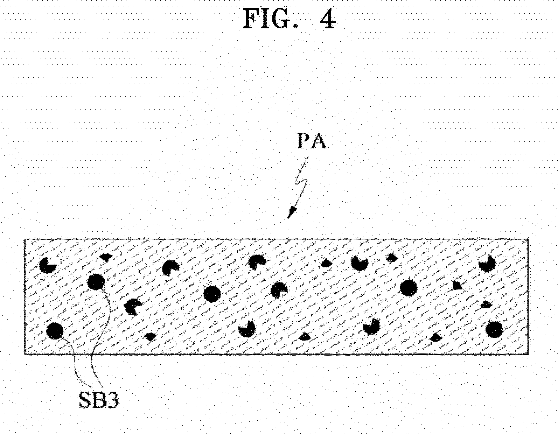

FIGS. 3 and 4 each illustrate provision of a reaction space as an embodiment of a function of a patch according to the present disclosure.

As shown in FIGS. 3 and 4, the patch PA according to the present disclosure may provide a space. In other words, the patch PA may provide a space, through which the liquid substance SB may move, wherein the space is formed by the network structure NS and/or the space constitutes the network structure NS.

The patch PA may provide space for activities other than diffusion of particles and/or irregular motion of particles (hereinafter referred to as non-diffusion activities). The non-diffusion activities may mean chemical reactions, but not necessarily limited thereto, and also mean changes in a physical state. More particularly, non-diffusion activities include chemical reactions in which a chemical composition of the substance changes before and after the action, a specific binding reaction between the components included in the substance, homogenization of solutes or particles included in the substance that are nonuniformly distributed, agglomeration of some components included in the substance, or biological activity of the substance.

When a plurality of substances are involved in the activity, a plurality of substances may be co-located in the patch PA prior to the baseline point. A plurality of substances may be injected sequentially.

By changing the environmental condition of the patch PA, the efficiency of the patch PA regarding providing a space for the non-diffusion activities may be improved. For example, the temperature condition of the patch PA may be changed or an electrical condition may be added to facilitate the activity or induce the initiation of activity.

As shown in FIGS. 3 and 4, a first substance SB1 and a second substance SB2 located in the patch PA may be transformed into a third substance SB3 by reaction inside the patch PA or may form the third substance SB3.

### Channel

### Significance

Substance migration may occur between the patch PA and the external area. Also, the substance may migrate from the patch PA to an external area of the patch PA, or the substance may migrate from the external area to the patch PA.

The patch PA may form a substance migration path or participate in substance migration. In particular, the patch PA may participate in the movement of the liquid substance SB captured in the patch PA or participate in the movement of an external substance through the liquid substance SB captured in the patch PA. The base substance BS or the additive substance AS may flow out of the patch PA, or an external substance may be introduced from the external area into the patch PA.

The patch PA may provide a function of a substance migration path. That is, the patch PA may participate in substance migration and provide a function as a channel for substance migration. The patch PA may provide a channel for substance migration due to the inherent properties of the liquid substance SB.

The patch PA may have a state in which the liquid substance SB may move between the patch PA and the external area or a state in which the liquid substance SB is immovable between the patch PA and the external area, depending on whether the patch PA is connected to the external area.

In addition, when channeling between the patch PA and the external area is initiated, the patch PA may have unique functions.

Hereinafter, a state in which the substance is movable and a state in which the substance may not move will be described first. Thereafter, detailed description in performing unique functions of the patch PA will be described in connection with whether the patch PA is connected to the external area.

Basically, the fundamental reason for the movement of the liquid substance SB between the patch PA and the external area is due to irregular motion and/or diffusion of the substance. However, it has already been described that it is possible to control external environmental factors (for example, control of temperature condition, control of electrical condition, etc.) in order to control the movement of substances between the patch PA and the external area.

### Movable state

In a state in which the substance is movable, a flow may be incurred between the liquid substance SB captured in the patch PA and/or the substance located in the external area. In a state in which the substance is movable, a movement of a substance may be incurred between the liquid substance SB captured in the patch PA and the external area.

For example, in a state in which the substance is movable, the liquid substance SB or some of components of the liquid substance SB may diffuse to the external area or move by irregular motion. Alternatively, in a state in which the substance is movable, the external substance located in the external area or some components of the external substance may diffuse to the liquid substance SB of the patch PA or move by irregular motion.

A state in which the substance is movable may be incurred by a contact. The contact may refer to connecting the liquid substance SB captured in the patch PA to the external area. The contact may mean that the flow area of the liquid substance SB overlaps at least partially with the external area. The contact may mean that the external substance is connected to at least a portion of the patch PA. A state in which the substance is movable may be understood as extension of a range in which the captured liquid substance SB may flow. In other words, in a state in which the substance is movable, the range, in which a liquid substance may flow, may be expanded to include at least a portion of the external area of the captured liquid substance SB.

For example, when the liquid substance SB is contacted with the external area, the range in which the captured liquid substance SB may flow may be expanded to include at least a portion of the contacted external area. In particular, when the external area is an outer plate, an area in which the liquid substance SB may flow may be expanded to include an area in contact with the liquid substance SB of the outer plate.

### Immovable state

In a state in which the substance is immovable, a movement of a substance may not be incurred between the liquid substance SB captured in the patch PA and the external area. A movement may be incurred in each of the liquid substance SB captured in the patch PA and the substance located in the external area.

A state in which the substance is immovable may be a state in which the contact is disconnected. In other words, when the patch PA is disconnected from the external area, the substance may not move in the liquid substance SB remaining in the patch PA and in the external area or the external substance.

In particular, a state in which the contact is disconnected may refer to a state in which the liquid substance SB captured in the patch PA is disconnected from the external area. The state in which the contact is disconnected may mean that the liquid substance SB is not connected to an external substance located in the external area. For example, a state in which the substance is immovable may be caused by the separation of the patch PA from the external area.

The 'movable state' defined in the present specification has a meaning distinct from the 'immovable state', but transition between states may occur due to time flow, environmental change, and the like. In other words, the state of the patch PA may change from a movable state to an immovable state, the state of the patch PA may change from an immovable state to a movable state, or the state of the patch PA may change from a movable state to an immovable state and then to a movable state.

### Classification of functions

### Transfer

In the present disclosure, the patch PA may transfer at least a portion of the liquid substance SB occupied by the patch PA to a desired external area due to the above-mentioned characteristics. The transfer of substance may mean that a portion of the liquid substance SB captured in the patch PA is separated from the patch PA as a predetermined condition is satisfied.

Partial separation of the liquid substance SB may mean that some substances are extracted or emitted or released from the area affected by the patch PA. This may be understood as a sub-concept of the channeling function of the patch PA described above, which defines transfer (delivery) of a substance located in the patch PA to the outside of the patch PA.

The desired external area may be another patch PA, a dried area, or a liquid area.

The predetermined condition for generating the transfer may be determined by an environmental condition such as a temperature change, a pressure change, an electrical property change, and a physical state change. For example, when the patch PA is in contact with an object having stronger binding force with the liquid substance SB than the network structure NS of the patch PA, the liquid substance SB may be chemically bonded with the object in contact with the patch PA, and consequently, at least a portion of the liquid substance SB may be transferred to the object.

Hereinafter, such a function of the patch PA is referred to as transfer (delivery) for convenience.

The transfer may occur when the liquid substance SB is movable between the patch PA and the external area and the liquid substance SB is passed via/through the immovable state between the patch PA and the external area.

In particular, when the liquid substance SB is in the movable state, the liquid substance SB may be diffused between the patch PA and the external area or be moved to the external area by irregular motion. In other words, the base substance and/or the additive substance AS included in the liquid substance SB may move from the patch PA to the external area. In the immovable state of the liquid substance SB, movement between the patch PA and the external area may not be possible. In other words, some of the substances that have migrated from the patch PA to the external area due to the diffusion and/or irregular motion of the liquid substance SB may not migrate back to the patch PA due to the transition from the movable state to the immovable state. Thus, some of the liquid substance SB may be partially transferred to the external area.

The transfer may be performed depending on a difference between an attractive force between the liquid substance SB and the network structure NS and an attractive force between the liquid substance SB and the external area or the external substance. The attractive force may be attributed to similarity in polarity or a specific binding relationship.

In particular, when the liquid substance SB is hydrophilic, and the external area or the external substance is more hydrophilic than the network structure NS of the patch PA, at least a portion of the liquid substance SB captured in the patch PA through the movable state and the immovable state may be transferred to the external area.

The transfer of the liquid substance SB may also be performed selectively. For example, when a specific binding relationship between some components included in the liquid substance SB and the external substance is acting, selective transfer of some components may occur through a state in which the substance is movable and a state in which the the substance is immovable.

In particular, assuming that the patch PA transfers a substance to the outer plate PL in the form of a plate, a substance that specifically binds to a portion (for example, a part of the solute) of the liquid substance SB captured in the patch PA may be applied to the outer plate PL. In this embodiment, the patch PA may selectively transfer the part of the solute specifically binding to the substance coated on the outer plate PL to the plate PL from the patch PA through the movable state and the immovable state.

Transfer will now be described in detail as a function of the patch PA, according to some examples of other areas where the substance is movable. The concept of "release" of the liquid substance SB and "transfer" of the liquid substance SB may be used interchangeably with each other in the detailed description.

Here, a case where the liquid substance SB is transferred from the patch PA to a separate outer plate PL will be described. For example, in the patch PA, a substance may migrate to a plate PL such as a slide glass.

As the patch PA and the plate PL are in contact with each other, the liquid substance SB captured in the patch PA may be at least partially diffused into or may be moved by irregular motion to the plate PL.

When the patch PA and the plate PL are separated from each other, some substances (that is, a portion of the liquid substance SB) that have been moved from the patch PA to the plate PL may not move back to the patch PA. As a result, some substances may be transferred from the patch PA to the plate PL. In this embodiment, some substances that are transferred may be the additive substances AS. In order for the substance in the patch PA to be 'transferred' by contact and separation, an attractive force and/or binding force acting between the substance and the plate PL is present, and the attractive force and/or binding force is greater than the attractive force acting between the substance and the patch PA. Therefore, when the transfer condition is not satisfied, transfer of substance between the patch PA and the plate PL may not occur.

In addition, the transfer of substance may be controlled by providing temperature or electrical condition to the patch PA.

The movement of a substance from the patch PA to the plate PL may depend on a contact area between the patch PA and the plate PL. For example, efficiency of substance migration of the patch PA and the plate PL may be increased or decreased depending on an area in which the patch PA contacts the plate PL.

When the patch PA includes a plurality of components, only some components may be selectively moved to the outer plate PL. In particular, a substance that specifically binds to some components of the plurality of components may be fixed to the outer plate PL. At this time, the substance fixed to the outer plate PL may be in a liquid or solid state, and may be fixed in a separate area. In this case, some substances of the plurality of components are moved to the plate PL by contact of the patch PA with the separate area to form a specific binding. When the patch PA is separated from the plate PL, only some components may be selectively released to the plate PL.

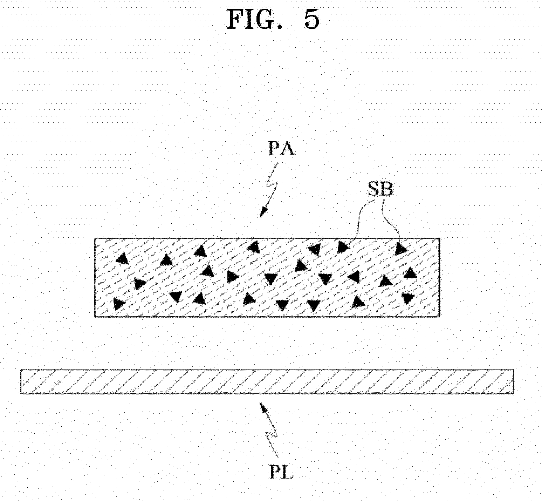

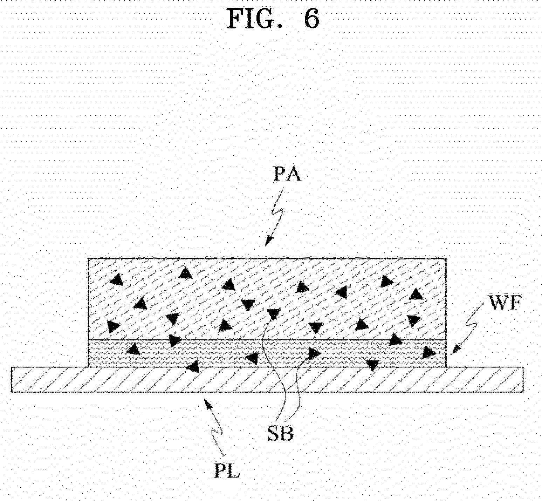

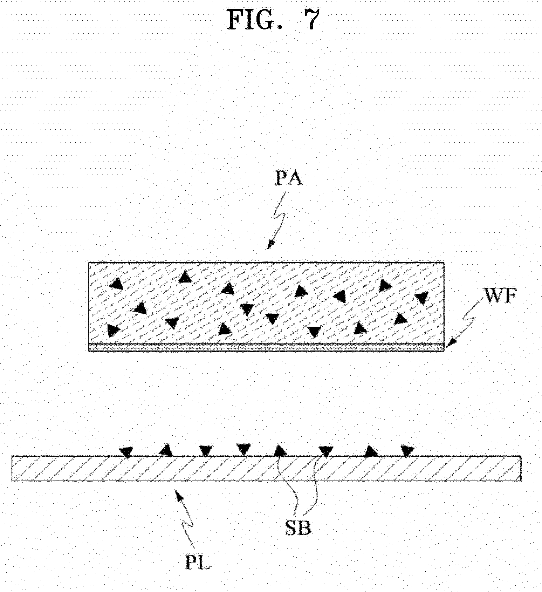

FIGS. 5 to 7 each illustrate transfer of a substance as an embodiment of a function of the patch PA according to the present disclosure. FIGS. 5 to 7 each illustrate transfer of a substance from the patch PA to the outer plate PL. As shown in FIGS. 5 to 7, when the patch PA contacts the outer plate PL, some substances stored in the patch PA may be transferred to the plate PL. At this time, transfer of the substance may be possible by moving the substance by contacting the plate. At this time, the water film WF may be formed near a contact surface where the plate and the patch PA are in contact with each other, and the substance may migrate through the formed water film WF.

Here, a case, where the liquid substance SB as a substance having liquidity SL is transferred from the patch PA, will be described. Here, the substance having liquidity SL may be a liquid substance flowing or contained in a separate storage space.

As the patch PA and the substance having liquidity SL are in contact with each other (for example, by adding the patch PA to a solution), the liquid substance SB captured in the patch PA may be at least partially diffused into or may be moved by irregular motion to the substance having liquidity SL. When the patch PA and the substance having liquidity SL are separated from each other, a portion of the liquid substance SB which has moved from the patch PA to the substance having liquidity SL may not return to the patch PA, thereby transferring some substances in the patch PA to the substance having liquidity.

The movement of a substance from the patch PA to the substance having liquidity SL may depend on a contact area between the patch PA and the substance having liquidity SL. For example, depending on a contact area between the patch PA and the substance having liquidity SL (for example, a depth of the patch PA injected into a solution), efficiency of substance migration between the patch PA and the substance having liquidity SL may increase or decrease.

The movement of a substance from the patch PA to the substance having liquidity SL may be controlled by physical separation between the patch PA and the substance having liquidity SL.

The additive substance AS may be transferred from the patch PA to the substance having liquidity as a distribution concentration of the additive substance AS in the liquid substance SB differs from a distribution concentration of the additive substance AS in the substance having the liquidity.

However, when the patch PA transfers the liquid substance SB to the substance having liquidity SL, physical separation between the patch PA and the substance having liquidity SL is not essential. For example, when a driving force/causal force causing movement of a substance from the patch PA to the liquid having liquidity becomes smaller than or equal to a reference value, the movement of a substance may be stopped.

In the 'transfer' of a substance from the patch PA to the substance having liquidity SL, a 'transfer condition' between the patch PA and the substance having liquidity SL may not be needed. Substances that have already migrated to the substance having liquidity SL are moved by diffusion and/or irregular motion within the substance having liquidity SL, and a distance between the substance moved and the patch PA is longer than a certain distance, it may be understood that the substance has been transferred to the substance having liquidity SL. This is because, in the case of the plate PL, since the movable range extended by the contact is in a very limited range, an attractive force between the substances moved to the plate PL and the patch PA may act significantly, however, in the relationship between the substance having liquidity SL and the patch PA, the movable range extended by the contact between the patch PA and the plate PL is relatively wide. Therefore, an attractive force between the substances moved to the plate PL and the patch PA becomes meaningless.

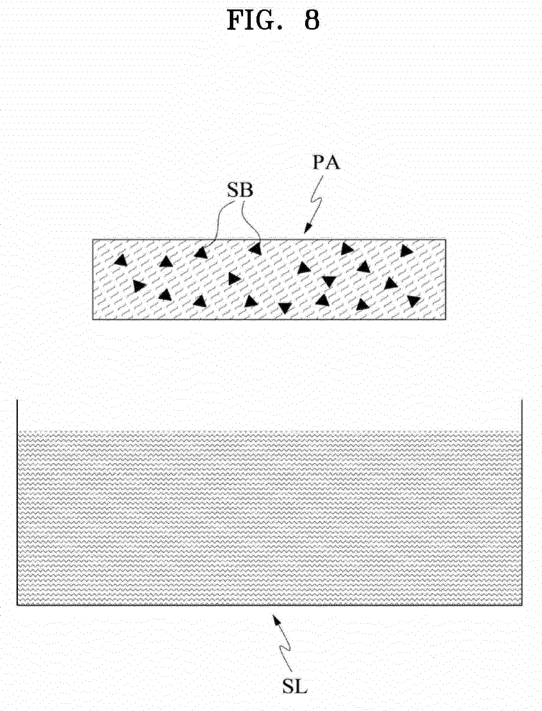

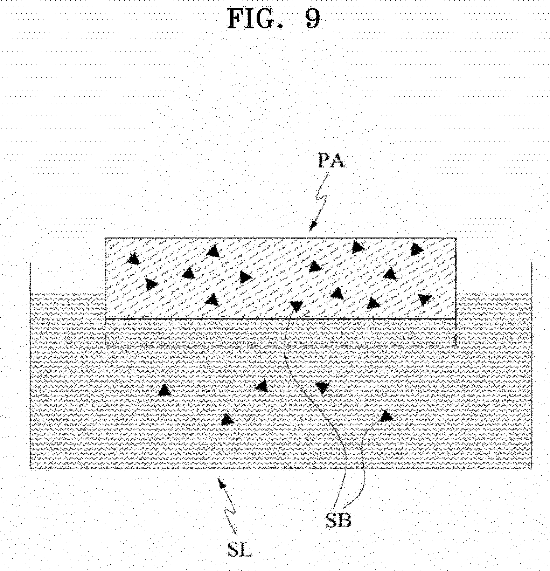

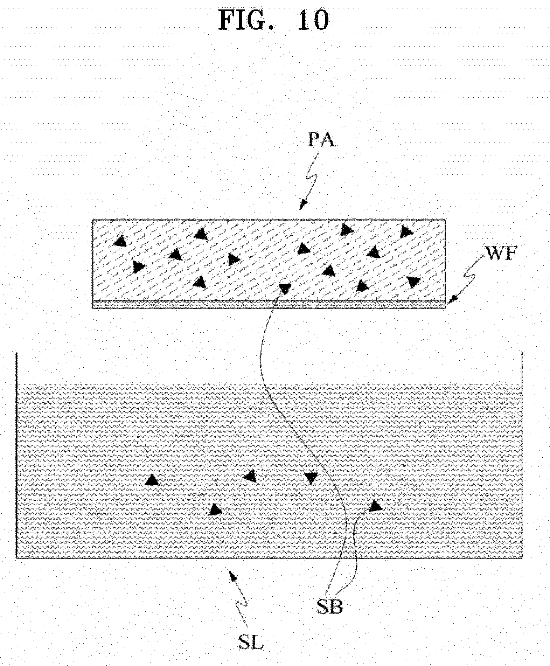

FIGS. 8 to 10 each illustrate transfer of a substance as an embodiment of a function of the patch PA according to the present disclosure. FIGS. 5 to 7 each illustrate transfer of a substance from the patch PA to the substance having liquidity. As shown in FIGS. 8 to 10, when the patch PA contacts the substance having liquidity, some substances stored in the patch PA may be transferred thereto. The transferring a portion of a stored substance is performed by placing or contacting the substance having liquidity with the patch PA such that the liquid substance SB captured in the patch PA and the substance having liquidity may be movable from a substance to a substance.

Here, it is assumed that a substance moves from the patch PA to another patch PA. In the contact area where the patch PA contacts the other patch PA, at least a portion of the liquid substance SB provided to the patch PA may move to the other patch PA.

In the contact area, the liquid substances SB provided to each patch PA may be diffused and moved to (the other) different patches PA.

Here, due to the movement of a substance, a concentration of the liquid substance SB provided to each patch PA may be changed. Also in this embodiment, as described above, the patch PA and the other patch PA may be separated, and at this time, a portion of the liquid substance SB of the patch PA may be transferred to the other patch PA.

Substance migration between the patch PA and the other patch PA may be performed by changing the environmental condition including the physical state change.

The movement of a substance from the patch PA to the other patch PA may depend on a contact area between the patch PA and the other patch PA. For example, efficiency of substance migration of the patch PA and the other patch PA may be increased or decreased depending on an area in which the patch PA contacts the other patch PA.

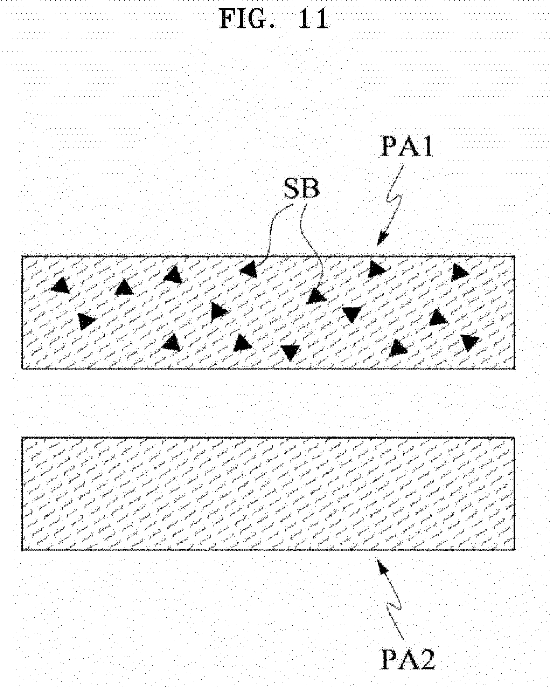

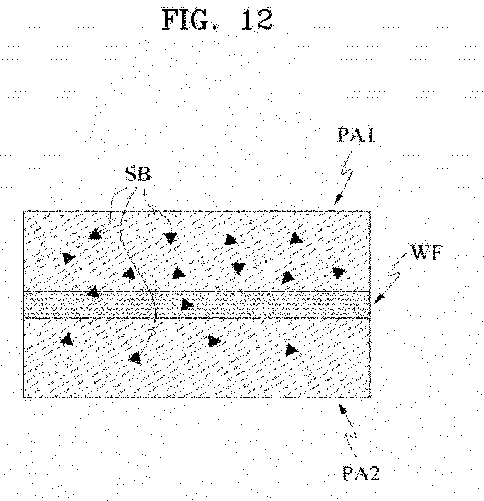

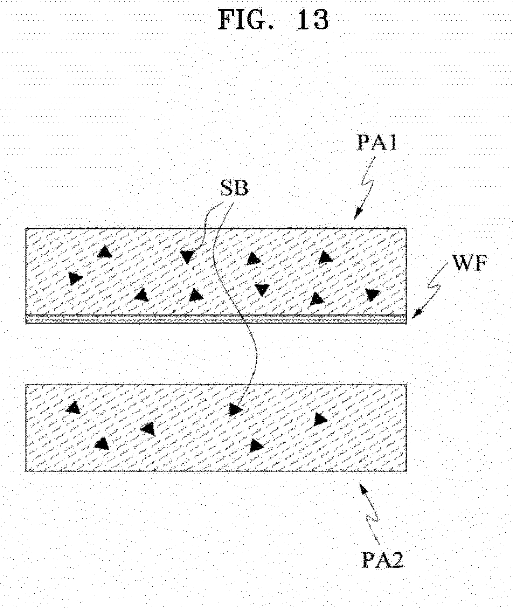

FIGS. 11 to 13 each illustrate transfer of a substance as an embodiment of a function of the patch PA according to the present disclosure. FIGS. 11 to 13 each illustrate transfer of a substance from the patch PA1 to the other patch PA2.

As shown in FIGS. 11 to 13, some substances stored in the patch PA1 may be transferred to the other patch PA2.

The transferring a portion of the substance may be accomplished by having the patch PA1 contact the other patch PA2 to allow the liquid substance SB captured in the patch PA1 and the substance captured in the other patch PA2 to exchange with each other.

### Absorption

Prior to the description, 'absorption' of the function of the patch PA according to the present disclosure may be treated similarly with the above-mentioned 'transfer' in some embodiments. For example, when the movement of a substance due to the concentration difference of a substance is assumed, it is common that the direction of movement of a substance to be moved may be controlled by varying a concentration of the liquid substance SB, particularly a concentration of the additive substance AS. Also, the control of substance migration through selective separation of the physical contact of the patch PA and the selective absorption may be likewise common, which may be clearly understood by those skilled in the art to which the present disclosure pertains.

In the present disclosure, the patch PA may capture an external substance according to the above-described characteristics. The patch PA may pull an external substance outside the area defined by the patch PA into an area where influence of the patch PA is applied. The pulled external substance may be captured like the liquid substance SB of the patch PA. The pulling of the external substance may be caused by an attractive force between the liquid substance SB captured in the patch PA and the external substance. Alternatively, the pulling of the external substance may result from an attractive force between the external substance and an area of the network structure NS not occupied by the liquid substance SB. The pulling of the external substance may be attributed to a force of surface tension.

Hereinafter, such a function of the patch PA is referred to as absorption for convenience. The absorption may be understood as a sub-concept of the channeling function of the patch PA described above, which defines migration of an external substance to the patch PA.

The absorption may occur via/through the patch PA in a state in which the substance is movable and a state in which the substance is immovable.

Substances that the patch PA may absorb are liquid or solid. For example, when the patch PA is in contact with an external substance including a solid state substance, absorption may be performed due to an attractive force between the liquid substance SB located in the patch PA and a solid state substance included in the external substance. In another example, when the patch PA is in contact with an external substance in a liquid phase, absorption may be performed by combining the liquid substance SB located in the patch PA and the external substance in a liquid phase.

The external substance absorbed by the patch PA may move to the inside of the patch PA through microcavities of the network structure NS constituting the patch PA or may be distributed on a surface of the patch PA. The distribution position of the external substance may be determined depending on a molecular weight or a particle size of the external substance.

The shape of the patch PA may be deformed during the absorption. For example, the volume or color of the patch PA may change. While the patch PA is absorbing, an external condition such as a temperature change or a physical state change may be added to the absorption environment of the patch PA to activate or deactivate the absorption by the patch PA.

Hereinafter, absorption will be described as a function of the patch PA, according to some examples of external areas providing a substance to be absorbed by the patch PA in the case of absorption.

Hereinafter, it is assumed that the patch PA absorbs an external substance from the separate outer plate PL. Here, the separate outer substrate may be a plate PL or the like, which does not absorb the external substance, but in which the external substance may be located.

The outer plate PL may be coated with the substance. In particular, the plate PL may be coated with the substance in a form of powder. The substance coated on the plate PL may be a single component or a mixture having a plurality of components.

The plate PL may be in a flat shape. The plate PL may be deformed to improve storage capability of the substance. For example, a well may be formed to improve the storage capability, or the plate PL of which a surface is modified with a negative or positive angle or the patterned plate PL may be used to improve contact with the patch.

The absorbing of a substance by the patch PA according to the present disclosure from the plate PA may be by contact between the plate PL and the patch PA. At this time, in the contact area near the contact surface between the plate PL and the patch PA, a water film WF may be formed due to the liquid substance SB captured in the patch PA and/or the substance coated on the plate PL. When the water film (aquaplane) WF is formed in the contact area, the substance coated on the plate PL may be captured in the water film WF. The substance captured in the water film WF may flow freely in the patch PA.

When the patch PA is separated from the plate PL by a distance equal to or longer than a predetermined distance, the substance coated on the plate PL may be absorbed by the patch PA by moving the water film WF with the patch PA. The substance coated on the plate PL may be absorbed by the patch PA, as the patch PA is separated from the plate PL by a distance equal to or longer than a predetermined distance. When the patch PA and the plate PL are separated from each other, the liquid substance SB provided to the plate PL may not move to the patch PA, or only a small amount thereof may be absorbed by the patch PA.

The whole or part of the substance coated on the plate PL may be specifically reacted with the whole or a part of substances captured in the patch PA. In this regard, the patch PA may selectively perform substance absorption from the separate plate PL. In particular, the selective absorption may be the case where the patch PA has a stronger attractive force than the plate PL for a portion of the substance captured in the patch PA.

For example, some substances may be fixed to the plate PL. In other words, some substances are fixed to the plate PL, and some substances are not fixed or may be coated thereon with liquidity. At this time, when the patch PA and the plate PL are contacted and separated, only substances other than the fixed substance among the substances coated on the plate PL may be selectively absorbed to the patch PA. Alternatively, selective absorption may occur due to polarity of the substance located in the plate PL and the substance captured on the patch PA regardless of whether the substance is fixed or not.

As another example, when the liquid substance SB captured in the patch PA specifically binds to at least a portion of the substance coated on the plate PL, upon contact and separation between the patch PA and the substance coated on the plate PL, only at least a portion of the specifically binding substance coated on the plate PL may be absorbed by the patch PA.

As another example, some of the substances coated on the plate PL may specifically react with substances previously fixed to the plate PL. In this case, only the substance coated on the plate PL, except the substance specifically reacting with the substance previously fixed to the plate PL, may be absorbed by the patch PA.

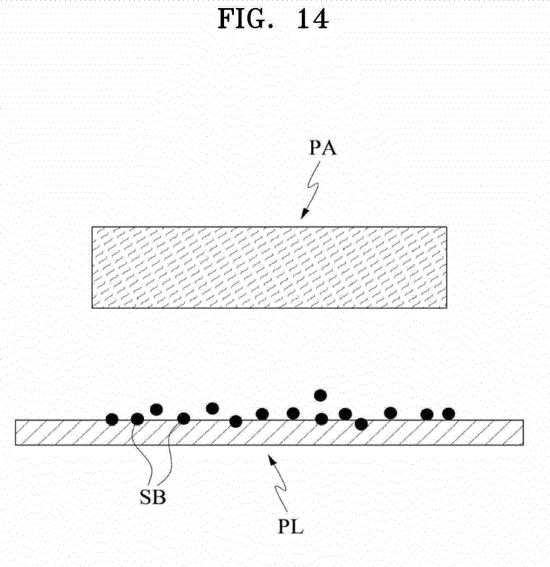

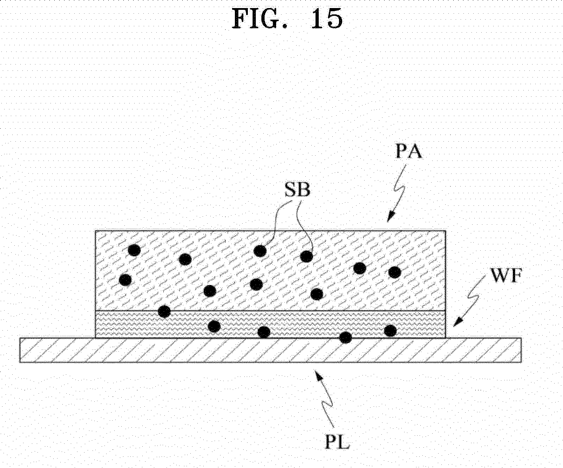

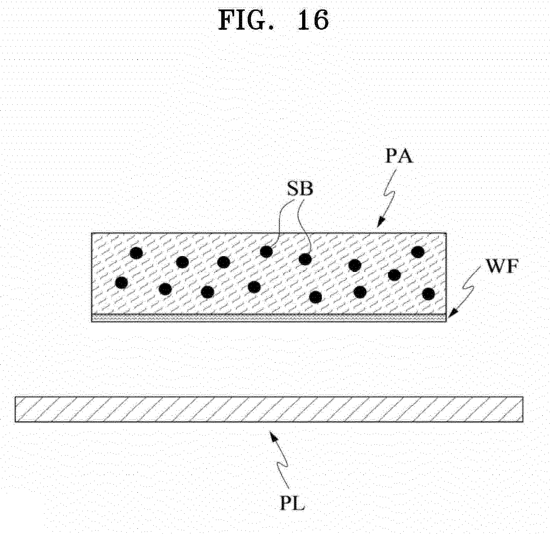

FIGS. 14 to 16 each illustrate absorption of a substance as an embodiment of a function of the patch PA according to the present disclosure. FIGS. 14 to 16 each illustrate absorption of a substance from the outer plate PL by the patch PA. As shown in FIGS. 14 to 16, the patch PA may absorb some substances located in the outer plate PL from the outer plate. The absorbing of substance may be performed by the formation of the water film WF, which is formed near a contact area between the outer plate PL and the patch PA by contacting the patch PA with the outer plate PL, the water film WF enabling migration of the substance to the patch PA through the water film WF.

Here, it is assumed that the substance is absorbed from the substance having liquidity SL to the patch PA. The substance having liquidity SL may be an external liquid substance flowing or contained in a separate storage space. In particular, since the substance having liquidity SL and the liquid substance SB captured in the patch PA have an environment in which the substances may mutually flow, a portion or all of the substances having liquidity SL may be absorbed in the patch PA. Here, the mutual flowable environment may be formed by at least partially contacting the patch PA with the substance having liquidity SL.

When the patch PA is contacted with the substance having liquidity SL, the patch PA may be in a state capable of moving a substance to and from the substance having liquidity SL. When the patch PA is separated from the substance having liquidity SL, at least a portion of the substance having liquidity SL may be absorbed by the patch PA.

The absorbing of a substance from the substance having liquidity SL to the patch PA may depend on a concentration difference of the substance having liquidity SL and the substance captured in the patch PA. In other words, when a concentration of the liquid substance SB captured in the patch PA with respect to the predetermined additive substance AS is lower than a concentration of the substance having liquidity SL has with respect to the predetermined additive substance AS, The predetermined additive substance AS may be absorbed by the patch PA.

When the substance is absorbed from the substance having liquidity SL to the patch PA, in addition to the dependency on a concentration difference while in contact as described above, an electrical factor may be added, or physical conditions may be changed to control the absorption by the patch PA. Furthermore, the substance captured in the patch PA and the substance to be absorbed may not be directly contacted, but may be indirectly contacted through a medium to perform substance absorption.

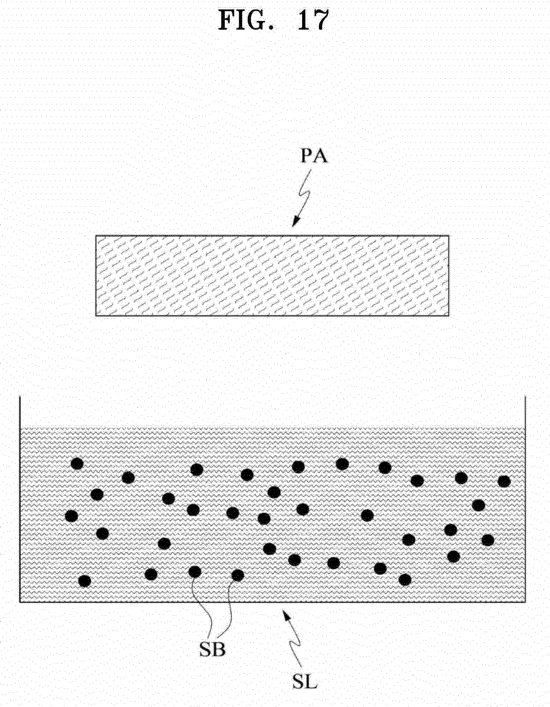

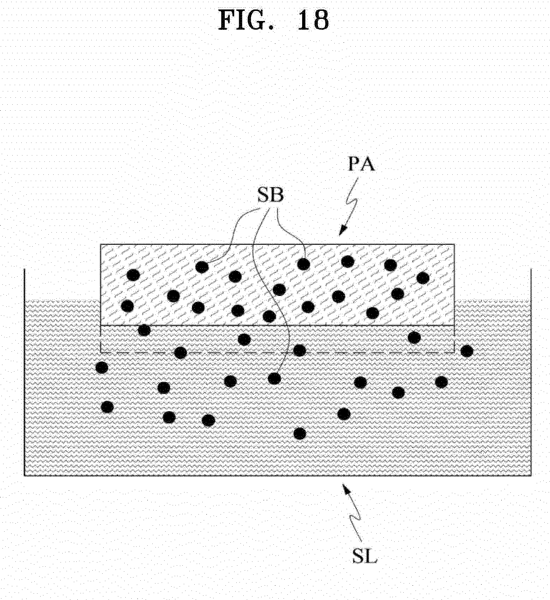

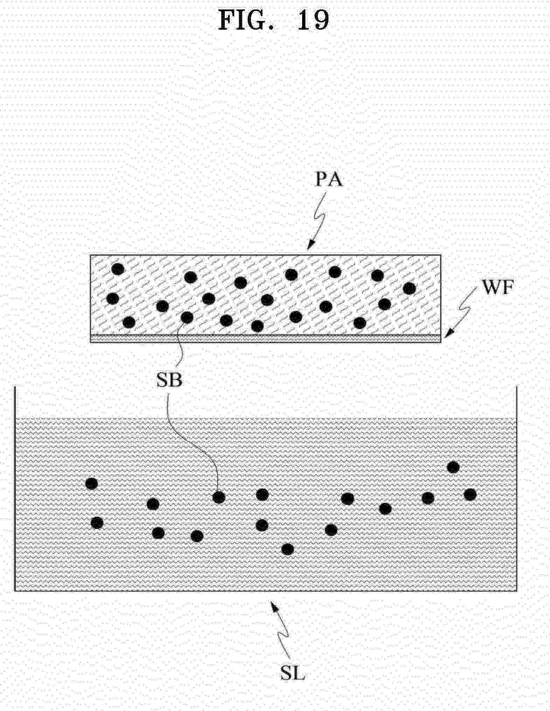

FIGS. 17 to 19 each illustrate absorption of a substance as an embodiment of a function of the patch PA according to the present disclosure. FIGS. 17 to 19 each illustrate absorption of a substance from the substance having liquidity SL by the patch PA. As shown in FIGS. 17 to 19, the patch PA may absorb some substance having liquidity SL. The absorbing of the substance may be achieved by adding the patch PA to the substance having liquidity SL or contacting the patch PA with the substance having liquidity SL to enable the liquid substance SB captured in the patch PA and the substance having liquidity SL to be movable with respect to each other.

Hereinafter, it is assumed that the patch PA absorbs an external substance from the other patch PL.

The absorbing an external substance from the other patch PA by the patch PA may occur due to a difference in a binding force between the absorbed external substance and the substance captured by the patch PA and a binding force between the absorbed external substance and the external substance not absorbed by the patch PA. For example, when the absorbed substance is hydrophilic, the patch PA is hydrophilic, and an attractive force of the patch PA and a substance to be absorbed is stronger than an attractive force between the other patch PA and the substance to be absorbed (that is, when the patch PA is more hydrophilic than the other patch PA), the external substance may be at least partially absorbed by the patch PA upon separation followed by contact between the patch PA and the other patch PA

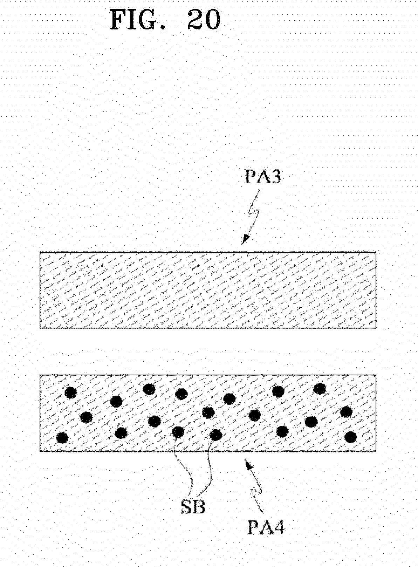

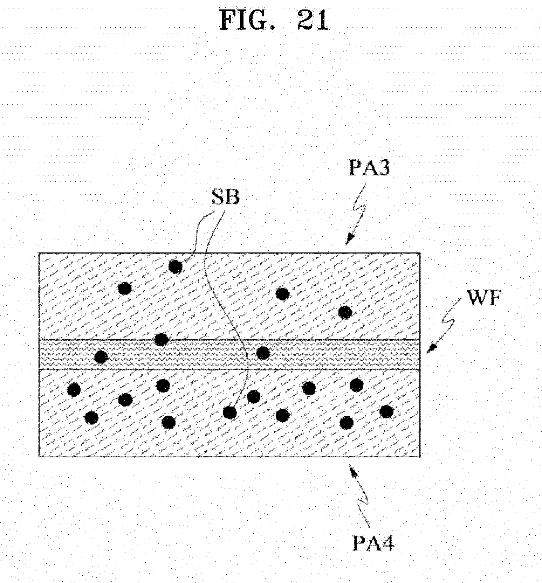

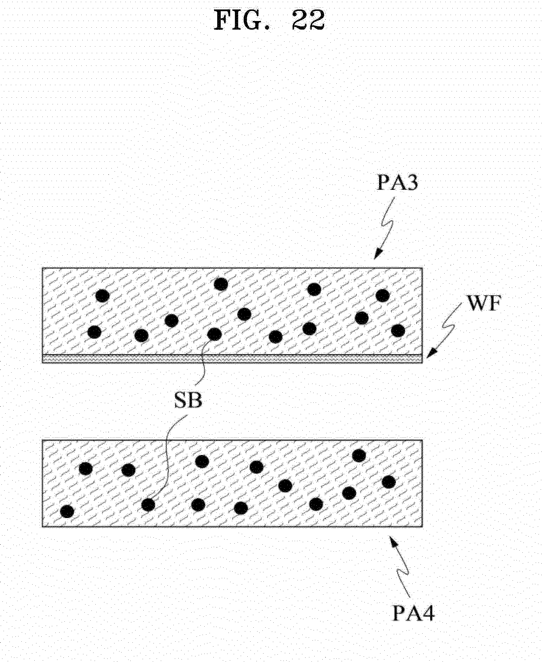

FIGS. 20 to 22 each illustrate absorption of a substance as an embodiment of a function of the patch PA according to the present disclosure. FIGS. 20 to 22 each illustrate absorption of a substance from the other patch PA4 by the patch PA3. As shown in FIGS. 20 to 22, the patch PA3 may partially absorb substances located in the other patch PA4. The absorbing of the substance may be achieved by contacting the patch PA3 with the other patch PA4 to enable the liquid substance SB captured by the patch PA3 and the liquid substance SB captured by the other patch PA4 to be movable with respect to each other.

Depending on a ratio of a frame structure of the three-dimensional network structure NS constituting the patch PA to the total volume of the patch PA, the binding force of the patch PA to the external substance to be absorbed may change. For example, as a volume ratio of the frame structure in the entire patch PA increases, the amount of the substance captured in the structure may be reduced. In this case, a binding force between the patch PA and the target substance may be reduced due to a decrease in contact area between the substance captured in the patch PA and the target substance.

In this regard, the polarity of the patch PA may be controlled by adjusting a ratio of the material constituting the network structure NS in a manufacturing process of the patch PA. For example, in the case of patch PA manufactured using agarose, a concentration of the agarose may be controlled to adjust a degree of absorption.

When the patch PA and the other patch PA are contacted and separated from each other, and the separate area is weaker than the patch PA in binding force with respect to the substance provided from the patch PA, the external substance to be absorbed may be separated from the other patch PA together with the patch PA.

### 2.2.4.3 Provision of environment

By the foregoing characteristics of the patch PA according to the present disclosure, an environment condition of a desired area may be controlled. The patch PA may provide an environment resulting from the patch PA in a desired area.

The environment condition resulting from the patch PA may depend on the liquid substance SB captured in the patch PA. The patch PA may create a desired environment for the substance located in the external area from or according to the characteristics of the substance received in the patch PA.

The controlling of the environment may be understood as changing the environment condition of a desired area. The changing of the environment condition of the desired area may be performed in such a manner that the area affected by the patch PA is expanded to include at least a portion of the desired area or the environment of the patch PA is shared with the desired area.

Hereinafter, such a function of the patch PA is referred to as provision of environment.

the provision of environment by the patch PA may be performed in a state in which a substance is movable between the patch PA and the external area to provide the environment. The provision of environment by the patch PA may be performed by a contact. For example, when the patch PA contacts a desired area (e.g., an external substance, the plate PL, or the like), the patch PA may provide a specific environment to the desired area.

The patch PA may provide an environment of appropriate pH, osmotic pressure, humidity, concentration, temperature, or the like to adjust the environment of a target area TA. For example, the patch PA may provide liquidity to the target area TA or a target substance. Such provision of liquidity may occur by partial migration of substance captured in the patch PA. A wetting/moist environment may be provided to the target area TA through the liquid substance SB captured in the patch PA or the base substance BS.

The environment factors provided by the patch PA may be retained constant according to the purpose. For example, the patch PA may provide homeostasis in the desired area. As another example, as a result of the provision of environment, the environment condition of the desired area may be adapted to the substance captured in the patch PA.

The provision of environment by the patch PA may be a result of diffusion of the liquid substance SB contained in the patch PA. That is, when the patch PA and the desired area are in contact, a substance may be movable through the contact area formed by the contact. In this regard, depending on a diffusion direction of the substance, an environment change due to osmotic pressure, an environment change depending on the ion concentration, provision of a damp environment, and a change in pH may be realized.

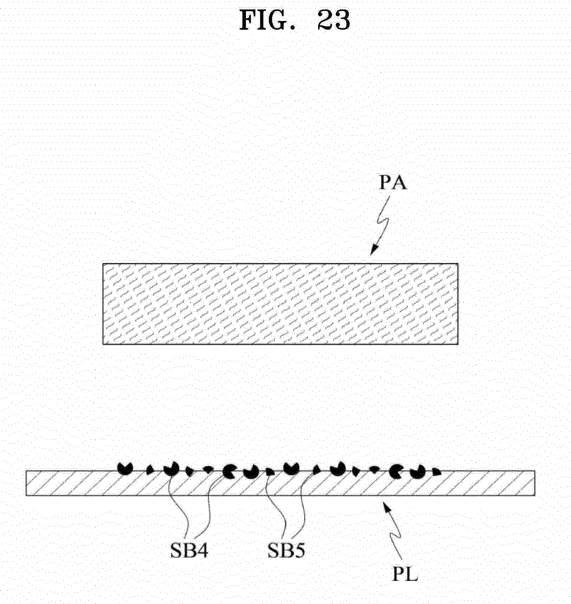

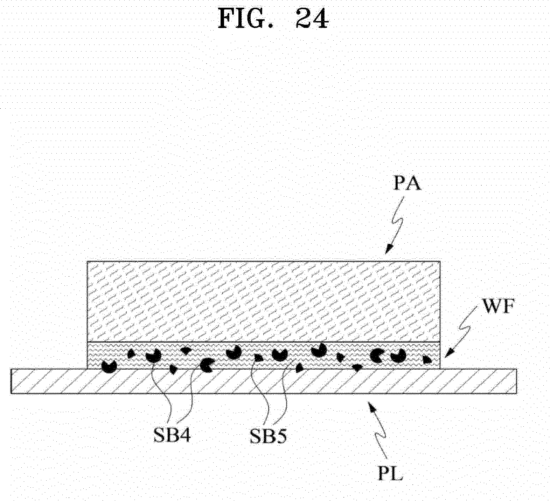

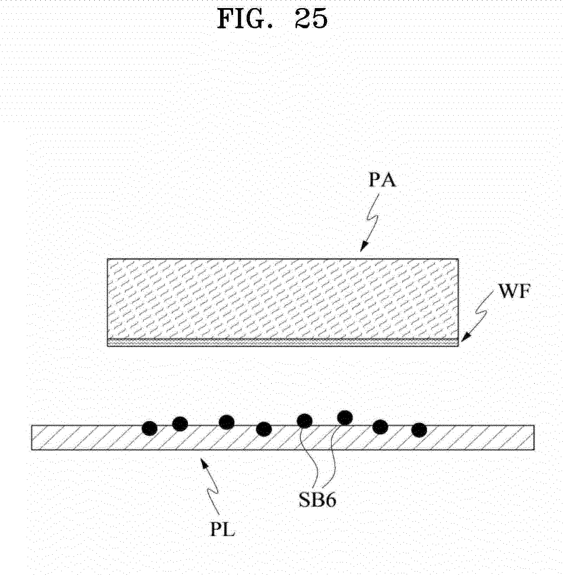

FIGS. 23 to 25 each illustrate provision of environment as an embodiment of a function of the patch PA according to the present disclosure. FIGS. 23 to 25 each illustrate provision of a predetermined environment to the outer plate PL by the patch PA. As shown in FIGS. 23 to 25, the patch PA may provide predetermined environment in the outer plate PL in which a fourth substance SB4 and a fifth substance SB5 are located. For example, the patch PA may provide a predetermined environment for forming a sixth substance SB6 by reacting the fourth substance SB4 and the fifth substance SB5 on the plate PL. The providing of the environment may be accomplished by forming the water film WF near the contact area by contacting the patch PA with the plate PL and capturing the fourth substance SB4 and the fifth substance SB5 in the formed water film WF.

### Application of patch

The patch PA according to the present disclosure may be implemented to perform various functions by appropriately applying the functions of the patch PA described above.

Hereinafter, the technical idea of the present disclosure will be described by disclosing some embodiments. However, the technical scope to which the function of the patch PA disclosed in the present disclosure is applied is to be construed to be extended to a range that may be derived by one of ordinary skill in the art, and the scope of rights of the present disclosure should not be construed as being limited by the embodiments.

### In-patch

The patch PA may provide a reaction area of substances. In other words, a reaction of substances may occur in at least a portion of a space area affected by the patch PA. Here, the reaction of substances may be a reaction between the liquid substances SB captured in the patch PA, and/or the captured liquid substance SB and the substance provided from outside of the patch PA. The providing of a reaction area of substances may be to activate or promote a reaction of substances.

In this case, the liquid substance SB captured in the patch PA may include at least one of a substance input at the time of the manufacture of the patch PA, a substance stored in the patch PA input into the patch PA after the manufacture of the patch PA, and a substance temporarily captured in the patch PA.

In other words, when a substance is captured in the patch PA at the time of activation of a reaction in the patch PA, the substance may undergo the reaction in the patch PA regardless of the form of the substance captured in the patch PA. Further, a substance added after the manufacture of the patch PA may act as a reaction initiator.

The provision of a reaction area for a reaction involving the liquid substance SB captured in the patch PA may be a sub-concept embodiment of Section 2.1.3 (i.e., Provision of reaction space) described above. The provision of a reaction area may also be a multi-concept combined with the functions described in Section 2.1.3 and Section 2.2.4.2 (i.e., Absorption) described above. The provision of a reaction area is not limited to these concepts, and be realized in a form in which two or more functions are combined.

### First Example

Hereinafter, it will be assumed that an absorption function and a function of providing a reaction space (hereinafter referred to as a providing function) of the patch PA are performed by a single patch PA. In this case, the absorption function and the providing function may be performed at the same time, or at different points in time, or sequentially performed to perform another function. These Examples may be understood that the patch PA may further include other functions in addition to the absorption and providing function.

As described above, the patch PA may perform a function of capturing a substance, and even when the substance is captured, the substance may have liquidity. When the distribution of some components of the liquid substance SB is non-uniform, the non-uniform components may diffuse. Even when the components of the liquid substance SB are uniformly distributed, the liquid substance SB may have a certain level of mobility due to the irregular motion of particles. Here, a reaction between substances, for example, specific binding between substances, may occur in the patch PA.

For example, in the patch PA, in addition to the reaction between captured substances, a reaction may also occur, in which a substance having liquidity newly captured in the patch PA and a substance captured in the patch PA are specifically binding with each other.

A reaction between the substance having liquidity and the captured substance may occur separately from an arbitrary space in which the substance having liquidity is provided. For example, after the patch PA absorbs the substance having liquidity from an arbitrary space, the patch PA may be separated from the arbitrary space, and a reaction between the absorbed substance and the substance captured in the patch PA may occur in the patch PA.

In addition, the patch PA may perform an absorption function on a substance having liquidity so that a reaction of the captured substance may occur. In other words, absorption of the substance having liquidity in the patch PA as a trigger may cause a reaction of the absorbed substance and the substance captured in the patch PA.

The reaction may be performed within the space defined by the patch PA.

Also, due to the reaction that occurs inside the patch PA, a composition of the liquid substance SB captured in the patch PA may be changed. In particular, when the substance captured in the patch PA is a compound, the chemical composition thereof may be changed before and after the reaction. Alternatively, the composition distribution may be changed depending on the position of the substance in the patch PA. This feature may be exemplified by diffusion or particles having a specific attractive force for other substances.

When the composition of the liquid substance SB is changed due to the reaction inside the patch PA, some substance is absorbed by the patch PA due to a difference in concentration between the patch PA and a substance outside the patch PA (in the case of a substance in contact, the substance in contact), or the substance may be released from the patch PA to the substance outside the patch PA.

### Second Example

Hereinafter, an embodiment will be described in which a storage function and a function of providing a reaction space of the patch PA are performed simultaneously for at least a predetermined time. In particular, a function for providing space for reaction of at least a portion of the liquid substance SB stored in the patch PA may be performed.

The patch PA may store a substance, and the stored substance may provide a reaction space. Here, the reaction space provided by the patch PA may be microcavities formed by the network structure NS of the patch PA or a surface area of the patch PA. Particularly, when the substance stored in the patch PA and the substance coated on a surface of the PA are reacted, the reaction space may be the surface area of the patch PA.

The reaction space provided by the patch PA may provide a specific environment condition. The patch PA may control the environmental condition of the reaction during the reaction of the liquid substance SB located in the patch PA. For example, the patch PA may perform a function as a buffering solution.

The patch PA may not require a separate storage container by storing the substance within the network structure. Also, when the reaction space of the patch PA is the surface of the patch PA, observation may be facilitated through the surface of the patch PA. To this end, the shape of the patch PA may be modified and designed to be easy to be observed.

The liquid substance SB stored in the patch PA may be denatured or react with other kinds of substances. The composition of the liquid substance SB stored in the patch PA may be changed over time.

The reaction may be a chemical reaction in which a chemical formula is changed, a physical state change, or a biological reaction. In this case, the liquid substance SB stored in the patch PA may be a substance of a single component or a mixture containing a plurality of components.

### Channeling

Hereinafter, the patch PA that performs a function of providing a substance migration path will be described. In particular, the patch PA may capture, absorb, release, and/or store the substance having liquidity and the like, as described above. Various embodiments of the patch PA may be implemented that perform functions of providing a substance migration path, as each or combined function of the patch PA described above. However, some embodiments will be described for better understanding.

### Third Example

The patch PA may be implemented to perform Section 2.2.4.1 (i.e., Transfer) and Section 2.2.4.2 (i.e., Absorption) of the functions of the patch PA described above. At this time, the absorption function and the transfer function may be provided simultaneously and provided sequentially.

The patch PA may perform the absorption and the transfer function simultaneously to provide a substance migration path. In particular, a migration path for an external substance may be provided by absorbing the external substance and transferring the external substance to an external area.

The providing of an external substance migration path by the patch PA may be performed by absorbing the external substance and releasing the external substance. In particular, the patch PA may contact the external substance to absorb the external substance and contact the external area to transfer the external substance to the external area. Here, the patch PA may capture the external substance and transfer the external substance to the external area by a process similar to the foregoing absorption and transfer process.

The external substance that is absorbed and transferred to the patch PA may be liquid or solid.

This feature may allow the patch PA to transfer some substance from the external substance to the other external substance. The patch PA may be in contact with and the external substance and the other external substances the same time. The patch PA may be in contact with the external substance and the other external substances at different point of time.

The patch PA may be in contact with the external substance and the other external substances at different point in time. When each external substance is in contact at a different point in time, the patch PA and the external substance are first contacted, and after the external substance and the patch PA are separated, the patch PA and the other external substance may be contacted. Here, the patch PA may temporarily store the substance captured from the external substance.

The patch PA may provide additional time delay while providing the substance migration path. In addition, the patch PA may perform a function of appropriately controlling the transfer amount and transfer rate of a substance to another external substance.

This series of processes may be performed in one direction based on the patch PA. As an example, substance absorption may be achieved through a surface of the patch PA, the environment may be provided in an inner space of the patch PA, and the substance may be released through the other surface facing the surface.

### Fourth Example

The patch PA may provide a reaction space of a substance while absorbing and releasing the substance, as the function of the patch PA described above. Here, the absorption of a substance, the release of a substance, and the provision of a reaction space may be performed simultaneously or sequentially.

According to an embodiment, the patch PA may provide a reaction space for at least a period of time to the absorbed external substance in the process of absorbing and releasing an external substance. The patch PA may provide a specific environment for at least a period of time to the liquid substance SB captured in the patch PA that includes the absorbed external substance.

The liquid substance SB captured in the patch PA and the external substance captured in the patch PA may be reacted in the patch PA. The external substance absorbed in the patch PA may be affected by the environment provided by the patch PA. The substance released from the patch PA may include at least a portion of a substance generated by the reaction. The external substance may be released from the patch PA by changing its composition, characteristics, and the like.

The absorbed substance may be released from the patch PA. Absorption of the external substance in the patch PA and release of the external substance from the patch PA are understood to be passing through the patch PA. The external substance passing through the patch PA may lose the identity due to the reaction in the patch PA or the influence of environment provided by the patch PA.

The process of absorption, substance reaction, and substance transfer of the external substance described above may proceed in one direction. In other words, substance absorption is performed at one position of the patch PA, provision of environment is performed at another position, and substance release may be performed at still another position.

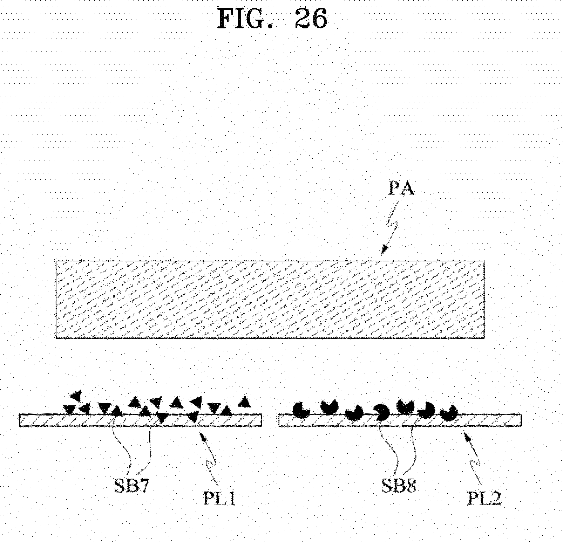

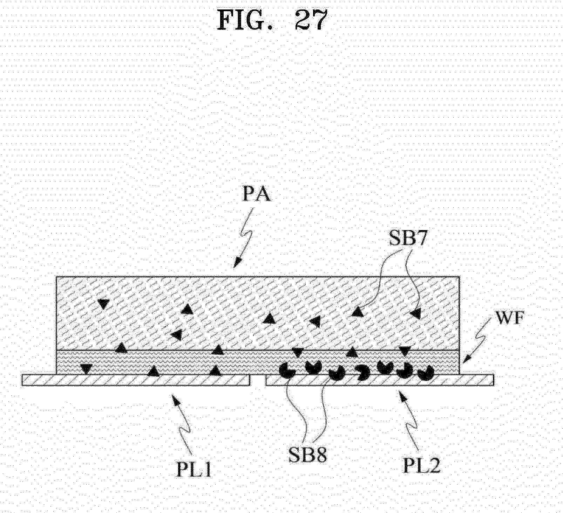

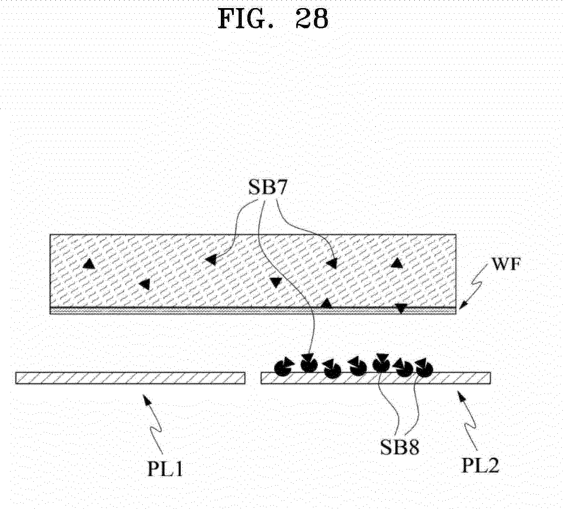

FIGS. 26 to 28 each illustrate an embodiment of a patch according to the present disclosure. FIGS. 26 to 28 each illustrate provision of a substance migration path between two plates PL. As shown in FIGS. 26 to 28, the patch PA may provide a substance migration path between a plate PL1 coated with a seventh substance SB7 and a plate PL2 coated with an eighth substance SB8. As an example, when the seventh substance SB7 is associated with the eighth substance SB8, and the eighth substance SB8 is fixed to the plate PL2, the patch PA may contact the plates PL1 and PL2 to allow the seventh substance SB7 to migrate through the patch PA to combine with the eighth substance SB8.

The seventh substance SB7 and the eighth substance SB8 are connected to the patch PA may be by a water film WF formed by contacting the patch PA with the respective plates PL1 and PL2.

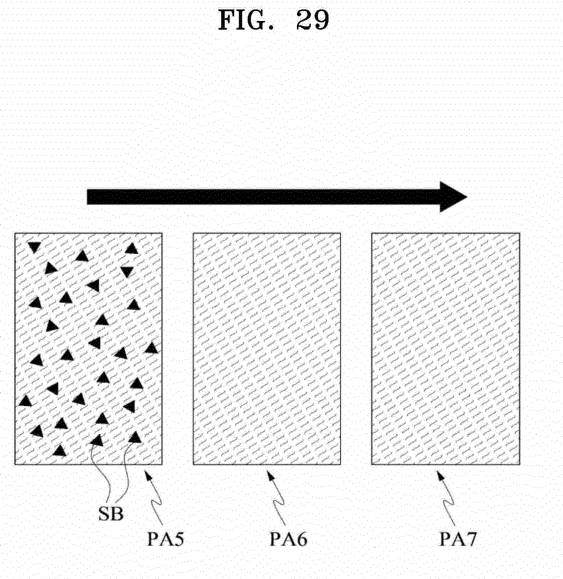

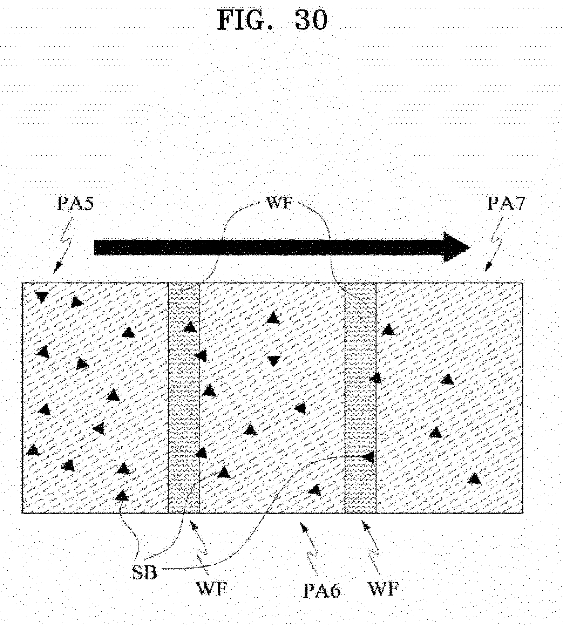

FIGS. 29 and 30 each illustrate an embodiment of a patch according to the present disclosure. FIGS. 29 and 30 each illustrate provision of a substance migration path between two patches. As shown in FIGS. 29 and 30, a patch PA6 providing a migration path may be in contact with a patch PA5 storing a substance to be moved and a patch PA7 that receives the substance to be moved. The patch PA6 providing the migration path contacts the patch PA5 storing the substance to be moved and the patch PA7 that receives the substance to be moved so that the substance to be moved may migrate to the patch PA7 that receives the substance to be moved. The movement of a substance between each patch may be achieved through the water film WF formed near the contact area between each patch.

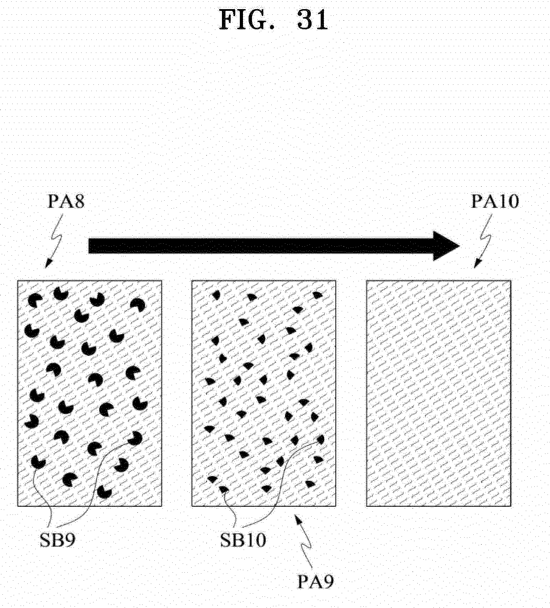

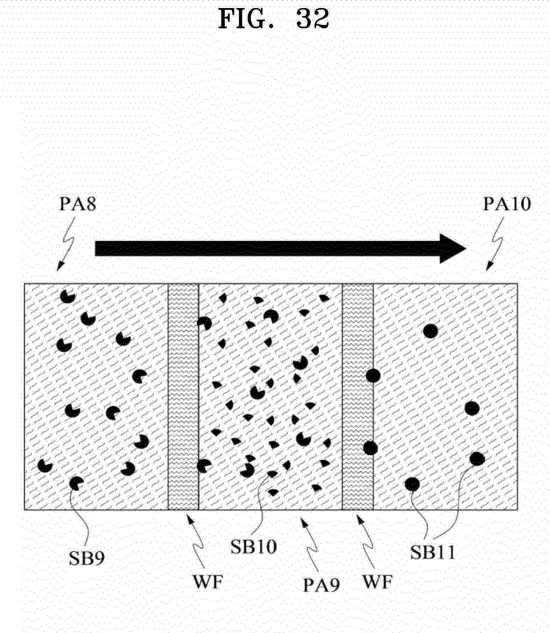

FIGS. 31 and 32 each illustrate an embodiment of a patch according to the present disclosure. FIGS. 31 and 32 each illustrate provision of a substance migration path between two patches. As shown in FIGS. 29 and 30, a patch PA9 providing a migration path may be in contact with a patch PA9 storing a ninth substance SB9 and a patch PA10 that receives the ninth substance SB9. the patch PA9 providing a migration path may contact the patch PA8 storing the ninth substance SB9 to absorb the ninth substance SB9. The absorbed ninth substance SB9 may react with a tenth substance SB10 stored in the patch PA9 providing a migration path to form an 11 th substance. The 11th substance SB11 may be transferred from the patch PA9 providing the migration path to the patch PA10 receiving the substance. The movement of a substance between each patch PA may be achieved through the water film WF formed near the contact area between each patch PA.

### Multi patches

The patches PA may be used alone or in combination as a plurality of patches PA. In this case, the expression that the patch PA may be used in combination as a plurality of patches PA may include that the patches PA used simultaneously and that the patches PA used sequentially.

When the plurality of patches PA are used simultaneously, each of the patches PA may perform different functions. Each patch PA of the plurality of patches PA may store the same substance, but each patch PA may also store different substances.

When the plurality of patches PA are used simultaneously, each patch PA may not contact each other, and thus, substance migration between the patches PA may not occur. Alternatively, when the plurality of patches PA are used simultaneously, each patch PA may perform a desired function while substances stored in each patch are interchangeable with each other.

The plurality of patches PA used together may be made in similar shapes or in the same size, however, the patches PA may also be used together even in different shapes.

In addition, each patch PA constituting the plurality of patches PA may have different densities of the network structure NS or different components constituting the network structure NS.

### 3.3.1 Contacting a plurality of patch

When the plurality of patches PA are used, the plurality of patches PA may contact the target area TA. The plurality of patches PA may perform a desired function by contacting the target area TA.

The plurality of patches PA may be contacted in different target areas TA in the presence of a plurality of target areas TA. The plurality of patches PA may perform desired functions by contacting the target area TA corresponding to each of the target areas TA in the presence of a plurality of target areas TA.

The plurality of patches PA may be contacted with the substance coated on the target area TA. Here, the substance coated on the target area TA may be fixed or have liquidity.

The desired function may be transfer or absorption of a substance. However, it is not necessary that each patch PA transfers or absorbs the same substance, but each patch PA may transfer different substances to the target area TA or may absorb different components from a substance located in the target area TA.

The desired function may differ depending on the patch PA constituting the plurality of patches PA. For example, one patch PA may perform a function of transferring a substance to a target area TA, while another patch PA may perform a function of absorbing a substance from the target area TA.

The plurality of patches PA may contain different substances, and the different substances may be transferred to one target area TA and used to derive a desired reaction. In order to cause the desired reaction, when a substance of a plurality of components is required, the substance of a plurality of components may be respectively stored in a plurality of patches PA and transferred to the target area TA. The use of such a plurality of patches PA may be particularly useful when the substances required for a reaction are mixed to be stored in a single patch PA, or when properties of the substance required for a desired reaction are lost or altered.

According to an embodiment, when the plurality of patches PA each include substances of different components, and the substances of different components have different specific binding relationships, the substances of different components may be transferred to the target area TA. The plurality of patches PA may be used to detect a plurality of specific binding from a substance coated on the target area TA by transferring the substance of different components.

According to another embodiment, the plurality of patches PA may include a substance of the same component, and each patch PA may have a different concentration of the substance of the same component. The plurality of patches PA including the substance of the same component may be contacted with the target area TA to identify the effect of a concentration of the substance included in the plurality of patches PA.

In the case of using the plurality of patches PA as described above, the patch PA bundles may be used in a more efficient form. In other words, the configuration of the plurality of patches PA used may be used differently each time. In other words, the plurality of patches PA may be made in the form of a cartridge. Here, the shape of each patch PA may be appropriately standardized.

The plurality of patches PA in the form of a cartridge may be suitable for a case where the patch PA storing each of a plurality of kinds of substances is prepared for a selective use according to a need.

In particular, when a plurality of kinds of substances is used to detect a specific reaction of each substance from the target area TA, a combination of specific reactions to be detected may be constructed in a different manner at each detection.

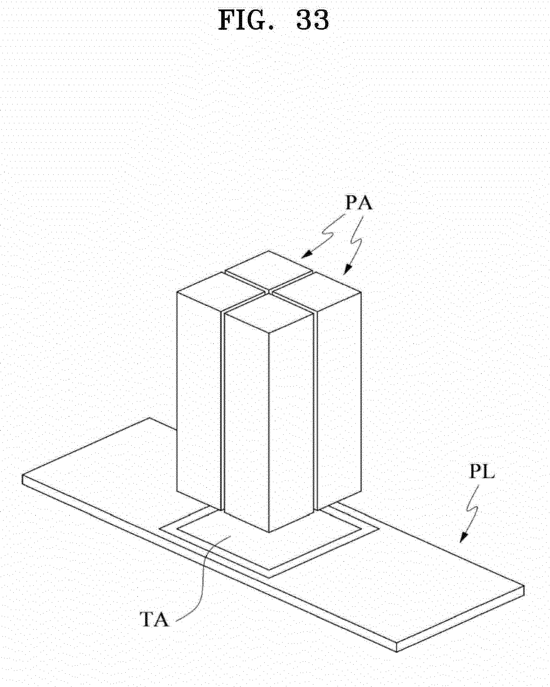

FIG. 33 illustrates an embodiment of the patch PA according to the present disclosure. FIG. 33 illustrates combined use of a plurality of patches PA. As shown in FIG. 33, the plurality of patches PA according to the present disclosure may contact the target area TA located on the plate PL. The patch PA constituting the plurality of patches PA may have a standardized shape. The plurality of patches PA may include a first patch and a second patch, and a substance stored in the first patch may be different from a substance stored in the second patch.

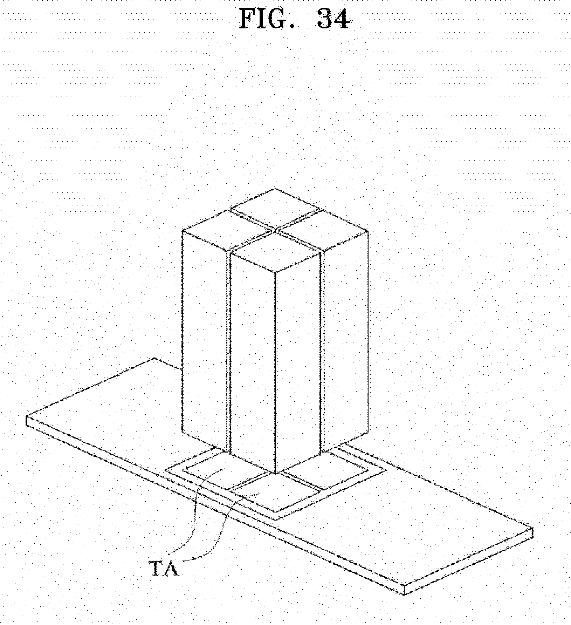

FIG. 34 illustrates combined use of a plurality of patches PA and the plate PL including a plurality of target areas TA. As shown in FIG. 34, the plurality of patches PA according to the present disclosure may contact the plurality of target areas TA located on the plate PL. The plurality of patches PA may include a first patch PA and a second patch PA, the plurality of target areas TA may include a first target area and a second target area, the first patch may be in contact with the first target area, and the second patch may be in contact with the second target area.

### 3.3.2 Fifth Example

The plurality of patches PA may perform a plurality of functions. As described above, each patch PA may simultaneously perform a plurality of functions, and each patch PA may perform different functions simultaneously. However, embodiments are not limited thereto. Each function may be performed in combination in a plurality of patches PA.

First, when each patch PA performs a plurality of functions simultaneously, each patch PA may perform both substance storage and release. As an example, each patch PA may store different substances and release each stored substance to the target area TA. In this case, each stored substance may be released simultaneously or sequentially.

Next, when each patch PA performs a plurality of different functions simultaneously, each patch PA may perform both substance storage and release separately. In this case, some of each of the patches PA may be in contact with the target area TA and release a substance to the target area TA.

### 3.3.3 Sixth Example

When a plurality of patches PA are used, the plurality of patches PA may perform a plurality of functions as described above. First, each patch PA may simultaneously store, release, and absorb a substance. Each patch PA may also separately store, release, and absorb a substance. However, embodiments are not limited thereto. Each function may be performed in combination in a plurality of patches PA.

As an example, at least some of a plurality of patches PA may store different substances and release each stored substance to the target area TA. Here, at least some of a plurality of the other patches PA may absorb a substance from the target area TA. Some of the plurality of patches PA may release a substance specifically binding to a substance located in the target area TA. Here, a specific binding may be detected by using the other patch PA absorbing a substance not forming the specific binding among the substances located in the target area TA.

### 3.3.4 Seventh Example

When a plurality of patches PA are used, each patch PA may simultaneously store and release a substance and provide an environment. Each patch PA may also separately store and release a substance and provide an environment. However, embodiments are not limited thereto. Each function may be performed in combination in a plurality of patches PA.

As an example, one patch PA of a plurality of patches PA may release a stored substance to a target area TA. Here, another patch PA may provide an environment to the target area TA. Here, the providing of an environment may be realized by transferring an environment condition of a substance stored in the other patch PA to the target area TA.

In particular, a reaction substance may be provided to the target area TA by the patch PA, and the other patch PA may be contacted with the target area TA to provide a buffering environment.

For another example, the plurality of patches PA may be in contact with each other. Here, at least one patch PA may store a substance and release the stored substance to the other patch PA providing an environment. In this embodiment, the patch PA providing the environment releases a substance and contacts each at least one patch PA that is not in contact with each other, and the patch PA may absorb the substance from each patch PA.

### Storage medium

### Significance of storage medium

According to an embodiment of the present disclosure, a storage medium may store a reagent. The reagent may be used in diagnosis of a sample.

The storage medium may transfer the reagent to the patch. The storage medium may contact a patch to transfer the reagent to the patch. The storage medium may transfer the reagent to a reaction area. The storage medium may contact the reaction area to transfer the reagent to the reaction area.

The reagent may be stored in a separate storage medium to alleviate deterioration of the reagent. When the reagent is stored in the storage medium, deterioration of the reagent may be delayed. In particular, when the patch is used in a reaction, the reagent may be stored for a long time in the patch to thereby cause deterioration thereof. The deteriorated reagent may be difficult to be used in a desired reaction. The storage medium may improve storing ability of the reagent. When the reagent is stored in the storage medium, a substance that may be prone to deteriorate may be easily stored. By storing the reagent in the storage medium, the storage period of the reagent may be extended, and the quality change during storage may be minimized.

Hereinafter, the storage medium, an inspection method using the storage medium, and an inspection module using the storage medium will be described.

### Function of storage medium

The storage medium according to the present disclosure may be used for handling of a reagent or a substance (hereinafter referred to as "substance"). The storage medium may be used for storing, retaining, and transferring of the substance. However, embodiments are not limited thereto, and the storage medium may be used in various manner for handling a substance.

Hereinafter, functions of the storage medium will be described.

### Storage

The storage medium described in the present disclosure may be used to store a substance. The storage medium may store the substance. The storage medium may alleviate quality change of the substance. The storage medium may delay deterioration of the substance. The storage medium may prevent deterioration of the substance.

In the present specification, it is understood that the storage medium "storing" a substance is independent of the "storing" function of the patch described above. The storage medium for storing a substance and the patch for storing a substance are not the same and may be understood as independent functions. The storage medium and the patch may store the substance in a similar mechanism. In the present specification, it is understood that the storage medium "storing" the substance encompasses that the storage medium receives or contains the substance.

The storage medium may store the substance in a storage state. The storage state may be a state in which the substance is provided at a high concentration. For example, the storage state may be a state where the substance is coagulated, the substance is dried, the substance is lyophilized, the substance is coated on the storage medium, the substance is printed on the storage medium, and the like. The storage state may be a non-activated state of a substance.

With respect to the non-activated state, a storage function of the storage medium will be described below.

The storage medium may store the substance in a solid phase. The storage medium may store a liquid substance and store the substance in a dried state. The storage medium may store a frozen substance. The storage medium may store a lyophilized substance.

The substance may be fixed and stored in the storage medium. The substance may be coated on a surface of the storage medium. The substance may be printed on one surface of the medium. The state where the substance is fixed may mean a state where the substance is not easily separated from the storage medium due to binding of the substance with the storage medium.

The storage medium may absorb and store the substance. The storage medium may absorb and store the substance in a liquid substance state. The substance may be absorbed in the storage medium in a solution state in which the substance is solute, and a solvent of the solution may be stored in the storage medium in a dried state. The storage medium may absorb the substance in a liquid substance state and store the substance in a dried state. The substance may be absorbed in the storage medium, frozen, and stored in the storage medium. The substance may be absorbed in the storage medium, lyophilized, and stored in the storage medium. The substance may be dried with a supporting substance to form the storage medium.

The substance may be absorbed and fixed in the storage medium. The state where the substance is fixed may mean a state where the substance is not easily separated from the storage medium due to binding of the substance with a skeletal structure of the storage medium. The substance may be bonded to the storage medium by an electrostatic attractive force.

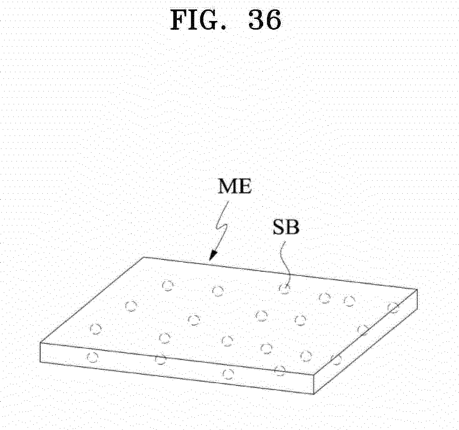

FIG. 36 illustrates an embodiment of a storage medium ME according to the present disclosure. As shown in FIG. 36, the storage medium ME may absorb and store the substance SB. The storage medium ME may adsorb and store the substance SB. The storage medium ME may include a microstructure for generating capillary force. The substance SB may be stored in microcavity formed by a fiber tissue constituting the storage medium ME.

The storage medium may store the substance in a state that the substance is coated on one surface of the storage medium. The storage medium may have one surface on which the substance is coated in a liquid phase. The substance may be coated on one surface of the storage medium in a solution state in which the the substance is solute. The substance may be stored in a dried state of a solvent of the solution. The storage medium may have one surface on which the substance is coated in a liquid phase and dried. The storage medium may have one surface on which the substance is coated in a dried-powder state. The storage medium may have one surface on which the substance is coated in a lyophilized state. The storage medium may have one surface on which the substance is coated and frozen.

The substance may be coated on and fixed in the storage medium. The state where the substance is fixed may mean a state where the substance is not easily separated from the storage medium due to binding of the substance with a surface of the storage medium.

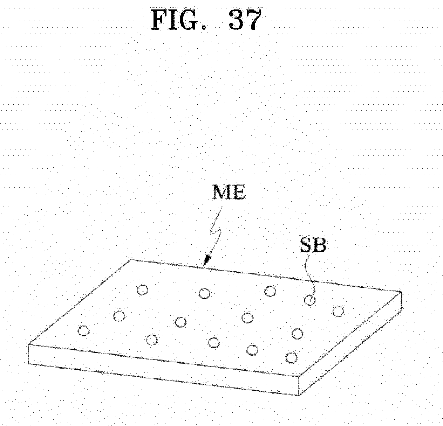

FIG. 37 illustrates an embodiment of a storage medium ME according to the present disclosure. As shown in FIG. 37, the storage medium ME may have one surface coated with the substance SB. The substance SB may be coated on a main surface of the storage medium ME. The substance may be coated on a surface of the storage medium ME. The substance SB may be fixed on one surface of the storage medium ME in a lyophilized state. However, embodiments are not limited thereto. The substance SB may be coated on a plurality of surfaces or the whole surface of the storage medium ME and stored.

By storing the substance in the storage medium of the present disclosure, space for storing the substance may be efficiently operated. The substance may be stored in the patch or stored in a separate storage container until the substance is used in a reaction. However, as the store space for the substance required for the reaction is larger than the needed amount, space utilization efficiency may be poor. In addition, when the substance is transported, substance outflow may occur. When the substance is stored using the storage medium according to the present disclosure, storage density of the substance is improved, and the storage space may be managed more efficiently. Also, by storing the substance using the storage medium, the substance may be safely and conveniently stored.

The storage medium may store various substances. The substance stored in the storage medium may be a hydrophilic substance or a hydrophobic substance. The substance may be fat-soluble or water-soluble.

The storage medium may store a staining reagent. The storage medium may store eosine. The storage medium may store a methylene blue. The storage medium may store a fluorescent staining substance. The storage medium may store a DAPI reagent.

The storage medium may store a nutrient substance. The storage medium may store a nutrient substance for culturing tissues or cells.

The storage medium may store a biological substance. The substance may be an antibody or an antigen. The substance may be a nucleic acid. The storage medium may store a genetic substance. The fine particle may be a probe for detecting a target gene sequence. The fine particle may be an enzyme. The fine particle may be a coenzyme. The storage medium may store a substance for diagnosing a disease.

The storage medium may store a fine particle. The fine particle may be a micro-particle or a nano-particle. The fine particle may be a magnetic bead. The fine particle may be a nano-capsule storing and/or transports a transferring substance. The fine particle may be insoluble in a fat-soluble solution or a water-soluble solution. The fine particle may also refer to a substance in a molecular unit.

### Retaining

The storage medium disclosed in the present disclosure may retain the substance. The storage medium may prevent deterioration of the substance. The storage medium may retain the substance while minimizing the quality change thereof. The storage medium may extend storage duration of the substance. The storage medium may retain the substance so that the storage duration of the substance is extended. The storage medium may extend the retaining duration during which the substance may be used for a desired reaction. The storage medium may retain the substance and maintain the quality of the substance from the time when the substance is stored in the storage medium to the time when the substance is available for a desired reaction.

The storage medium of the present disclosure may maintain the condition that the substance is available for a desired reaction. The storage medium may alleviate quality deterioration of the substance. The storage medium may delay quality deterioration of the substance. The storage medium may extend the duration during which the substance has a state that may be utilized for the reaction.

Hereinafter, the expression that the storage medium "retains" a substance may be interpreted to mean to include the function of the storage medium described above in addition to the delay in the quality change of the substance. The storage medium may "retain" the substance by "storing" substance in the storage medium.

The storage medium may retain the substance in a non-activated state. The non-activated state may be a state in which reactivity of the substance is deteriorated. The non-activated state may be a state in which the degradation rate of the substance is lowered. The non-activated state may be a state in which a function of the substance is deteriorated. The non-activated state may be a state in which liquidity of the substance is deteriorated. The non-activated state may be a state in which mobility of the substance is deteriorated.

The non-activated state may be a state in which the substance may not perform a desired reaction.

For example, the non-activated state may be a dried state of the substance. The non-activated state may be an air-dried or lyophilized state of the substance. The non-activated state may be a state in which a temperature is lower than an activation temperature of the substance. The non-activated state may be a frozen state of the substance. The non-activated state may be a state in which contact with the outside is blocked. The non-activated state may be a state in which the substance is coated.

The storage medium may retain the substance in the storage medium. The storage medium may retain the substance in the skeletal structure that forms the storage medium. The storage medium may retain the substance by fixing the substance to the skeletal structure of the storage medium. As shown in FIG. 36, the substance may permeate into the storage medium and be retained.

The storage medium may retain the substance on a surface of the storage medium. The storage medium may fix the substance on one surface of the storage medium and retain the substance. The storage medium may retain the substance by adsorbing the substance on one surface of the storage medium. As shown in FIG. 37, the substance may be retained by being coated on a surface of the storage medium.

The storage medium may retain various substances. The storage medium may retain a substance that is unfavorable to a long retainment by the patch. For example, a substance retained in the storage medium may be a fluorescence-labeled substance for diagnosis of a biological reagent. The substance retained in the storage medium may be an antibody to diagnose biological reagents. The substance retained in the storage medium may be a substrate to diagnose biological reagents. The substance retained in the storage medium may be a probe to diagnose biological reagents.

In some embodiments of the present disclosure, the storage medium may retain a hydrophilic substance. The storage medium may retain a substance that is soluble in an aqueous solution. For example, the storage medium may retain a compound for controlling a pH. For example, the storage medium may retain a hydrophobic substance. The storage medium may retain a substance that is soluble in the fat soluble solution.

The storage medium may retain a substance for diagnosing a sample. The storage medium may retain a reagent for identifying whether a sample includes a target substance. The substances retained in the storage medium may be a substance to diagnose biological reagents. The substance may be a substance for detecting whether a sample includes a target substance. The substance may be a catalyst for improving a reaction rate.

The substance may be a reagent for identifying whether a sample includes a target substance. The substance may be a marker for detecting a target substance. The substance may be a staining marker, a fluorescent marker, a magnetic marker, or an electrochemical marker.

The substance may be an antibody for detecting a target protein. The substance may be a substrate for detecting a marker.

The substance may retain a marker. The storage medium may retain a staining reagent. The storage medium may retain a biological substance. The substance may be an antibody or an antigen. The substance may be a substrate. The substance may be a nucleic acid. The storage medium may retain a genetic substance. The substance may include a probe for detecting a target gene sequence. The substance may include an enzyme. The substance may include a coenzyme. The storage medium may retain a biological substance. The storage medium may retain a substance for diagnosing a disease.

The storage medium may retain a fine particle. The fine particle may be a micro-particle or a nano-particle. The fine particle may be a magnetic bead. The fine particle may be a nano-capsule storing and/or transports a transferring substance. The fine particle may be insoluble in a fat soluble solution or a water soluble solution. The fine particle may also refer to a substance in a molecular unit.

The storage medium may further include an additional substance to improve retaining efficiency of the substance. The storage medium may further include a storage substance of which quality is required to be maintained and an additional substance to maintain the quality of the storage substance.

The substance that may be stored, retained or transferred by the storage medium disclosed in the present disclosure is not limited to the above examples. A Substance that may be handled by the storage medium may include various substances described in the present specification. However, embodiments are not limited thereto. The storage medium may be used for store and/or transfer of various substances requiring improved retainability.

The storage medium may retain a plurality of kinds of substances. The plurality of kinds of substances may be mixed and retained. The plurality of kinds of substances may be separately retained. The storage medium may retain the first substance and the second substance that react with each other. Here, the first substance and the second substance may be retained in the storage medium in a non-activated state.

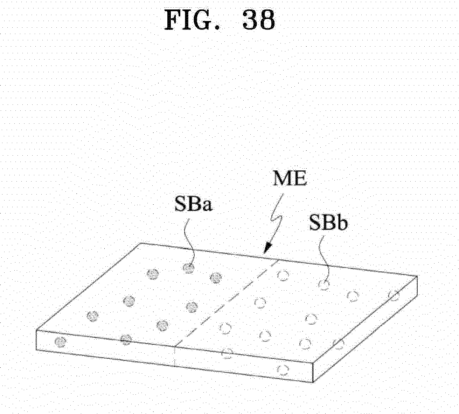

FIG. 38 illustrates an embodiment of a storage medium ME according to the present disclosure. As shown in FIG. 38, the substance SB may be differently retained in the storage medium ME depending on the area. For example, a first area of the storage medium ME may be coated with a first substance SBa. A second area of the storage medium ME may be coated with a second substance SBb. For example, the first area of the storage medium ME may be absorbed with the first substance SBa. The second area of the storage medium ME may be absorbed with the second substance SBb. As shown in FIG. 38, the first substance SBa and the second substance SBb may be separately stored, and thus, a reaction may not occur between the first substance SBa and the second substance SBb in the storage medium ME.

A user may properly select a timing for reaction between the first substance SBa and the second substance SBb.

### Transfer

The storage medium of the present disclosure may be used for transfer of the substance. The storage medium may transfer a substance. The storage medium may store and/or retain a substance and transfer the substance to the outside. The storage medium may transfer the substance to the patch according to the present disclosure. The storage medium may contact the patch and transfer a substance to the patch.

In the present specification, it is understood that the storage medium "transferring" a substance is independent of the "transferring" function of the patch described above. The storage medium transferring a substance and the patch transferring a substance are not the same and may be understood as independent functions. The storage medium and the patch may transfer the substance in a similar mechanism. The transferring of the substance by the storage medium may be understood to include providing the substance by the storage medium.

The storage medium may transfer the substance to a target area. The storage medium may release the substance. The storage medium may provide the substance to an external area. The storage medium may provide an environment in which the substance may migrate to an external area.

The storage medium may transfer the substance to an external area or release the substance by allowing the substance to be dissolved by a solvent provided from the outside. The storage medium may provide an activation temperature of the substance so that the substance is liquefied and provided to the external area. The providing of an environment in which the substance is movable to another area may be providing an environment that allows the substance to thaw.

The transferring of a substance to a target area by the storage medium may mean that when the storage medium contacts the patch and be separated from the target area after a predetermined time, the substance stored in the the storage medium is moved to at least a portion of the target area.

Hereinafter, some examples of the target area will be described for the transfer of substances from the storage medium to the target area.

### Transfer to patch

The storage medium may transfer the substance to the patch according to the present disclosure. The storage medium may contact the patch and transfer the substance to the patch. The storage medium may contact the patch to allow the substance to contact a base substance included in the patch. The storage medium may contact the patch to allow the substance to be dissolved in a base substance included in the patch.

The transferring of the substance to the patch may be diffusion of the substance to the patch.

The patch of the present disclosure may be used with a storage medium for storing a substance. The patch may absorb a substance from a storage medium for storing a substance. The patch may absorb a substance from the storage medium and store the substance in the patch. The patch may store the substance absorbed from the storage medium and transfer the substance. The patch may transfer the absorbed substance from the storage medium to another patch. The patch may transfer the absorbed substance from the storage medium to a reaction area. The patch may provide a reaction space for the substance transferred from the storage medium. The patch may provide a reaction space for the substance absorbed from the storage medium and transfer the substance produced by the reaction to the outside.

The storage medium may transfer a hydrophilic substance to a hydrophilic patch. The storage medium may transfer a hydrophobic substance to a hydrophobic patch. The transferring of the substance to the hydrophilic or hydrophobic patch by the storage medium may be diffusion of the substance to a hydrophilic base substance or hydrophobic base substance respectively included in the hydrophobic or hydrophilic patch.

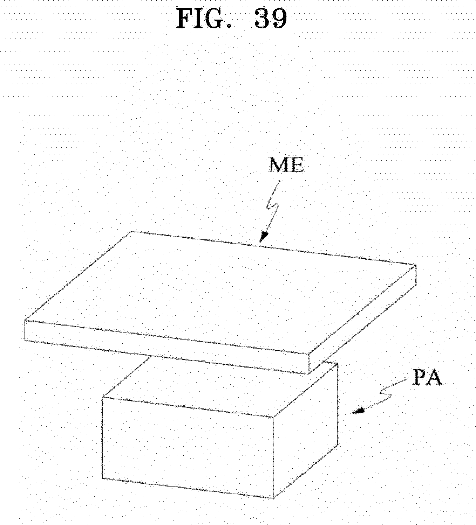

FIG. 39 illustrates a patch PA and a storage medium ME according to an embodiment of the present disclosure. As shown in FIG. 39, the storage medium ME may contact one surface of the patch PA to transfer the substance. The storage medium ME may retain the substance in one surface to be in contact with the patch PA and transfer the substance to the patch PA through the surface to be in contact with the patch PA.

FIG. 39 shows only the case where the storage medium ME contacts an upper surface of the patch PA, but the storage medium ME may transfer the substance to the patch PA by contacting a lower surface or a side surface of the patch PA. The storage medium ME may also transfer the substance to the patch by contacting a plurality of surfaces of the patch PA.

To increase the transfer efficiency of the substance, the contact surface of the patch and the storage medium may be made wider. The contact surface of the patch and the storage medium may be wider than the height of the patch. The storage medium may contact the patch, and the substance may be absorbed in the patch. The storage medium may have a water-soluble or fat-soluble skeletal substance.

The storage medium may store a first substance and a second substance together. The first substance and the second substance may be mixed and stored in a mixed state in the storage medium. The first substance and the second substance may not be mixed and stored in the storage medium. When the first substance and the second substance are mixed and cause an undesired chemical or biological reaction, the storage medium may separately store the first substance and the second substance.

By separately storing the first substance and the second substance in the storage medium, deterioration of a substance due to an undesired reaction may be prevented.

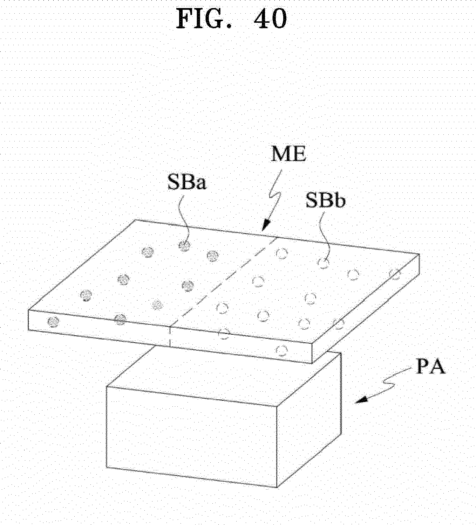

FIG. 40 illustrates a patch PA and a storage medium ME according to an embodiment of the present disclosure. FIG. 40 illustrates an embodiment of the present disclosure, and as described in FIG. 38, FIG. 40 illustrates transferring the first substance SBa and the second substance SBb to the patch PA by using the storage medium ME in which the first substance SBa and the second substance SBb are separately stored. As shown in FIG. 40, the storage medium ME may include a first area storing the first substance SBa and a second area storing the second substance SBb.

As shown in FIG. 40, the storage medium may be contacted with the patch PA such that the first area and the second area may be in contact with the patch PA. The storage medium ME may transfer the first substance SBa and the second substance SBb to the patch PA when the first area and second area contact the patch PA. The first substance SBa and the second substance SBb may be transferred to the patch PA to perform a chemical or biological reaction.

The storage medium ME is may be contacted with the patch PA such that the first area may be contacted with the patch PA, while the second area may not be contacted with the patch PA. The storage medium ME may be contacted with the patch PA such that the first area may be contacted with the patch PA to transfer the first substance SBa to the patch PA, while the second substance SBb may not be transferred. By sequentially contacting the first area and the second area with the patch PA, the first substance SBa and the second substance SBb may be sequentially transferred to the patch PA.

FIG. 40 only shows the storage medium ME including the first area and the second area, however, the storage medium ME may include a plurality of areas, and each area may include different substances.

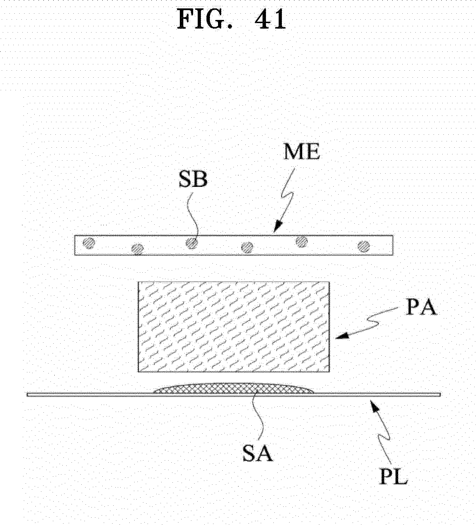

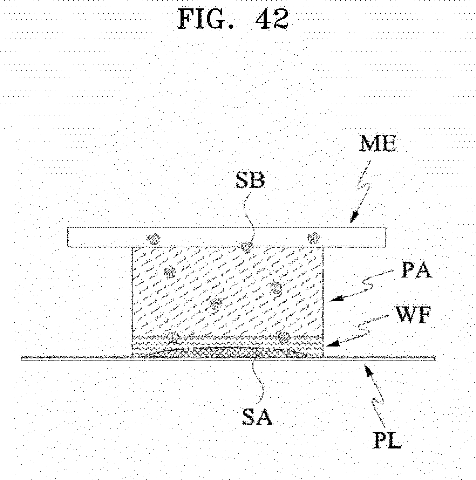

FIGS. 41 and 42 each illustrate A method of inspecting a sample by using a patch PA, a storage medium ME, and a plate PL according to an embodiment of the present disclosure.

As shown in FIG. 41, one surface of the storage medium ME storing the substance SB to be transferred to the patch PA may be disposed to face the patch PA. The storage medium ME may be disposed on the patch PA. As shown in FIG. 41, an upper surface of the patch PA may face the storage medium ME, and a lower surface of the patch PA may face a sample SA. As shown in FIG. 41, the plate PL may face a lower surface of the patch PA and may include a reaction area on which the sample SA is located.

As shown in FIG. 42, a lower surface of the storage medium ME may contact the patch PA. When the lower surface of the storage medium contacts the patch PA, the storage medium may transfer a substance to the patch PA. As shown in FIG. 42, the patch PA may contact the sample SA. The contacting of the patch PA with the sample SA the patch PA may be to a sufficient degree to form a water film WF in the reaction area on which the patch PA is located.

The patch may provide the substance SB received from the storage medium ME in the reaction area where the sample SA is located. As shown in FIG. 42, the patch PA may transfer the substance SB received from the storage medium ME to the reaction area via the water film WF.

The patch PA may contact the reaction area in contact with the storage medium ME. An upper surface of the patch PA may be contacted with the storage medium ME such that the patch PA may absorb the substance SB from the storage medium ME, and then the patch PA may be separate from the storage medium ME. The patch PA may provide the substance SB in the reaction area by contacting a lower surface with the sample SA after being separated from the storage medium ME.

The storage medium ME may retain the first substance and the second substance that react with each other. Here, the first substance and the second substance may be retained in the storage medium ME in a non-activated state. The first substance and the second substance may be transferred to the patch PA. The first substance and the second substance may react in the patch PA.

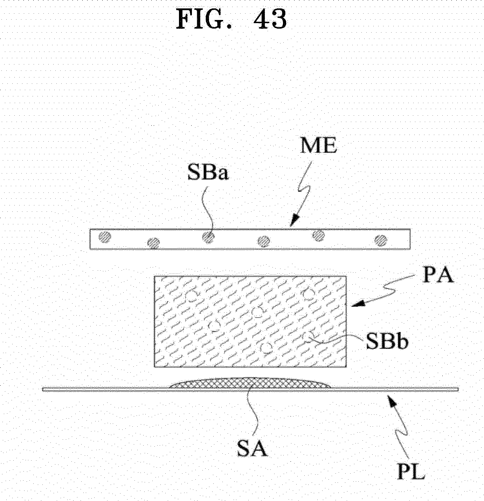

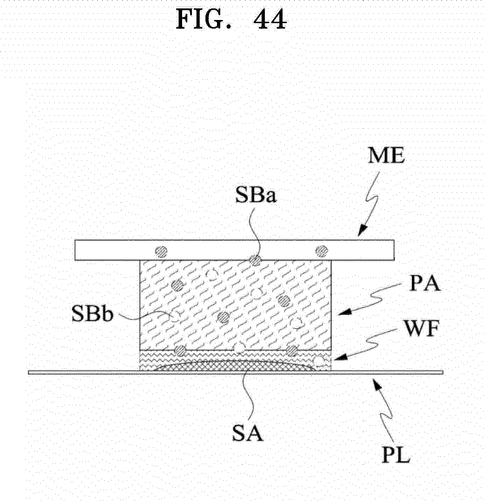

FIGS. 43 and 44 each illustrate A method of inspecting a sample by using a patch PA, a storage medium ME, and a plate PL according to an embodiment of the present disclosure.

As shown in FIG. 43, the storage medium may store a first substance. The first substance may be in a non-activated state. The patch may store a second substance. The patch may store a second substance that provides an active condition to the first substance. The patch may store a second substance that reacts with the first substance.

As shown in FIG. 44, the storage medium may contact an upper surface of the patch to transfer the first substance to the patch. The patch may contact the sample to provide the first substance and/or the second substance to a reaction area on which the sample is located. FIG. 44 only illustrates a case where the first substance and/or the second substance are provided to the reaction area, however, embodiments of the present disclosure are not limited thereto. The patch may provide the first substance activated by the second substance to the reaction area. The patch may provide a reaction product produced by a reaction of the first substance and the second substance to the reaction area.

When a sample is inspected using the first substance and the second substance, as shown in FIGS. 43 and 44, the first substance and the second substance may be stored separately in the patch and the storage medium, respectively. By separately storing the first substance and the second substance, the first substance and the second substance may be stored for a long period. Thus, a problem of the first substance reacting with the second substance may be resolved wherein the problem causes the first substance and the second substance to be unavailable in inspection of the sample. In this case, a substance that is more difficult to retain may be stored in the storage medium.

### Transfer to reaction area

The storage medium may provide the substance to a reaction area. The reaction area may be on a plate. In the reaction area, a sample to be diagnosed may be located. The providing of the substance to the reaction area by the storage medium may be allowing the substance to be movable to the reaction area by contacting the storage medium with the reaction area.

The storage medium may transfer the substance to the reaction area with the patch of the present disclosure. The storage medium may transfer the substance to the patch to provide the substance to the reaction area by contacting the patch with the sample located on the reaction area.

The storage medium may transfer the substance to a reaction area. The storage medium may contact the reaction area to transfer the substance to the reaction area. The reaction area may be on a plate. The reaction area may be coated with a reaction substance that reacts with the substance. In the reaction area, a sample to be inspected by the substance may be located.

The storage medium may contact the patch to transfer the transferring substance to the reaction area. The storage medium may contact the patch to activate a substance stored in the storage medium and transfer the activated substance to the reaction area. The storage medium may contact the patch and the reaction area to receive a base substance from the patch and to provide the substance to the reaction area. For example, one surface of the storage medium may contact the patch to absorb a base liquid from the patch and provide the substance activated by the base liquid to the reaction area.

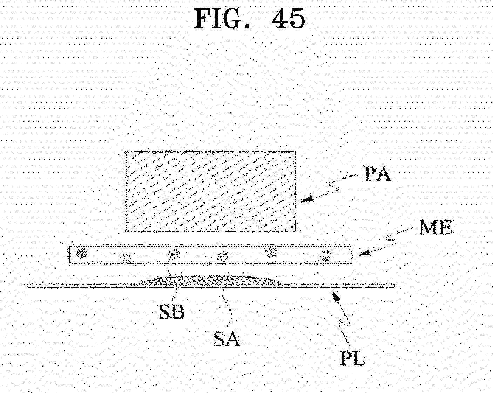

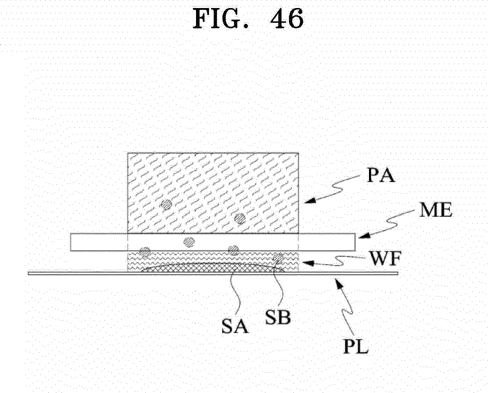

FIGS. 45 and 46 each illustrate A method of inspecting a sample by using a patch PA, a storage medium ME, and a plate PL according to an embodiment of the present disclosure.

As shown in FIG. 45, the patch PA may be disposed on the storage medium ME. An upper surface of the storage medium ME may face the patch PA. A lower surface of the storage medium ME may face the plate PL. The storage medium ME may store an inspection reagent SB for inspecting a sample located on the plate PL. The storage medium may absorb and store the reagent SB. The plate PL may include a reaction area on which a sample is located.

As shown in FIG. 46, the patch PA may contact an upper surface of the storage medium ME. The upper surface of the storage medium ME may contact the patch PA. The lower surface of the storage medium ME may contact the sample SA. The upper surface of the storage medium ME may contact the patch PA, while the lower surface of the storage medium ME is in contact with the sample SA. The lower surface of the storage medium ME may contact the sample SA, while the upper surface of the storage medium ME is in contact with the patch PA. The reagent SB stored in the storage medium ME may receive an active condition when the storage medium ME contacts the patch PA.

The reagent SB may be accommodated in the base substance stored in the patch PA when the storage medium ME contacts the patch PA. When a lower surface of the storage medium ME contacts the sample SA, and an upper surface of the storage medium ME contacts the patch PA, a base substance stored in the patch PA may migrate to the storage medium ME. The base substance stored in the patch PA may migrate to the reaction area. The base substance may form the water film WF in the reaction area. When the reagent SB may be provided to the reaction area via the water film WF upon contact of the storage medium ME with the sample SA.

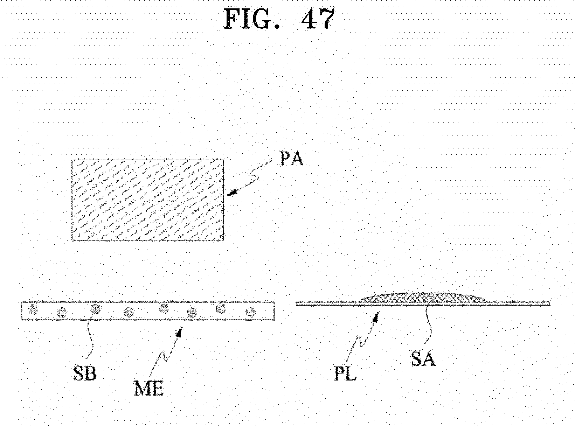

FIGS. 47 to 49 each illustrate A method of inspecting a sample by using a patch PA, a storage medium ME, and a plate PL according to an embodiment of the present disclosure.

As shown in FIG. 47, the patch PA may be disposed on the storage medium ME.

The patch PA may be prepared at a predetermined distance from the storage medium ME in a direction, e.g., in a vertical direction.

An upper surface of the storage medium ME may face the patch PA. The storage medium ME may store an inspection reagent SB for inspecting a sample located on the plate PL. The reagent SB may be stored in the storage medium ME by absorption or coating. The storage medium ME may be located on the same plane with the plate PL. FIG. 47 illustrates that the storage medium ME stores the reagent SB by absorption, however, the storage medium ME may have one surface on which the reagent SB is coated in a dried state for storage.

The plate PL may include a reaction area on which a sample is located. The plate PL may be disposed on the same plane with the storage medium ME. The plate PL may be disposed at a predetermined distance from the storage medium ME in a direction, e.g., in a horizontal direction.

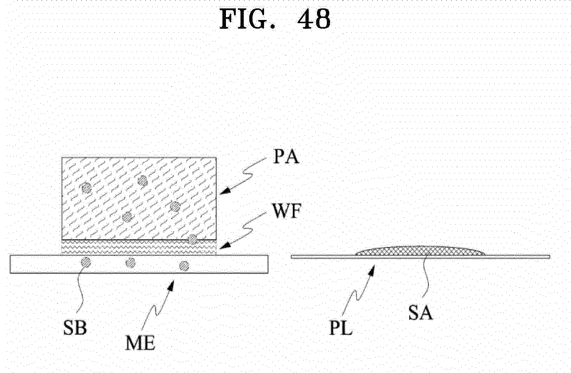

As shown in FIG. 48, the patch PA may contact the storage medium ME and absorb the reagent SB stored in the storage medium ME. When the patch PA contacts the storage medium ME, the water film WF may be formed in the contact area of the patch PA and the storage medium ME. The reagent SB may be transferred from the storage medium ME to the patch PA via the water film WF.

The patch PA may be separated from the storage medium ME. When the patch PA is separated from the storage medium ME, the reagent SB may be received in an activated state. The patch PA may provide a reaction space for the reagent SB.

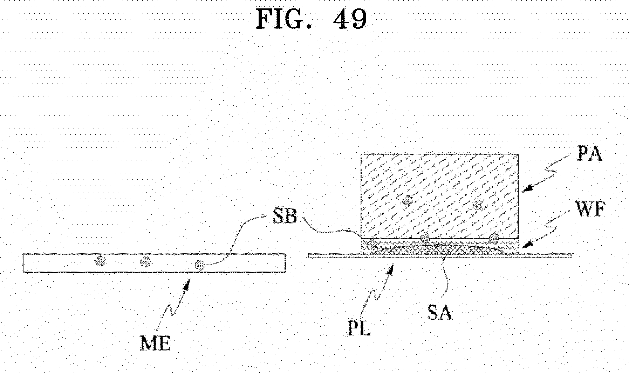

As shown in FIG. 49, the patch PA may contact the plate PL. The patch PA may contact the sample SA. When the patch PA contacts the plate PL, the reagent SB may be provided to the reaction area on which the sample SA is located. When the patch PA contacts the plate PL, the water film WF may be formed in the contact area. The reagent SB may be provided to the reaction area via the water film WF.

As shown in FIGS. 47 to 49, when the sample SA is inspected by sequentially contacting one surface of the patch PA with the storage medium ME and the sample SA, a transferring rate of the reagent SB stored in the storage medium ME may be improved.

In particular, when the patch PA contacts the storage medium ME, the patch PA may absorb the reagent SB via the contact surface. Here, the reagent SB may be diffused from the contact surface into the interior of the patch PA. When the patch PA contacts the sample SA through the contact surface that was contacted with the storage medium ME, the reagent SB may be provided to the sample SA even before the reagent SB is evenly diffused inside the patch PA.

Here, regarding FIGS. 41 to 49, embodiments of the substance transfer method using the storage medium has been described . However, the embodiments of the substance transfer method described above may be implemented by an inspection module or inspection device described below.

The patch may absorb a substance from the storage medium and store the substance in the patch. The absorbing of the substance from the storage medium by the patch may result from the patch contacting the storage medium followed by diffusion of the substance from the storage medium into the patch. The absorbing of the substance from the storage medium by the patch may be absorption of the substance released from the storage medium by the patch due to application of a predetermined pressure on the storage medium, followed by contacting of the patch with the storage medium. The absorbing of the substance from the storage medium by the patch may be absorption of the substance by the patch due to migration of the substance from the storage medium to the patch due to application of an electric field to the storage medium and the patch, followed by contacting of the patch with the storage medium.

### Activation of substance

The storage medium may store the substance in a non-activated state. The substance may be activated when moved to the outside. The substance may be activated in the storage medium. The substance may be activated upon inflow of an external substance. When an active condition is provided to the substance, the substance may be activated. Hereinafter, activation of a substance retained or stored in the storage medium will be described.

The storage medium may transfer the substance to the outside so that the substance has an active condition. The storage medium may transfer the substance to a patch so that the substance is activated. In other words, the patch may impart an active condition to the substance. The patch may receive the substance and accommodate the substance in an activated state. The imparting of an active condition to the substance may be activation of the substance by using a base substance stored in the patch.

The active condition of the substance may refer to a condition where the substance is in a state where it may perform the desired reaction. The active condition of the substance may be a pH condition in which the substance may perform a reaction. The active condition of the substance may be a temperature condition in which the substance may perform a reaction. The active condition of the substance may be a phase condition in which the substance may perform a reaction.

For example, the substance may be stored in a non-activated state as a solid phase and may be received in an activated state as a liquid phase.

The desired reaction may be a reaction to perform inspection. The desired reaction may be a reaction to perform a diagnosis using a sample. The desired reaction may be a reaction to inspect whether the sample includes a target substance. The desired reaction may be a reaction to inspect whether the sample contains a target protein. The desired reaction may be a reaction to inspect whether the subject of inspection is infected with a target disease.

The substance may be accommodated in the patch in an activated state. The substance may be activated by a substance stored in the patch. The substance stored in the patch may provide an active condition to the substance transferred from the storage medium. The substance stored in the patch may provide an active condition to the base substance transferred from the storage medium. The additive substance stored in the patch may provide an active condition to the substance transferred from the storage medium. For example, the substance may be accommodated in an activated state in a base substance stored in microcavities of the patch, and the activated state may be a state of a liquidity.

### Shape and structure of storage medium

Hereinafter, the structure, and the like of the storage medium according to the present disclosure the shape will be described in detail.

The storage medium disclosed in the present disclosure may be provided in a flat shape. For example, the storage medium may be provided in a sheet shape. The storage medium may be provided in a film shape. The storage medium may be provided in a plate shape. The storage medium may be provided in a planar shape.

The storage medium disclosed in the present disclosure may include a supporting structure. The storage medium may store or retain a substance by using the supporting structure. The storage medium may include a fibric structure. The storage medium may include a water-soluble skeletal substance. The storage medium may include a fat-soluble skeletal substance. The storage medium may be provided in a sheet shape including a fabric structure.

The storage medium may include a fabric structure. The storage medium may have a structure formed by microfibers that are interwined. The fabric structure may have a fibril structure. The fabric tissue may be a cellulose tissue. The storage medium may be a paper.

The storage may be a nitrcocellurose membrane.

The fabric structure may be natural or synthetic.

The storage medium may include at least one functional surface. The functional surface may be a coating surface, an absorption surface, a contact surface, a transfer surface, or the like.

One surface of the storage medium may be coated with a substance. The surface of the storage medium coated with a substance may be a coating surface. The storage medium may have a coating surface as a storing surface for storing a substance. The substance may be prepared to be fixed on the coating surface. The coating surface may be coated with the substance. The substance on the coating surface may be air-dried.The substance on the coating surface may be lyophilized. The coating surface may be coated with a lyophilized substance. The coating surface may include the fabric structure. The storage medium may be provided in a plate shape and include the coating surface.

A substance may be absorbed via one surface of the storage medium. One surface of the storage medium that absorbs the substance may be an absorption surface. The storage medium may have an absorption surface as a storing surface for storing a substance. The storage medium may include an absorption surface that absorbs the substance. The absorption surface may absorb and store the substance. The absorption surface may contact a substance to absorb the substance and store the dried substance. The absorption surface may adsorb and store the substance. The absorption surface may include the fabric structure. The storage medium may be provided in a sheet shape and include the absorption surface.

The storage medium may contact a target area via one surface. The storage medium may include a contact surface. The storage medium may contact the target area via the contact surface. The storage medium may provide a reagent stored in the storage medium to a target area via a contact surface. The storage medium may transfer a substance via one surface. The storage medium may transfer a substance to a target area via the contact surface. The contact surface may include the fabric structure.

The surfaces that may be included in the storage medium are not necessarily separately present. One surface may serve as at least two functional surfaces. For example, a coating surface coated with the substance may contact the target area. An absorption surface absorbing the substance may be a contact surface that contacts the target area. The contact surface may be the same functional surface as the coating surface. When the storage medium is not permeable, the coating surface may be implemented with the same functional surface as the contact surface. The absorption surface may be the same functional surface as the contact surface. The storage medium may have one surface that absorbs and stores the substance and transfers the substance to the target area.

In addition, the storage medium may include a plurality of functional surfaces. For example, the storage medium may include a plurality of contact surfaces. The storage medium may include a plurality of coating surfaces. The storage medium may include a plurality of absorption surfaces.

The storage medium may be prepared as a single use product. The storage medium may be prepared by using cellulose. The storage medium may be prepared as a multi-use product. The storage medium may be prepared by using polymer.

After transferring the substance to the target area, the shape of the storage medium may be maintained. After transferring the substance to the target area, the shape of the storage medium may be deformed. When the storage medium contacts the target area, the shape of the storage medium may be deformed.

The storage medium may contact the patch to be absorbed in the patch. The storage medium may contact the patch to be dissolved. The storage medium may contact the patch to be decomposed. The storage medium may have a water-soluble supporting structure.

### Use of storage medium

Hereinafter, methods for storing and transferring substances using the patch and storage medium disclosed in the present disclosure are described.

### Use of a plurality of media

The storage medium disclosed in the present disclosure may be provided in a plural number. According to an embodiment of the present disclosure, a plurality of storage media may be used to transfer a substance to a target area. The plurality of storage media may each store different substances. The plurality of storage media may each store the same substance.

The plurality of storage media may be used in strategic transfer of a substance. For example, the plurality of the storage media may be used in sequentially transferring a plurality of kinds of substances. The plurality of storage media may be used in transferring the plurality of kinds of substances to a target area. The plurality of storage media may be used to sequentially generate a desired reaction in the target area, in consideration of reactivity of a plurality of kinds of substances. The plurality of storage media may also be used to incrementally transfer an amount of a substance.

Hereinafter, a case of using a plurality of the storage media will be described.

The target area to which the plurality of storage media transfer the substance may be the patch disclosed in the present disclosure. a first storage medium for storing a first substance and a second storage medium for storing a second substance may be transferred to the patch. The patch may absorb the first substance from the first storage medium and absorb the second substance from the second storage medium. The patch may store the first substance absorbed from the first storage medium and the second substance absorbed from the second storage medium.

The plurality of storage media may transfer the substance to the patch so that a desired reaction is performed in the patch. In the patch, substances that are absorbed from the medium and reaction substances that react may be pre-stored. When the patch receives a substance from the storage medium, the pre-stored reaction substance and the substance received from the storage medium may react in the patch.

The patch may transfer the product generated by the reaction to the outside.

The plurality of storage media may transfer the substance sequentially to the patch. For example, when some of the first substance is transferred from the patch to the first storage medium, the second substance may be transferred from the second storage medium to the patch. When the first storage medium for storing the first substance contacts the patch and be separated from the patch after a predetermined time, the second storage medium for storing the second substance may then contact the patch to transfer the second substance to the patch.

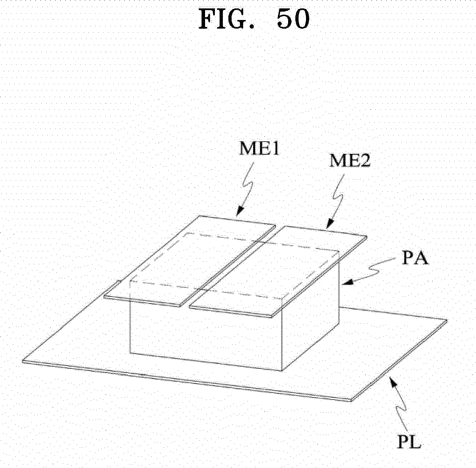

FIG. 50 is a schematic view of an embodiment of transfer of a substance by using a plurality of storage media according to the present disclosure. As shown in FIG. 50, a sample located on the plate may be inspected by using the plurality of storage media. As shown in FIG. 50, a substance may be transferred to the patch PA according to the present disclosure by using a first storage medium ME1 and a second storage medium ME2.

The first storage medium ME1 and the second storage medium ME2 may respectively retain the first substance and the second substance that react with each other. The first substance may be retained in the first storage medium ME1 in a non-activated state. The second substance may be retained in the second storage medium ME2 in a non-activated state. As the first storage medium ME1 and the second storage medium ME2 contacts the patch PA, the first substance and the second substance may be transferred to the patch PA. The first substance and the second substance may be activated in the patch PA. The first substance and the second substance may react in the patch PA. The first storage medium ME1 and the second storage medium ME2 may sequentially contact the patch PA.

FIG. 50 illustrates a case where the first storage medium ME1 and the second storage medium ME2 contacts an upper surface of the patch PA to transfer a substance. However, embodiments of the present disclosure are not limited thereto. For example, the first storage medium ME1 and the second storage medium ME2 may contact a lower surface of the patch PA. When the first storage medium ME1 and the second storage medium ME2 contacts a lower surface of the patch PA and a sample located on the plate, a substance may be provided to a reaction area on which the sample is located.

Also, FIG. 50 illustrates a case where the first storage medium ME1 and the second storage medium ME2 contacts the same surface of the patch PA to transfer a substance, however, embodiments of the present disclosure are not limited thereto. According to the present disclosure, the first storage medium ME1 and the second storage medium ME2 may each contact different surfaces of the patch PA to transfer a substance. For example, the first storage medium ME1 may contact an upper surface of the patch PA, and the second storage medium ME2 may contact a side surface of the patch PA to transfer a substance.

### Use of a plurality of patches

According to an embodiment of the present disclosure, the storage medium disclosed in the present disclosure may transfer a substance to a plurality of patches. A plurality of patches may absorb a substance from the storage medium.

The storage medium may transfer a substance to a plurality of patches. The storage medium may contact a plurality of patches and transfer a substance to the plurality of patches. The storage medium may contact the plurality of patches simultaneously. The storage medium may contact the plurality of patches sequentially.

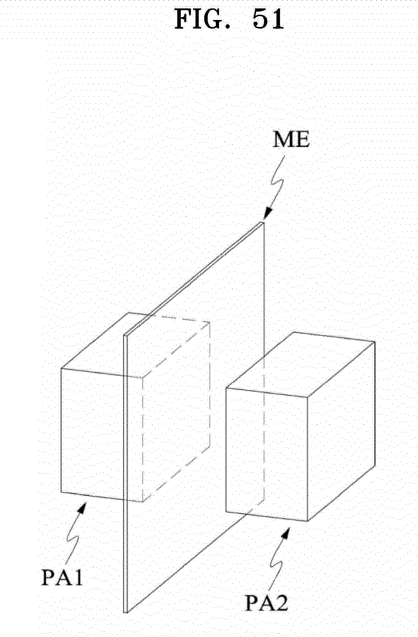

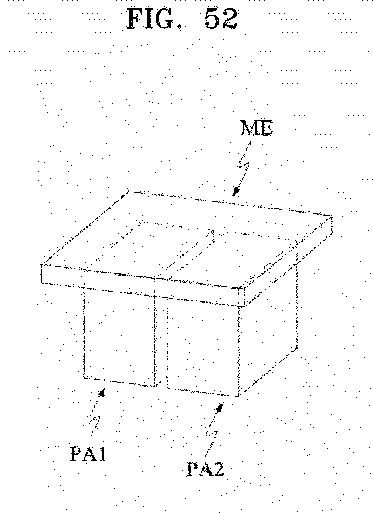

FIGS. 51 and 52 are each a schematic view of an embodiment of transfer of a substance by using a plurality of patches PA. As shown in FIGS. 51 and 52, a sample located on a plate may be inspected by using the plurality of patches PA and the storage medium. As shown in FIGS. 51 and 52, the storage medium ME may transfer a substance to a first patch PA1 and a second patch PA2. When the storage medium ME contacts the first patch PA1 and the second patch PA2, a substance stored in the storage medium ME may be activated. The storage medium ME may store the substance in a non-activated state and transfer the substance to the the first patch PA1 and the second patch PA2. As shown in FIG. 51, the storage medium ME may have a pair of contact surfaces that each contact the first patch PA1 and the second patch PA2. As shown in FIG. 52, the storage medium ME may have one contact surface that contacts the first patch PA1 and the second patch PA2.

Hereinafter, embodiments in which a plurality of the storage media and/or a plurality of the patches according to the present disclosure will be described.

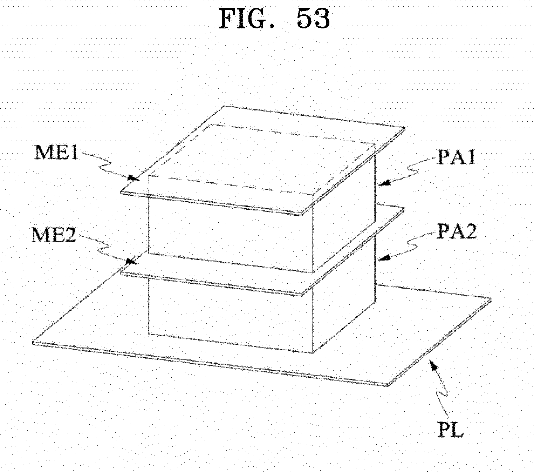

FIG. 53 is a schematic view of an embodiment of transfer of a substance by using a plurality of storage media and a plurality of patches according to the present disclosure. As shown in FIG. 53, a sample located on the plate may be inspected by using the plurality of patches and the plurality of storage media.

As shown in FIG. 53, the first storage medium ME1 may transfer a first substance to the first patch PA1. The first patch PA1 and the second storage medium ME2 may contact each other to provide the first substance to the second storage medium ME2. The second storage medium ME2 may contact the first patch PA1 and the second patch PA2 to provide a second substance to the second patch PA2. The second patch PA2 may contact a sample located in the plate PL to provide the second substance. The second patch PA2 may provide a first substance to a reaction area on which the sample is located. The second patch PA2 may provide a product produced by a reaction between the first substance and the second substance to the reaction area.

FIG. 53 illustrates a case where the first storage medium ME1, the first patch PA1, the second storage medium ME2, and the second patch PA2 are in contact, however, the first storage medium ME1, the first patch PA1, the second storage medium ME2, and the second patch PA2 may be sequentially contacted.

For example, when the first storage medium ME1 contacts the first patch PA1, the first patch PA1 may contact the second storage medium ME2. When the first patch PA1 contacts the second storage medium ME2, the second storage medium ME2 may contact the second patch PA2.

FIG. 53 illustrates a case where the storage medium transfers a substance to the patch, however, embodiments of the present disclosure are not limited thereto. When the first storage medium ME1 or the second storage medium ME2 contacts a sample located on the plate PL, a substance may be provided to a reaction area on which the sample is located.

### Examples

The storage medium and the patch may be used to inspect a sample. The storage medium and the patch may be used to diagnose a sample. The storage medium and the patch may be used to diagnose a target disease.

According to an embodiment of the present disclosure, the storage medium may be used to transfer a substance needed for inspection of a sample to the patch, and the patch may be contacted with the sample to inspect the sample.

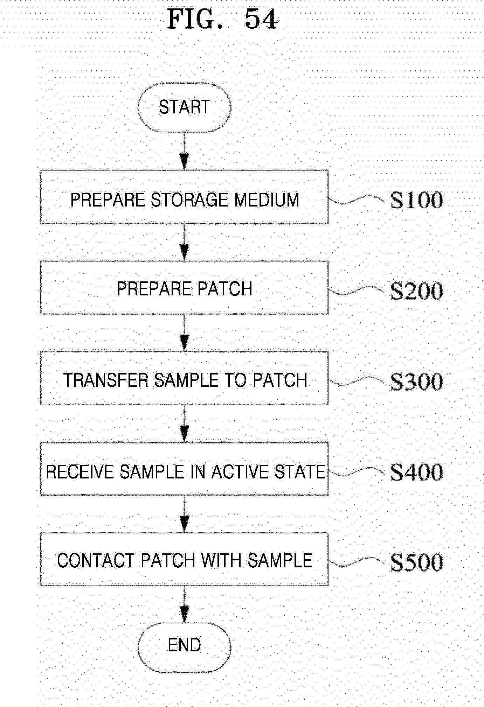

FIG. 54 is a flowchart for illustrating A method of inspecting a sample according to an embodiment of the present disclosure. As shown in FIG. 54, an inspection method according to an embodiment of the present disclosure may include a step S100 of preparing a storage medium, a step S200 of preparing a patch, a step S300 of transferring a reagent to the patch, a step S400 of accommodating the reagent put into an activated state, and a step S500 of contacting the patch with a sample.

The step S100 of preparing a storage medium may include preparing a storage medium for storing a reagent in a non-activated state, wherein the reagent is used to inspect whether the sample includes a target substance. The storage medium may store the reagent in an air-dried state. The storage medium may store the reagent that is lyophilized. The storage medium may include one surface coated with the reagent. The storage medium may store the reagent in a state in which the reagent may not inspect whether the sample includes a target substance.

The step S200 of preparing a patch may include preparing a patch on a side of the storage medium, the patch including a network structure in which multiple microcavities are formed and a base substance stored in the microcavities and imparting an active condition to the reagent. The polarity of the base substance may be similar to that of the reagent. The base substance may be an aqueous solution.

The step S300 of transferring a reagent to the patch may include contacting the storage medium with a surface of the patch on the side of the storage medium for transferring a portion of the reagent stored in the storage medium to the patch. The reagent may be diffused to a base substance included in the patch. The reagent may be captured by the base substance and moved to the patch.

The step S400 of accommodating the reagent put into an activated state may include accommodating the transferred reagent put into an activated state by the base substance in the patch. The patch may accommodate the reagent in a state in which the reagent may inspect whether the sample includes the target substance. The patch may accommodate the reagent in a dissolved state. The patch may accommodate the reagent dissolved in the base substance. The patch may accommodate the reagent diffused in the base substance.

The step S500 of contacting the patch with a sample may include contacting the patch with the sample to provide the reagent to a reaction area on which the sample is located. The patch may be contacted with the sample such that a water film may be formed by the base substance in a reaction area on which the sample is located. The patch may provide the reagent diffused in the base substance to the reaction area with the base substance. The patch may provide the reagent dissolved in the base substance to the reaction area.

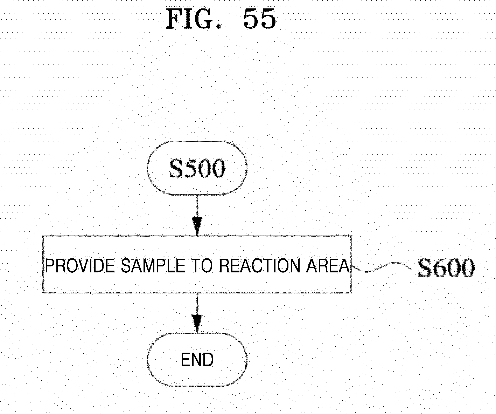

FIG. 55 is a flowchart for illustrating A method of inspecting a sample according to an embodiment of the present disclosure. As shown in FIG. 55, the inspection method according to an embodiment of the present disclosure may further include a step S600 of providing a reagent to a reaction area, in addition to the inspection method shown in FIG. 54.

The step S600 of providing a reagent to a reaction area may be providing the reagent to a reaction area on which the sample is located. Here, the providing of a reagent to a reaction area may be performed after the step S500 of contacting the patch with a sample. The reagent may be transferred to the patch, and the patch may provide the reagent to a reaction area by contacting the sample. The patch may contact the sample, and then, the patch may contact the storage medium to receive a reagent from the storage medium to provide the reagent to the reaction area.

The inspection method may include contacting the patch with the sample and contacting the patch with the storage medium such that the reagent may be transferred from the storage medium via the patch to provide the reagent to the reaction area.

According to another embodiment of the present disclosure, a storage medium for storing a substance for inspecting a sample may be contacted with the sample, and then, the patch may be contacted with the storage medium to perform inspection of the sample.

According to another embodiment of an inspection method of the present disclosure, the inspection method may include preparing a storage medium, preparing a patch, activating a reagent, and contacting the sample with the storage medium.

The preparing of the storage medium and the preparing of the patch may be prepared in a similar manner as in the foregoing examples.

The activating of a reagent may include activating the reagent by contacting the storage medium with a surface of the patch on the side of the storage medium. The activating of a reagent may include providing mobility to the reagent. The activating of a reagent may include providing an environment that allows the reagent to inspect the target substance. The activating of a reagent may include dispersing the reagent by capturing the reagent in a base substance of the patch.

The contacting of the storage medium with a sample may include contacting another surface of the storage medium on a side of the sample with the sample to provide the reagent to a reaction area on which the sample is located.

The inspection method according to an embodiment may further include providing the reagent to a reaction area, on which the sample is located, and the reagent may be provided in an activated state. Here, the providing of the reagent to the reaction area may include providing the reagent put into an activated state by the base substance to the reaction area. In other words, while one surface of the storage medium is in contact with the patch, the other surface may be contacted with the sample to provide the activated reagent to the reaction area by the patch.

The activating of the reagent may include activating the reagent stored in the storage medium of which the other surface is in contact with the sample. In other words, in the inspection method, one surface of the storage medium may be contacted with the sample, and then the other surface of the storage medium may be contacted with the patch to provide the reagent to the sample.

According to another embodiment of the present disclosure, a gel patch for inspecting a sample may be prepared, the patch may be contacted with a storage medium, the patch may be contacted with the sample to inspect the sample. The inspection method may include preparing a patch, preparing a storage medium; contacting the patch with the storage medium to transfer the reagent to the patch; accommodating the reagent put into an activated state by the base substance in the patch; and contacting the patch with the sample to provide the reagent to the reaction area on which the sample is located.

According to another embodiment of an inspection method of the present disclosure, the inspection method of a sample using a patch and a storage medium may be include contacting a storage medium with the patch for storing a substance to transfer a substance stored in the storage medium to the patch, providing the substance transferred to patch with an active condition by the patch, and providing the substance to a reaction area.

The preparing of a patch may include preparing a patch including a base substance that activates a reagent and a network structure in which multiple micropores are formed, wherein the multiple micropores may store the base substance, and being capable of absorbing a substance from an external area or providing a substance to an external area.

The inspection method according to an embodiment may further include providing the reagent to the reaction area by the patch, and the providing of the reagent to the reaction area may include providing the reagent accommodated in an activated state by the base substance to the reaction area. In other words, in the inspection method, the reagent stored in the storage medium may be stored in the patch, and the reagent may be transferred to the sample.

### Inspection module

### Feature of Inspection module

An inspection module for inspecting a sample by using the patch and the storage medium disclosed in the present disclosure is described hereinafter. In the inspection module, the storage medium, the patch, and the reaction area may be connected to one another to allow substance migration.

The inspection module may diagnose a sample. The sample may be a biological sample for diagnosing a target disease. The inspection module may perform immunologic, hematological, genetic, or histological diagnosis of the sample.

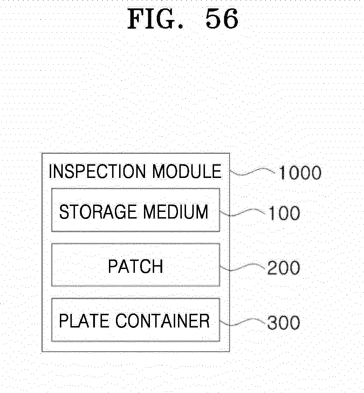

FIG. 56 illustrates an embodiment of an inspection module according to the present disclosure. As shown in FIG. 56, the inspection module according to an embodiment of the present disclosure may include a storage medium 100, a patch 200, and a plate container 300.

The storage medium 100 may be detached from the inspection module. The storage medium 100 may be prepared outside of the inspection module. The storage medium 100 may be prepared as a single use product.

The storage medium 100 may store a reagent for inspecting whether the sample includes a target substance. The storage medium 100 may store a reagent in a non-activated state for inspecting whether the sample includes a target substance. The storage medium 100 may include a storing surface storing the reagent in a non-activated state and a patch contact surface that may contact the patch for activating the reagent. Here, the patch may activate the reagent by the base substance, upon contact with storage medium, and may provide the activated reagent to a reaction area on which a sample to be inspected is located.

The storage medium 100 may store the reagent that is lyophilized.

The storage medium 100 may provide the activated reagent to the reaction area upon contact with the sample, while in contact with the storage medium. The storage medium 100 may contact the patch 200, while the storage medium 100 is in contact with the sample.

The storage medium 100 may further include a sample contact surface that contacts the sample. Here, the providing of the activated reagent to the reaction area by the patch may include providing the activated reagent through the sample contact surface of the storage medium to the reaction area.

The patch 200 may be detached from the inspection module. The patch 200 may be prepared outside of the inspection module. The patch 200 may be prepared as a single use product.

The patch 200 may be provided as a patch in gel phase for inspecting a sample. The patch 200 may contact the sample to provide the reagent put into an activated state to the reaction area. The patch 200 may include a base substance providing a reagent with an active condition, the base substance being for identifying whether the sample includes a target substance; a network structure in which multiple microcavities are formed, the multiple microcavities storing the base substance; and a medium contact surface that contacts a storage medium in which the reagent is stored in a non-activated state. The patch may receive the reagent from the storage medium and accommodate the reagent put into an activated state by the base substance.

The patch 200 may provide the reagent to the reaction area upon contact with the sample, while in contact with the storage medium 100. The patch 200 may accommodate the reagent put into an activated state upon contact with the storage medium, while in contact with the sample, and may provide the reagent to the reaction area. The patch 200 may contact the storage medium to activate the reagent and may induce transfer of the activated reagent to the plate.

The patch 200 may further include a sample contact surface that contacts the sample and provides the reagent to a reaction area on which the sample is located. The medium contact surface may be opposite the sample contact surface. The medium contact surface may be identical to the sample contact surface.

The plate container 300 may contain a plate. The plate container 300 may contain a single use plate. The plate may be prepared outside of the inspection module. The plate may be detached from the inspection module.

The patch 200 may include a network structure forming multiple micropores and a base substance imparting an active condition to the reagent, wherein the patch accommodates the reagent put into an activated state upon contact with the storage medium.

The plate container 300 may contain a plate including a reaction area on which the sample is located and to which the reagent put into an activated state is provided.

As shown in FIG. 56, a case of the inspection module including the storage medium 100, the patch 200, and the plate container 300 is described; however, embodiments of the present disclosure are not limited thereto. The inspection module may include a storage medium container instead of the storage medium 100. The inspection module may include a plate in which a sample is located instead of a plate container 300.

### Operation of Inspection module

The inspection module of the present specification may operate diagnosis of a sample. Hereinafter, operation of inspection of an inspection module including the storage medium 100, the patch 200, and the plate container 300.

The storage medium 100 may store the reagent in a non-activated state and may be located on a side of the first main surface of the patch. The storage medium 100 may be spaced upwards with respect to the patch. The storage medium 100 may be moved downwards with respect to the patch. As the storage medium 100 contains a storage medium container, as the storage medium container is moved downwards, the storage medium 100 may be moved downwards towards the patch side.

The patch 200 may have a first main surface that may receive a reagent, wherein the reagent is used to inspect whether a sample includes a target substance, and a second main surface that may contact the sample to provide the reagent to the sample. The patch 200 may be spaced downwards with respect to the storage medium at a predetermined distance. The patch 200 may be moved upwards to the storage medium 100 such that the first main surface may receive the reagent.

The patch 200 may be spaced upwards with respect to the plate container at a predetermined distance. The patch 200 may be moved downwards to the plate container 300. The patch 200 may be moved towards the plate such that the second main surface may provide the reagent to a plate contained in the plate container 300.

The contacting of the patch 200 with the sample may be contacting via the storage medium 100. The patch 200 may be spaced upwards with respect to the storage medium 100 at a predetermined distance. The patch 200 may be moved downwards such that the patch 200 may contact the storage medium 100. The patch 200 may contact the storage medium 100 to activate the reagent. The patch may be moved downwards with the storage medium 100 towards the sample and/or the plate. Alternatively, the patch 200 may be moved downwards towards the storage medium 100 that is in contact with the sample.

### Example 1 - Immunoassay

According to an embodiment of the present disclosure, the storage medium and the patch may be used to perform immunological inspection of a sample.

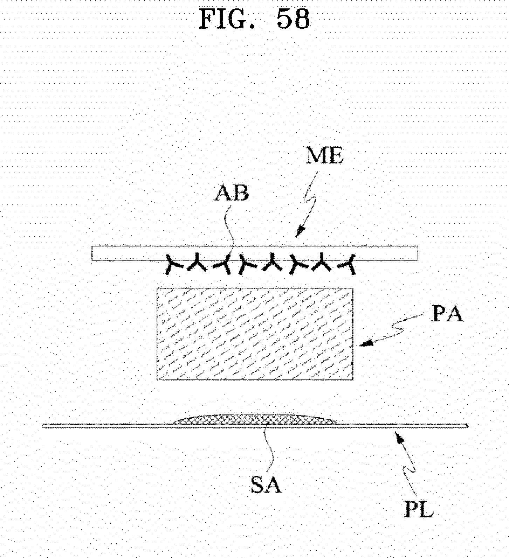

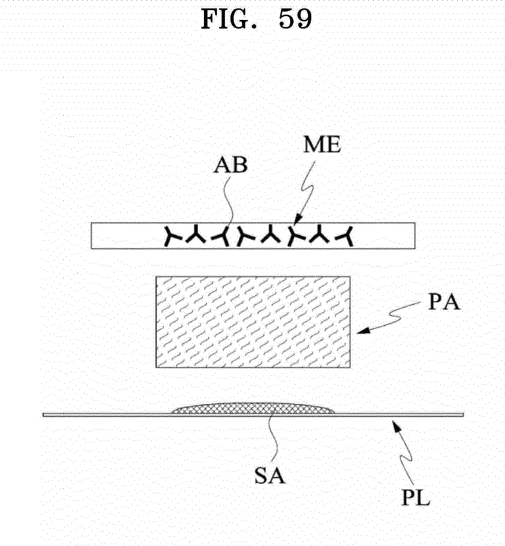

FIGS. 58 and 59 each illustrate embodiments of a module for inspecting the sample by using a storage medium and a patch. The inspection module may include a storage medium for storing an antibody, a patch, and a plate on which a sample is located. The antibody stored in the storage medium may be in a non-activated state.

As shown in FIG. 58, the inspection module may include a storage medium in which an antibody is stored in one surface, a patch, and a plate. The storage medium may store an antibody in one surface thereof towards the patch. The storage medium may store an antibody in a lower surface thereof.

The storage medium may have one surface in which a lyophilized antibody is stored.

As shown in FIG. 59, the inspection module may include a storage medium in which an antibody is stored by absorption, a patch, and a plate. The storage medium may store a lyophilized antibody in the storage medium by absorption.

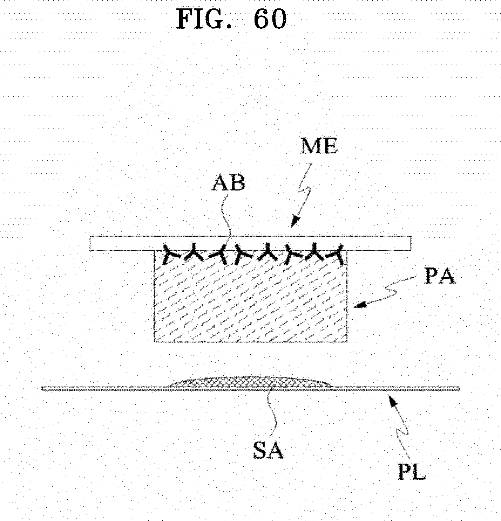

FIGS. 60 to 62 each illustrate a method of performing an immunologic inspection of a sample by using an inspection module according to the present disclosure. As shown in FIGS. 60 to 62, a target protein included in the sample may be detected by using the storage medium and the patch. The storage medium and the patch may be used to inspect the sample using an antigen-antibody reaction.

As shown in FIGS. 60 to 62, the inspection module may inspect a sample by using enzyme linked immunosorbent assay (ELISA) as immunologic inspection of the sample.

The inspection module may detect the target protein via direct ELISA. The direct ELISA may be understood as a direct type of ELISA in which an antigen is fixed onto a plate PL, and an antibody that reacts with the antigen may be directly bound to an enzyme to detect an amount of the antigen.

As shown in FIG. 60, the inspection module may transfer the antibody to the patch by contacting the storage medium with the patch. The inspection module may allow the storage medium shown in FIGS. 58 and 59 to contact the patch to transfer an antibody to the patch.

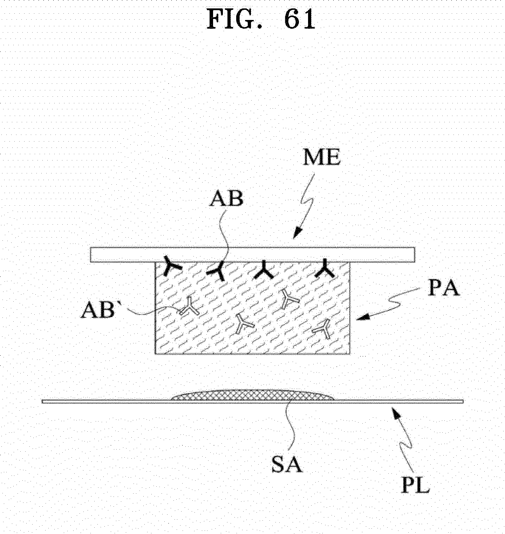

As shown in FIG. 61, the patch may accommodate the antibody in an activated state. The patch may contact the storage medium to receive and activate the antibody. The patch may store an activated antibody AB'. The patch may include a base substance and accommodate the antibody in an activated state by the base substance. The patch may include the base substance and capture the antibody by the base substance to activate the antibody.

The antibody may be moved to the patch and be activated. the antibody may contact a base substance stored in the patch and be activated. The antibody may have liquidity in the patch. The antibody may have mobility in a base substance stored in the patch.

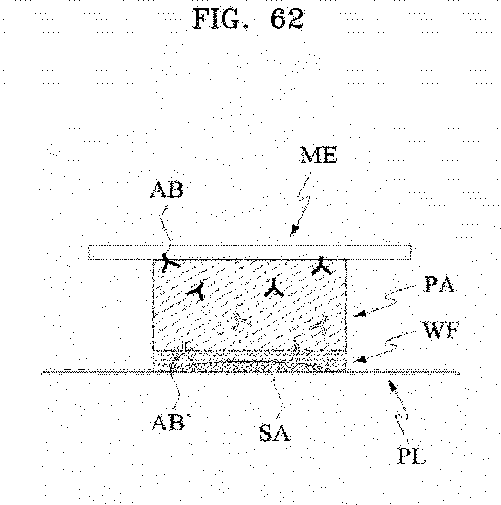

As shown in FIG. 62, the inspection module may allow the patch to contact the sample to provide the antibody to a reaction area on which the sample is located. The patch may contact the sample to form the water film WF in a reaction area on which the sample is located. The patch may provide the antibody AB' in an activated state to the reaction area on which the sample is located. The patch may store a base substance and contact the sample to form the water film WF of the base substance in the reaction area.

When the patch provides the antibody to the reaction area, the antibody may contact the sample. The antibody may form a specific binding with a target protein included in the sample. By detecting the antibody forming a specific binding with the target protein, the sample may be inspected.

Although it is not illustrated, the patch may be separated from the sample. When the patch is separated from the sample, antibodies not bound to a target protein included in the sample from among the antibodies provided to the reaction area through the water film may be absorbed by the patch. When the patch is separated from the sample, antibodies not bound to the target protein may be removed from the reaction area. Here, the antibodies that are not removed from the reaction area with the patch and remaining in the reaction area may be detected to perform diagnosis of the sample.

FIGS. 60 to 62 illustrate embodiments of an inspection module in which the storage medium contacts the patch, and the patch contacts the sample, however, the inspection method of the present disclosure is not limited thereto. In the inspection module, the patch may contact the sample in a state in which the storage medium is separated from the patch. In the inspection module, the storage medium may contact the patch in a state in which the patch is in contact with the sample.

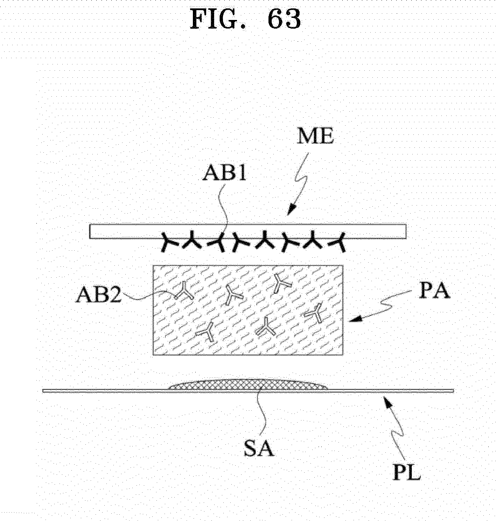

FIGS. 63 to 66 each illustrate an inspection method of the sample by detecting a target protein included in the sample via an indirect method. The indirect method is one of the methods of performing ELISA. In the indirect method of ELISA, an antigen may be fixed on to a plate, and a primary antibody reacting with the antigen and a secondary antibody bound to the primary antibody and an enzyme may be used to detect the amount of the antigen.

When the primary antibody and the secondary antibody are retained together for a long time, a non-specific binding may be formed, causing detection of a target protein by using the primary antibody and the secondary antibody difficult. In contrast, as shown in the embodiments illustrated in FIGS. 60 to 63, when the primary antibody and the secondary antibody are stored separately, the time interval from when the first antibody and the second antibody are co-located in the patch to when the first antibody and the second antibody are provided to the sample may be minimized. In other words, the first antibody and the second antibody may be located together in the patch to reduce the risk of forming non-specific bindings.

As shown in FIGS. 63 to 66, the inspection module may detect a target protein included in the sample by using the patch and the storage medium of the present specification. The inspection module may detect the target protein by using a first antibody AB1 and a second antibody AB2.

The first antibody AB1 and the second antibody AB2 may respectively be a primary antibody and a secondary antibody. The first antibody AB1 and the second antibody AB2 may respectively be a secondary antibody and a primary antibody. The first antibody AB1 may be a secondary antibody to which a marker (e.g., a fluorescent marker) is attached.

FIG. 63 illustrates the inspection module and a sample to be inspected. As shown in FIG. 63, the inspection module may include the storage medium ME storing the first antibody AB1, the patch PA storing the second antibody AB2, and the plate PL on which a sample SA to be inspected is located.

The storage medium may store the first antibody AB1 in a non-activated state. The storage medium may store the first antibody AB1 in a lyophilized state.

The storage medium may store the second antibody AB2 in an activated state. The patch may include a base substance, and the second antibody AB2 may store the base substance in a dispersed state.

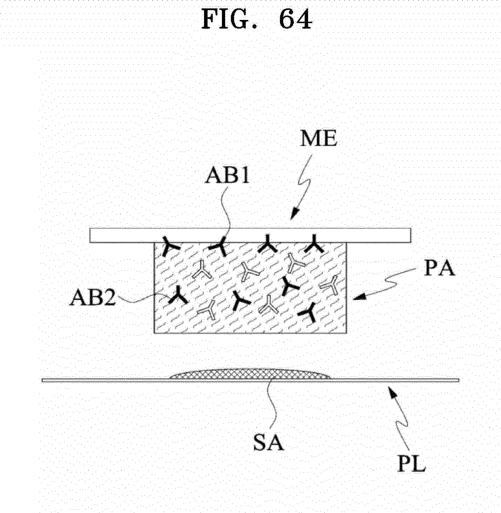

FIG. 64 illustrates the storage medium contacting the patch in the inspection module. The inspection module may transfer the first antibody AB1 to the patch by contacting the storage medium with the patch. The first antibody AB1 may be accommodated in the patch in an activated state. The first antibody AB1 may be activated by a base substance in the patch. The first antibody AB1 may have mobility in the patch.

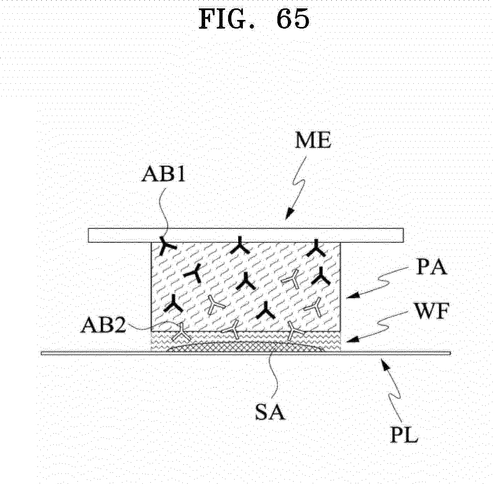

FIG. 65 illustrates approaching the patch to the sample in the inspection module. FIG. 65 illustrates approaching the patch to the sample in the inspection module such that the first antibody AB1 and the second antibody AB2 may be bound to a target protein included in the sample.

As shown in FIG. 65, in the inspection module, a patch for storing the first antibody AB1 and the second antibody AB2 in an activated state may approach to the sample. In the inspection module, as the patch contacts the sample, a base substance included in the patch may form a water film in a reaction area on which the sample is located. The first antibody AB1 and the second antibody AB2 may be dispersed in the base substance and have mobility. The first antibody AB1 and the second antibody AB2 may be provided to the sample. The first antibody AB1 and the second antibody AB2 may approach to the sample.

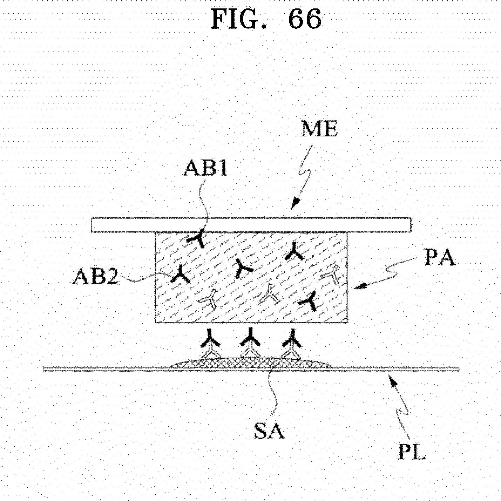

FIG. 66 illustrates separating the patch and the storage medium from the reaction area in the inspection module. As shown in FIG. 66, as the patch is separated from the reaction area in an inspection module, an antibody not bound to the target protein from among the first antibody AB1 and the second antibody AB2 may be separated along with the patch from the reaction area. From among the antibodies provided to the reaction area, a primary antibody (e.g., the second antibody AB2) specifically bound to the target protein and a secondary antibody (e.g., the first antibody AB1) specifically bound to the primary antibody bound to the target protein may not be separated along with the patch from the reaction area. The specifically binding antibodies may remain in the reaction area. The specifically binding antibodies may be detected to inspect the sample. By detecting the specifically binding antibody from the reaction area, whether the sample includes the target protein may be inspected, and the sample may be diagnosed.

According to another embodiment of the present disclosure, a first storage medium for storing a first antibody and a second storage medium for storing a second antibody may be separately prepared, and the first antibody and the second antibody may be transferred to the patch by using the first storage medium and the second storage medium. By using the patch, an antigen protein included in a sample may be detected by an indirect ELISA method. According to an embodiment, the first antibody and the second antibody may both retain in the storage medium in a non-activated state to thereby prevent denaturation of the antibodies. By separately storing the first antibody and the second antibody, a non-specific binding between the first antibody and the second antibody may be prevented.

In the foregoing embodiments, only an immunological method of diagnosing the sample is described, however, the inspection method using the storage medium and the patch may be performed in various ways.

The inspection module may inspect the sample hematologically using the storage medium and the patch. The storage medium and the patch may be used to inspect the sample using a blood staining reagent. The storage medium may store a blood staining reagent. The storage medium may provide the staining reagent to the sample along with the patch.

The inspection module may inspect the sample histologically using the storage medium and the patch. The inspection module may inspect a tissue sample. The inspection module may inspect the tissue sample by detecting the target protein contained in the tissue sample. The inspection module may stain the tissue sample to inspect the shape of the tissue sample.

The inspection module may be used to amplify specific gene sequences included in the sample. The inspection module may transfer reagents used for amplification of specific gene sequences included in the sample to the sample using the storage medium and the patch.

### Example 2 - Sequential delivery.

According to an embodiment of the present disclosure, an inspection module may be provided in which a storage medium container may be moved to transfer a substance to a patch. According to an embodiment of the present disclosure, an inspection module may be provided in which a plurality of storage media may be moved to transfer a substance to a patch.

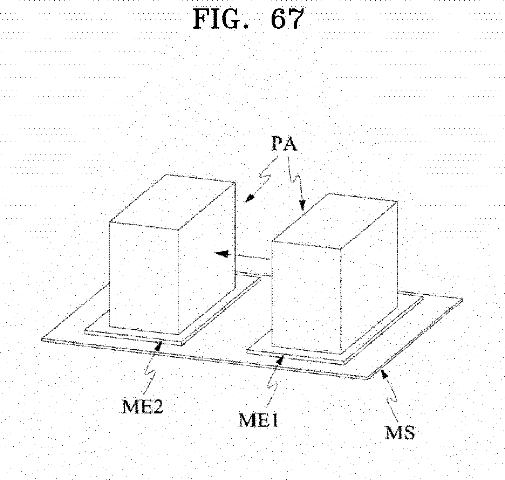

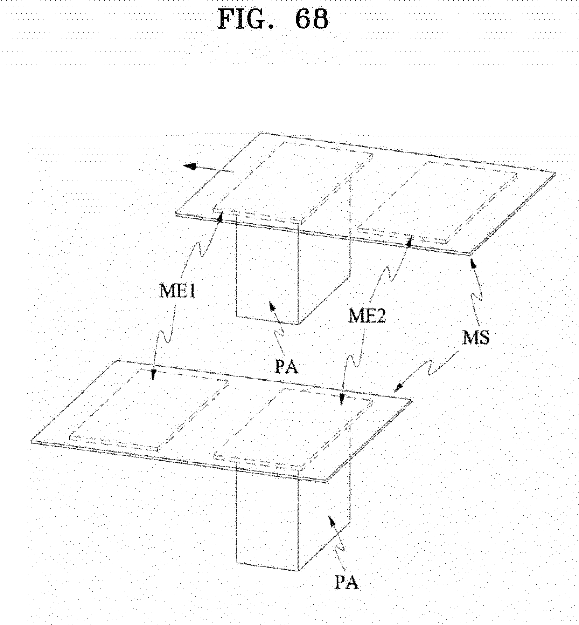

FIGS. 67 and 68 each illustrate a schematic view of embodiments of an inspection module in which a first substance and a second substance are sequentially transferred to one of the patches PA.

As shown in FIGS. 67 and 68, an inspection module according to an embodiment of the present disclosure may inspect a sample by using the patch PA, the first storage medium ME1, and the second storage medium ME2. The first storage medium ME1 and the second storage medium ME2 may respectively store a first substance and a second substance.

In the inspection module, a storage medium container MS may contain the first storage medium ME1 and the second storage medium ME2. In the inspection module, the patch PA may contact the first storage medium ME1 to transfer the first substance to the patch PA. In the inspection module, the patch PA may contact the second storage medium ME2 to transfer the second substance to the patch PA.

As shown in FIG. 67, in the inspection module, the patch PA may contact the first storage medium ME1, the patch PA may be separated from the first storage medium ME1, and the patch PA may contact the second storage medium ME2. In the inspection module, the patch PA may be moved to a first direction (e.g., a horizontal direction), in which the first storage medium ME1 and the second storage medium ME2 are arranged, and the first substance and the second substance may be sequentially transferred to the patch PA In the inspection module, the patch PA may be moved from a first location in which the first storage medium ME1 is located to a second location in which the second storage medium ME2 is located. The moving of the patch PA in the first direction by the inspection module may be a relative movement of the patch PA with respect to the storage medium container MS. The moving of the patch PA by the inspection module may include moving of the patch PA in a vertical direction to increase a gap between the patch PA and the storage medium.

As described above, the inspection module has been described with a case where the patch PA is contacted with the first storage medium ME1 and the second storage medium ME2, however, the inspection module according to the present disclosure is not limited thereto.

For example, as shown in the embodiments of the inspection module of FIGS. 47 to 49, the patch PA may be operated to sequentially contact the storage medium and the sample. In particular, the inspection module according to an embodiment of the present disclosure may be implemented in which a reaction area is located in a location of the second storage medium ME2 shown in FIG. 67. The inspection module may absorb the reagent by contacting one surface of the patch PA with the storage medium for a predetermined time. In the inspection module, the patch PA may be separated from the storage medium, and one surface of the patch may be contacted with asample to be inspected. In other words, the inspection module may receive the reagent from the storage medium by using the patch PA to provide the reagent to a reaction area.

As shown in FIG. 68, in the inspection module, the storage medium container MS may be moved in a first direction. In the inspection module, the storage medium container MS may be moved in a first direction (e.g., a horizontal direction), which is the opposite direction in which the first storage medium ME1 and the second storage medium ME2 are arranged to allow the storage medium container MS to contact the patch PA. In the inspection module, the storage medium container MS may be moved in a first direction and a second direction (e.g., vertical direction) orthogonal to first direction to transfer the first substance and the second substance to the patch PA.

In the inspection module, the storage medium container MS may be moved in a first direction to transfer the first substance and second substance to the patch PA. The moving of the storage medium container MS in the first direction by the inspection module may be a relative movement of the storage medium container MS with respect to the patch PA.

As shown in FIGS. 67 and 68, the storage medium container MS includes only the first storage medium ME1 and the second storage medium ME2. However, even when the storage medium container includes only one storage medium, the foregoing movement of the patch PA or the medium container MS may be similarly applied. The inspection module may include a first mode to place the storage medium container MS such that the storage medium does not contact the patch PA and a second mode to place the storage medium container MS such that the storage medium contacts the patch PA. In the inspection module, the mode of the storage medium container MS may be changed from the first mode to the second mode to transfer the substance to the patch PA.

### Inspection device

The inspection module disclosed in the present specification may be provided as a part of an inspection device. Hereinafter, an inspection device including the inspection module disclosed in the present specification will be described.

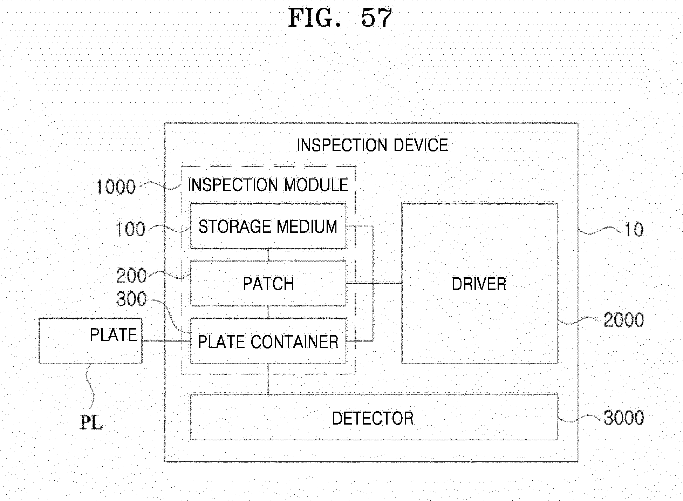

FIG. 57 illustrates an embodiment of an inspection device 10 according to the present disclosure; As shown in FIG. 54, the inspection device 10 according to an embodiment of the present disclosure may include an inspection module 1000, a driver 2000, and a detector 3000. The inspection module included in the inspection device shown in FIG. 57 may be understood similarly with the inspection module shown in FIG. 53. The inspection device 10 may inspect a sample located on plate PL contained in the plate container 300.

The driver 2000 may drive the inspection module. The driver 2000 may drive the storage medium 100, the patch 200, and the plate container 300 included in the inspection module. The driver 2000 may drive the storage medium 100 such that the patch 200 contacts the storage medium 100. The driver 2000 may drive the patch 200 such that the patch 200 contacts a sample located on the plate PL. The driver 2000 may separate the patch 200 and/or storage medium 100 from the sample.

The detector 3000 may detect a reaction for inspecting the sample. The detector 3000 may detect a reaction performed on the plate PL. The detector 3000 may detect a reaction performed on the patch 200.

The detector 3000 may be provided with a luminometer, a complementary metal-oxide semiconductor (CMOS) camera, or a charged coupled device (CCD) camera and may measure emission emitted from the reaction area.

The detector 3000 may detect the reaction by measuring the emission from the reaction area.

The detector 3000 may include a fluorometer to which a filter is attached. The detector 3000 may detect a reaction for inspecting the sample by measuring fluorescence of the reaction area on which the sample is located.

The detector 3000 may include an electrochemical sensor. The detector 3000 may detect an electrochemical change due to a reaction for inspecting the sample.

The detector 3000 may be provided with a spectrophotometer and quantify color formation of the reaction area. The detector 3000 may detect the reaction by comparing a color of the reaction area. The detector 3000 may measure absorbance to detect the reaction.

The detector 3000 may include an imaging module. The detector 3000 may optically detect a reaction for inspecting the sample. The detector 3000 may be provided with an imaging module and photograph the reaction area. The detector 3000 may inspect the sample using the photographed image.

The inspection device may be provided in a shape of a diagnosis kit.

### Experimental Example

Hereinafter, some embodiments for inspecting samples using the patch and the storage medium disclosed herein are described. Hereinafter, Comparative Example 1 will be described as an example of inspecting a sample using the patch described in the present specification, and Experimental Examples 1 to 5 will be described as examples of inspecting a sample using the patch and the storage medium described in the present specification.

The following examples illustrate the use of an antigen-antibody reaction on blood samples or the inspection of samples using a reagent that selectively stains blood components. However, the inspection method described in the present disclosure is not limited thereto.

### Comparative Example 1

According to one example of the present disclosure, a patch may be used to inspect whether a sample fixed on a plate includes a target protein.

The patch may be an agarose gel patch. The patch may store a PBS buffer solution. The patch may store an antibody that specifically binding to a target protein. The antibody may be an antibody for detecting a surface protein of a target cell (e.g., an immune cell). The substance may be an antibody for detecting CD4. The antibody may be a fluorescence-labeled antibody.

In the foregoing example, inspecting of the sample using the patch may include contacting the patch with a plate fixed with the blood sample and separating the patch from the plate. The inspecting of a sample may include detecting the antibody from the plate separated from the patch.

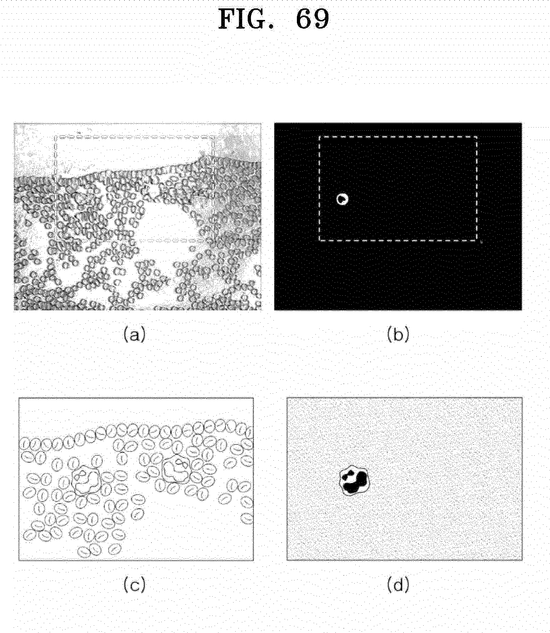

FIG. 69 shows, with reference to the above example, an experimental result according to the first Comparative Example when inspecting a blood sample fixed on a plate.

FIG. 69 (a) shows an image of a blood sample photographed using an optical microscope. FIG. 69 (b) shows an area within the dotted line in FIG. 69 (a). As shown in FIGS. 69 (a) and 69 (b), the blood sample contained red blood cells and white blood cells.

According to the first Comparative Example, the blood sample may be inspected using a patch produced with a 2 % agarose solution. The patch may store a PBS buffer and a CD4 antibody. According to the first Comparative Example, the patch may be prepared to include the 2 % agarose solution, the PBS buffer, and a CD 4antibody.

According to the first Comparative Example, the CD4 antibody may be labeled with fluorescein isothiocyanate (FITC).

According to the first Comparative Example, the blood sample may be fixed on the plate using methanol.

According to the first Comparative Example, the patch may be contacted for a period of time with a plate on which the blood sample is fixed and separated therefrom. The patch may be contacted with the plate for about 15 minutes. The patch may be separated from the plate after 15 minutes of contact with the plate.

FIG. 69 (c) shows an image obtained by imaging the same blood sample shown in FIG. 69 (a) using a fluorescence image. FIG. 69 (d) shows an area within the dotted line in FIG. 69 (c). As shown in FIGS. 69 (c) and 69 (d),

in particular, FIGS. 69 (c) and 69 (d) show images obtained by imaging the plate using a fluorescence microscope after contacting the blood sample with the patch and separating the blood sample from the patch. As shown in FIGS. 69 (c) and 69 (d), leukocytes included in the blood sample were selectively detected. That is, according to Comparative Example 1, a leukocyte including a CD4 protein among leukocytes included in a blood sample may be selectively detected. As shown in FIGS. 69 (c) and 69 (d), by detecting the FITC of the antibody bound to the CD4 protein distributed on the surface of the leukocyte, leukocytes contained in the sample may be deteced.

In case of inspecting a sample using the patch as in Comparative Example 1, the procedure may be greatly simplified, and the time required may be saved compared to detecting antigens included in the sample in a conventional manner. In particular, as in the case of Comparative Example 1, when the target protein contained in the sample is detected using a patch, the specific binding reaction of the antibody is sufficiently induced without washing or drying the sample. In addition, it is possible to receive a reaction result of the antibody within a short time (for example, within 15 minutes) than the normal inspection time by performing the process of contacting the blood sample with the patch and separating the blood sample from the patch without the process described above.

### Experimental Example 1

According to one example of the present disclosure, a patch including an antibody may be used to inspect whether a sample fixed on a plate includes a target protein. Here, the patch may be prepared without the antibody being stored.

The patch may be contacted with a liquid containing the antibody to absorb and store the antibody. The patch may be contacted with a plated coated with a liquid containing the antibody to absorb and store the antibody. As shown in an example of the present disclosure of FIGS. 47 to 49, the patch may absorb the antibody by contacting the plate with a coated reagent (i.e., a liquid containing the antibody). An enzyme may be attached to the antibody.

According to the present example, the sample may be inspected using a patch that stores a substrate for the enzyme attached to the antibody.

According to the present example, the inspecting of the sample may include contacting the patch that stores the antibody with the sample and separating the patch from the sample. The inspecting of the sample may include contacting the patch that stores the substrate with the sample and separating the patch from the sample. The inspecting of the sample may include contacting one surface that absorbed an antibody by contacting with the liquid of the patch with the sample and separating the one surface from the sample. The inspecting of the sample may include observing color formation result from a product produced from a reaction between the substrate and the enzyme.

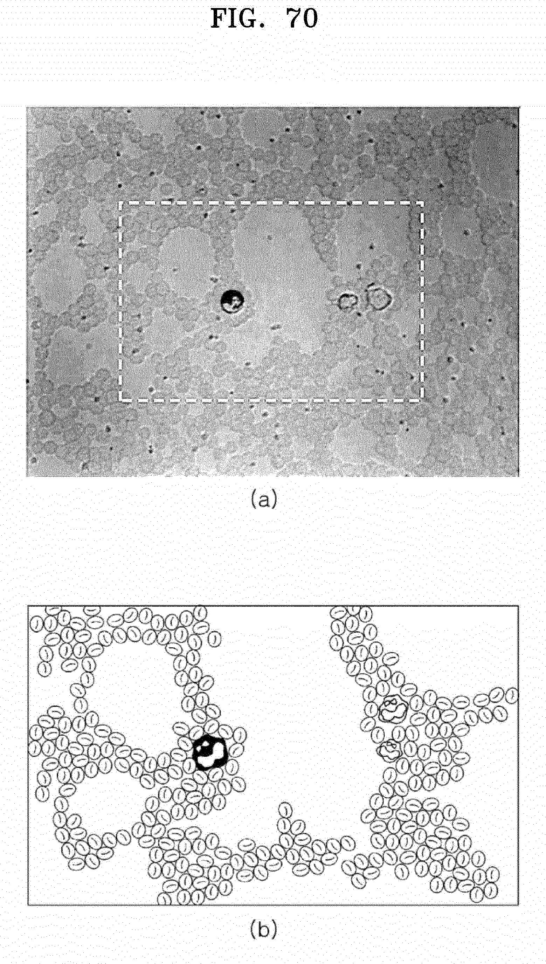

FIG. 70 (a) shows, with reference to the above example, an experimental result according to the first Experimental Example when inspecting a blood sample fixed on a plate. In particular, FIG. 70 (a) shows an image of a blood sample observed using an optical microscope when detecting a T lymphocyte by using a first patch for storing a CD4 antibody to which HRP is binding and a second patch for storing a 3,3-diaminobenzidine (DAB) solution.

FIG. 70 (b) shows an area within the dotted line in FIG. 70 (a).

In the first Experimental Example, as shown in FIGS. 70 (a) and 70 (b), CD4+ leukocytes may be selectively detected from among the leukocytes included in the blood sample. In the first Experimental Example, the first patch for storing a CD4 antibody to which HRP is binding was contacted with the sample and separated therefrom, and a DAB substrate was transferred to the sample by using the second patch for storing a 3,3-diaminobenzidine (DAB) solution to detect a T lymphocyte included in the sample.

In the first Experimental Example, the inspecting of blood may be performed by preparing the first patch and the second patch, contacting the first patch with the plate and separating the first patch from the plate, and contacting the second patch with the plate and separating the second patch from the plate.

According to the first Experimental Example, the inspecting of blood may include preparing a hydrogel patch containing a 2 % agarose solution and a PBS solution. The hydrogel patch may further contain Triton X-100.

The inspecting of blood may include preparing a first patch for storing the antibody by contacting one surface of the hydrogel patch with a PBS solution including a CD4 antibody bound to HRP. Here, in the first Experimental Example, one surface of the patch may be contacted with the solution including the antibody for absorption thereof. The first patch may contact the plate to provide the antibody to the sample. The first patch may be separated from the plate.

The inspecting of blood may include preparing a first patch for storing the antibody by contacting one surface of the hydrogel patch with a dried plate coated with a PBS solution including a CD4 antibody bound to HRP. In other words, the first patch may be prepared similarly with the embodiment shown in FIG. 47 of the present disclosure.

A second patch for storing DAB, a chromogenic substrate for the HRP, may be prepared.

The second patch may be prepared by storing the DAB solution in the patch by contacting the DAB solution with the hydrogel patch described above. The second patch may be prepared by storing the DAB solution in the patch by contacting a dried plate coated with a DAB solution with the hydrogel patch described above. Here, one surface of the patch in the first Experimental Example may contact the DAB for one minute to absorb the DAB.

The second patch may contact the plate (for example, for 10 minutes) to provide the substrate to the sample.

According to Experimental Example 1, due to the provision of the sample, the DAB may produce a product by a reaction of an enzyme attached to the antibody with the DAB. According to Experimental Example 1, inspection of the sample may be performed by observing the target lymphocytes expressed by the product.

As in Experimental Example 1, one surface of the hydrogel patch may be used to absorb the antibody, and the sample may be contacted with the one surface. The patch may provide the antibody or the like to a sample. As in Experimental Example 1, a reaction time required for inspecting the sample may be shortened by providing the antibody by contacting the one surface of the patch that absorbs the antibody to the sample.

By performing inspection of a sample as in Experimental Example 1, a pre-processing process (e.g., a blocking process) and/or a washing process of the sample may be omitted. In this case, a signal for detecting a target without a background signal was properly received.

As in Experimental Example 1, when inspecting a sample, by contacting a patch with the sample and then separating the patch therefrom, an antibody, which is not bound to a target, may be separated from the sample together with the patch such that the sample may be inspected without any additional procedure such as washing.

### Experimental Example 2

According to an embodiment of the present disclosure, a blood sample may be inspected by using the patch and the storage medium. Here, the patch may be prepared without the antibody being stored. The patch may contact the storage medium for storing the antibody to absorb the antibody. An enzyme may be attached to the antibody.

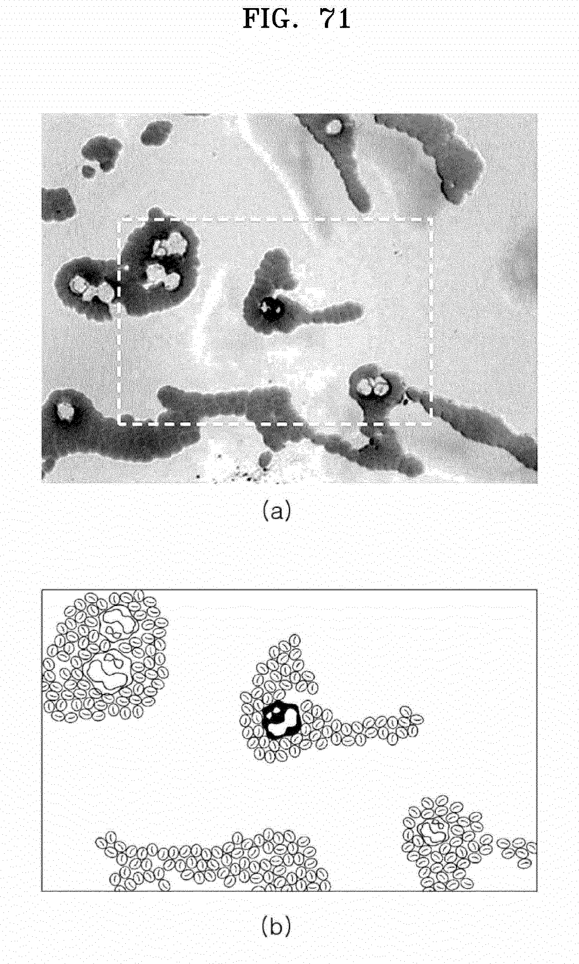

FIG. 71 shows, with reference to the above example, an experimental result according to the second Experimental Example when inspecting a blood sample fixed on a plate. In particular, FIG. 71 (a) shows an image of the sample photographed using an optical microscope after contacting a storage medium with a patch, contacting the patch with a blood sample, and coating the blood sample with a DAB solution. FIG. 71 (b) shows an area within the dotted line in FIG. 71 (a). In the second Experimental Example, as shown in FIGS. 71 (a) and 71 (b), leukocytes including a target protein may be selectively detected from among the leukocytes included in the blood sample.

In the second Experimental Example, the storage medium may store antibodies that are air-dried to detect the target protein contained in the blood sample.

In the second Experimental Example, one surface of the patch may be contacted with the sample, and the other surface of the patch may be contacted with the storage medium to provide the antibody to the sample via the patch. In particular, in the second Experimental Example, the inspecting of the blood may be performed by contacting a lower surface of a hydrogel patch with a blood sample, contacting an upper surface of the patch with a storage medium for storing an antibody, separating the patch and the storage medium from the sample, and coating the sample with a DAB solution.

The patch may contact the sample and then contact the storage medium, or the patch may contact the storage medium, and then contact the sample.

In the second Experimental Example, the patch may be made using a PBS solution containing a 2 % agarose solution. The patch may further contain Triton X-100.

In the second Experimental Example, the storage medium may be a nitrocellulose (NC membrane. The storage medium may store the CD4 antibody to which HRP is attached in an air-dried state. For example, the storage medium may store a certain amount (e.g., 50 µL to 100 µL) of an CD4 antibody solution (1:100 to 1:1000) in a dried state.

The storage medium may store the antibody on a surface thereof. the storage medium may absorb the antibody and store the antibody in a dried state.

In the second Experimental Example, the storage medium may contact an upper surface of the patch, and the CD4 antibody may be transferred to the patch. The patch may remain in contact with the storage medium and the sample for a period of time. For example, the patch may contact the storage medium and the sample at 37 degrees for 10 minutes.

In the second Experimental Example, the storage medium and the patch may be separated from the sample, and the sample may be treated with a DAB solution to observe the sample for inspection of the sample.

In the second Experimental Example, the storage medium may store the antibody, and the antibody may be provided to the sample via the patch, thereby reducing a reaction time for detecting the target protein included in the sample. In addition, as in the second Experimental Example, the antibody may be stored in an air-dried state to minimize deterioration of the antibody and extend a shelf life. In addition, as in the second Experimental Example, the storage medium may store the antibody, and the antibody may be provided to the sample via the patch, thereby easily receiving the detection result of the target protein without treatments such as washing or blocking.

### Experimental Example 3

According to an embodiment of the present disclosure, a blood sample may be inspected by using the patch and the storage medium. Here, the patch may be prepared without the antibody being stored. The patch may contact the storage medium for storing the antibody to absorb the antibody. A fluorescence-labeled substance may be attached to the antibody.

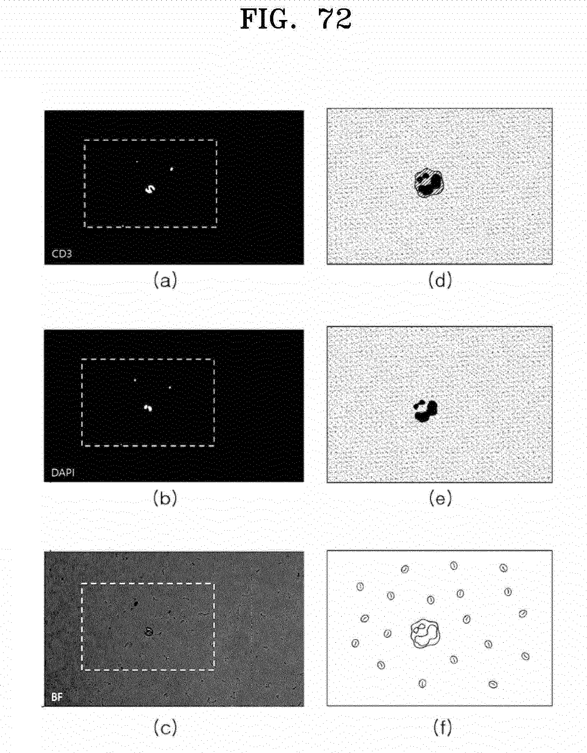

FIG. 72 shows, with reference to the above example, an experimental result according to the third Experimental Example when inspecting a blood sample fixed on a plate. FIG. 72 (a) shows a first fluorescence image of the blood sample photographed using a first fluorescence filter for detecting the fluorescence-labeled antibody. In particular, FIG. 72 (a) shows a photographed image of a fluorescence sample treated with a first fluorescence (e.g., Alexa Fluor 594) and a second fluorescence (e.g., DAPI) using a fluorescence filter for detecting a first fluorescence according to the third Experimental Example. FIG. 72 (d) shows an area within the dotted line in FIG. 72 (a).

FIG. 72 (b) shows a second fluorescence image of the blood sample photographed using a second fluorescence filter for detecting the fluorescence-labeled antibody.

In particular, FIG. 72 (b) shows a photographed image of a fluorescence sample treated with a first fluorescence (e.g., Alexa Fluor 594) and a second fluorescence (e.g., DAPI) using a fluorescence filter for detecting a ssecond fluorescence according to the third Experimental Example. FIG. 72 (e) shows an area within the dotted line in FIG. 72 (b).

FIG. 72 (c) shows an image of the blood sample photographed using an optical microscope. FIG. 72 (f) shows an area within the dotted line in FIG. 72 (c).

As shown in FIGS. 72 (a) to 72 (f), in Experimental Example 3, leukocytes included in the blood sample were selectively detected. In Experimental Example 3, nucleuses of leukocytes included in the blood sample were selectively detected.

The experimental procedure according to the third Experimental Example may be implemented substantially similarly to those of the second Experimental Example described above.

According to the third Experimental Example, the storage medium may store the CD3 antibody to detect CD3. The storage medium may store a CD3 antibody to detect a leukocyte that contains a CD3 protein. In particular, the storage medium may absorb a certain amount (e.g., 50 µL to 100 µL) of an CD4 antibody solution (1:100 to 1:1000) in a dried state and store the antibody in a dried state.

A fluorescent substance may be labeled to the antibody.

The fluorescent substance may be Alexa Flour 594.

The fluorescent substance may be 4',6-diamidino-2-phenylindole (DAPI).

In the third Experimental Example, as in the second Experimental Example, the storage medium may be contacted with one surface of the patch, and the other surface of the patch may be contacted with the sample to inspect the sample by using the antibody.

As in the third Experimental Example, when a fluorescence-labeled antibody is separately retained in the storage medium, as compared with a case where an antibody is stored together with the patch to retain or transport, the quality deterioration of the antibody may be alleviated, and deterioration of the fluorescence may be prevented.

### Experimental Example 4

According to an embodiment of the present disclosure, a blood sample may be inspected by using the patch and the storage medium for storing a fluorescent reagent. Here, the patch may be prepared without the fluorescent reagent being stored. The patch may contact the storage medium for storing the fluorescent reagent to absorb the fluorescent reagent. The fluorescent reagent may be a fluorescent reagent for staining DNA.

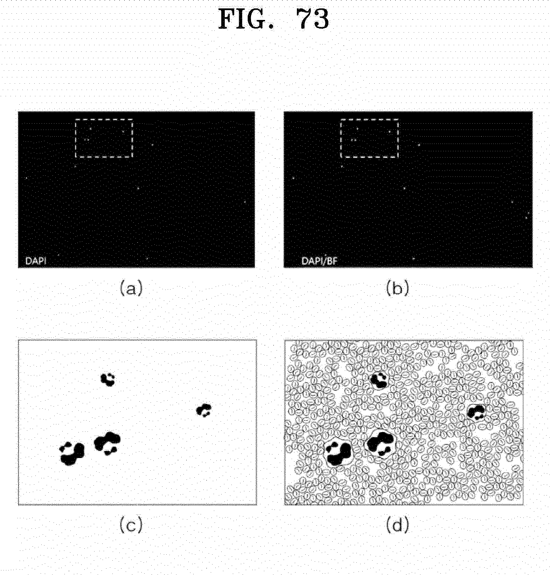

FIG. 73 shows, with reference to the above example, an experimental result according to the fourth Experimental Example when inspecting a blood sample fixed on a plate. In particular, FIG. 73 shows an experimental result according to the fourth Experimental Example for detecting a genetic substance included in a blood sample.

In the fourth Experimental Example, the storage medium may store a DAPI reagent. The storage medium may absorb a PBS solution including a certain amount (50 µL to 100 µL) of DAPI, and the DAPI reagent may be stored in a dried state.

In the fourth Experimental Example, the inspecting of the blood sample may be performed by contacting the storage medium for storing the DAPI reagent with one surface of the patch, contacting the other surface of the patch with the sample, and separating the storage medium and the sample from the patch. When the storage medium for storing the DAPI reagent is contacted with one surface of the patch, the DAPI reagent may be transferred to the patch. The patch for storing the DAPI reagent may provide the DAPI reagent to the sample by contacting with the sample. When the patch for storing the DAPI reagent is separated from the sample, the reagent not bound to DNA included in the sample may be separated from the sample along with the patch.

In the fourth Experimental Example, after separating the patch for storing the DAPI reagent from the sample, the sample may be inspected by detecting fluorescence of the plate on which the sample is located.

As in the fourth Experimental Example, by storing and transferring the fluorescent reagent using the storage medium and transferring the fluorescent reagent to the sample via the patch, quality deterioration of the fluorescent reagent may be prevented, and a shelf life thereof may be extended.

FIG. 73 (a) shows a fluorescent image of the blood sample photographed using a fluorescent filter for detecting a genetic substance included in the blood sample, according to the fourth Experimental Example. In particular, FIG. 73 (a) is a fluorescent image of the blood sample fluorescence-treated using the storage medium for storing the foregoing DAPI reagent and a hydrogel patch. FIG. 73 (a) is a fluorescent image of the blood sample after contacting a lower surface of the patch with the sample, culturing for 3 to 5 minutes at room temperature while an upper surface of the patch is in contact with the storage medium, and separating the patch from the sample. FIG. 73 (c) shows an area within the dotted line in FIG. 73 (a).

FIG. 73 (b) shows an image of the blood sample photographed using an optical microscope to confirm the experimental result according to the fourth Experimental Example. In particular, FIG. 73 (b) is a bright field image of the blood sample fluorescence-treated using the storage medium for storing the foregoing DAPI reagent and a hydrogel patch. FIG. 73 (d) shows an area within the dotted line in FIG. 73 (b).

As shown in FIGS. 73 (a) to 73 (d), in Experimental Example 4, nucleuses of leukocytes included in the blood sample were selectively detected.

### Experimental Example 5

According to an embodiment of the present disclosure, a blood sample may be inspected by using the patch and the storage medium for storing a fluorescent staining substance. Here, the patch may be prepared without the staining substance being stored. The patch may contact the storage medium for storing the fluorescent reagent to absorb the fluorescent staining substance.

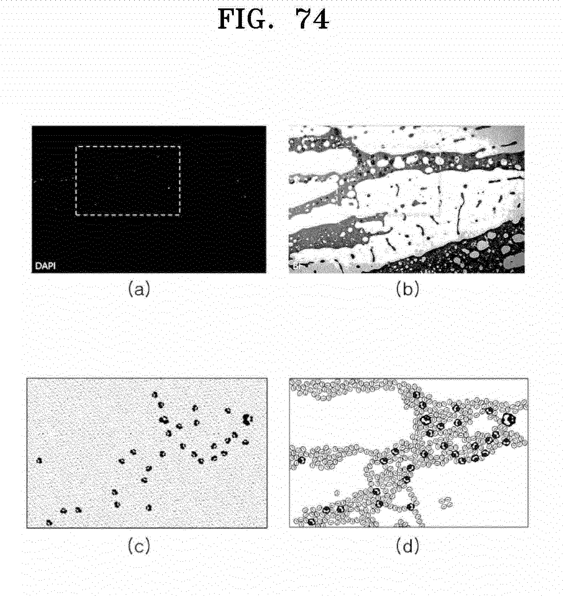

FIG. 74 shows, with reference to the above example, an experimental result according to the fifth Experimental Example when inspecting a blood sample fixed on a plate. The experimental procedure according to the fifth Experimental Example may be implemented substantially similarly to those of the second Experimental Example described above. However, in the fifth Experimental Example, the patch may absorb a DAPI staining reagent from the storage medium. Also, according to the fifth Experimental Example, the blood sample may be subjected to color staining first, followed by fluorescence staining.

FIG. 74 (a) shows an image of a blood sample photographed using a fluorescence filter, in which the blood sample is obtained in Experimental Example 5. In particular, FIG. 74 (a) shows an image photographed using a fluorescent filter for detecting a target protein included in the blood sample, according to the fifth Experimental Example. FIG. 74 (c) shows an area within the dotted line in FIG. 74(a).

FIG. 74 (b) shows an image of a blood sample photographed using an optical microscope, in which the blood sample is obtained in Experimental Example 5. FIG. 74 (d) shows an image of a blood sample photographed using a fluorescence filter, in which the blood sample is obtained in Experimental Example 5.

As shown in FIGS. 74 (a) to 74 (d), in Experimental Example 5, DNA included in the blood sample were selectively detected.

In the fifth Experimental Example, by using the patch and the storage medium according to the present specification, a blood sample was color-stained first and fluorescence-stained. Accordingly, color and fluorescence staining results were observed in one plate at the same time.

### Review

Through the first Comparative Example and the first to fifth Experimental Examples, it was found that the sample may be inspected using the patch and/or the storage medium disclosed in the present specification. In other words, it was found that the sample may be inspected using general staining, fluorescence staining, and antigen-antibody reaction using the patch and/or the storage medium described in the present specification.

Also, when comparing the Comparative Example with Experimental Examples, retaining the staining reagent or antibody in the storage medium in Experimental Examples improved retention of the reagent and prolonged the quality maintenance period was extended. Also, it was found that the time required to perform the inspection does not increase or may be shortened as compared with the case of the Comparative Examples.

The above description is merely illustrative of the technical idea of the present disclosure. It will be understood by one of ordinary skill in the art to which the present disclosure pertains that various changes or modification may be made therein without departing from the spirit of the present disclosure. Accordingly, embodiments of the present disclosure described above may be implemented separately or in combination. Accordingly, the embodiments disclosed in the present disclosure do not limit the scope of the technical idea of the present disclosure. Therefore, the scope of the disclosure sought to be protected is defined by the appended claims, and all technical ideas within the scope will be construed as being included in the present disclosure.

### MODE OF DISCLOSURE

[As described above, the relevant matters have been described in the BEST MODE.

### INDUSTRIAL APPLICABILITY

As described above, in the present disclosure, a storage medium for storing a substance may be fully or partially applied to an inspection method or an inspection module.

## Claims

1. A method of inspecting a sample, the inspection method comprising:
preparing a storage medium for storing a reagent in a non-activated state, wherein the reagent is used to inspect whether the sample comprises a target substance;
preparing a patch on a side of the storage medium, the patch comprising a network structure in which multiple microcavities are formed and a base substance stored in the microcavities, and imparting an active condition to the reagent;
contacting the storage medium with a surface of the patch on the side of the storage medium for transferring a portion of the reagent stored in the storage medium to the patch;
accommodating, in the patch, the transferred reagent put into an activated state by the base substance; and
contacting the patch with the sample to provide the reagent to a reaction area on which the sample is located.

2. The method of claim 1, further comprising: providing the reagent to a reaction area, on which the sample is located,
and the providing of the reagent comprises transferring the reagent to the patch in contact with the sample.

3. The method of claim 1, wherein the accommodating of the transferred reagent put into an activated state is **characterized by** storing the reagent put into an activated state in the patch in contact with the sample.

4. The method of claim 1, wherein the contacting of the patch with the sample is **characterized by** contacting the patch accommodating the reagent put into an activated state with the sample.

5. The method of claim 1, wherein the contacting of the patch with the sample comprises contacting the surface of the patch in contact with the storage medium and another surface of the patch with the sample.

6. The method of claim 1, wherein the contacting of the patch with the sample comprises contacting the surface of the patch in contact with the storage medium with the sample.

7. The method of claim 1, wherein the reagent is lyophilized in the storage medium.

8. The method of claim 1, wherein the providing of an active condition for the reagent comprises contacting the reagent with a base substance to liquefy the reagent.

9. The method of claim 1, wherein the activated state of the reagent is a state in which fine particles comprised in the reagent have mobility.

10. The method of claim 1, wherein the providing of an active condition for the reagent comprises contacting the reagent with a base substance to impart mobility to fine particles comprised in the reagent.

11. The method of claim 1, wherein the providing of an active condition for the reagent comprises providing a pH condition for the reagent to inspect the target substance.

12. The method of claim 1, wherein the reagent comprises an antibody for detecting a target protein, and the antibody is stored in a solid state in the storage medium.

13. The method of claim 1, wherein the transferring of the reagent to the patch comprises transferring the reagent to a base substance comprised in the patch.

14. The method of claim 1, wherein the contacting of the patch with the sample comprises indirectly contacting the patch with the sample through the storage medium in contact with the sample.

15. The method of claim 1, wherein the sample is a biological sample for diagnosing a target disease.

16. A method of inspecting a sample, the inspection method comprising:
preparing a storage medium for storing a reagent in a non-activated state, wherein the reagent is used to inspect whether the sample comprises a target substance;
preparing a patch on a side of the storage medium, the patch comprising a network structure in which multiple microcavities are formed and a base substance imparting an active condition to the reagent;
activating the reagent by contacting one surface of the storage medium with a surface of the patch on the side of the storage medium; and
contacting another surface of the storage medium on a side of the sample with the sample to provide the reagent to a reaction area on which the sample is located.

17. The method of claim 16, further comprising:
providing the reagent to a reaction area on which the sample is located, wherein the reagent is **characterized by** being provided to the reaction area in an activated state.

18. The method of claim 17, wherein the providing of the reagent to the reaction area comprises providing, to the reaction area, the reagent put into an activated state by the base substance.

19. The method of claim 16, wherein the activating of the reagent comprises activating the reagent stored in the storage medium of which the other surface is in contact with the sample.

20. The method of claim 16, wherein when the one surface of the patch is in contact with the storage medium, a base substance comprised in the patch and transferred to the storage medium through the one surface of the patch induces the activated reagent to be provided to the sample.

21. The method of claim 16, wherein the providing of the reagent to a reaction area on which the sample is located comprises inducing provision of the reagent by transferring the base substance to the storage medium, upon contact of one surface of the storage medium with the patch.

22. The method of claim 16, wherein the contacting of the storage medium with the sample comprises contacting, with the sample, the storage medium of which the one surface is in contact with the patch.

23. The method of claim 16, wherein the reagent comprises an antibody for detecting a target protein.

24. The method of claim 17, wherein the reagent is lyophilized in the storage medium.

25. A method of inspecting a sample, wherein a storage medium is contacted with a patch for storing a substance to transfer a substance stored in the storage medium to the patch, the patch imparts an active condition to the substance transferred to the patch, and the substance is provided to a reaction area, the inspection method using a patch and a storage medium and comprising:
preparing a patch comprising a base substance that activates a reagent and a network structure in which multiple microcavities are formed, wherein the multiple microcavities store the base substance, and the patch being capable of absorbing a substance from an external area or providing a substance to an external area;
preparing a storage medium for storing a reagent in a non-activated state, wherein the reagent is used to inspect whether the sample comprises a target substance;
contacting the patch with the storage medium to transfer the reagent to the patch;
accommodating, in the patch, the reagent put into an activated state by the base substance; and
contacting the patch with the sample to provide the reagent to the reaction area on which the sample is located.

26. The method of claim 25, the inspection method further comprising:
the patch providing the sample to the reaction area, wherein the providing of the sample to the reaction area comprises providing, to the reaction area, the sample put into an activated state by the base substance and accommodated.

27. The method of claim 25, wherein the accommodating of the reagent put into an activated state in the patch comprises imparting fluidity to the reagent by the base substance in the patch.

28. The method of claim 25, wherein the storage medium stores the reagent that is lyophilized.

29. The method of claim 25, wherein the reagent comprises an antibody for detecting a target protein.

30. A gel patch for inspecting a sample, the patch comprising:
a base substance imparting an active condition to a reagent, the base substance being for identifying whether the sample comprises a target substance;
a network structure in which multiple microcavities, the multiple microcavities storing the base substance, are formed; and
a medium contact surface that contacts a storage medium in which the reagent is stored in a non-activated state, wherein the patch receives the reagent from the storage medium, and accommodates the reagent put into an activated state by the base substance.

31. The patch of claim 30, wherein the patch further comprises a sample contact surface that contacts the sample and provides the reagent to a reaction area on which the sample is located.

32. The patch of claim 31, wherein the medium contact surface is identical to the sample contact surface.

33. The patch of claim 31, wherein the medium contact surface is arranged opposite the sample contact surface.

34. The patch of claim 31, wherein the contacting of the patch with the sample comprises indirect contacting through the storage medium.

35. The patch of claim 30, wherein the patch provides the base substance to the storage medium through the medium contact surface by contacting the storage medium, and the storage medium, by contacting the sample, provides the reagent put in an activated state by the base substance to a reaction area on which the sample is located.

36. The patch of claim 30, wherein the accommodating of the reagent put into an activated state in the patch comprises imparting activity to the reagent by contacting of the patch with the base substance.

37. A storage medium for inspection of a sample, wherein the inspection is performed using the storage medium and a patch, the patch comprises a base substance and a network structure in which multiple microcavities are formed, the multiple microcavities storing the base substance, the storage medium stores a reagent used to inspect whether the sample comprises a target substance, and comprises a storage surface storing the reagent in a non-activated state and a patch contact surface that contacts the patch for activating the reagent, and the patch activates the reagent by the base substance, upon contact with storage medium, and provides the activated reagent to a reaction area on which a sample to be inspected is located.

38. The storage medium of claim 37, wherein the storage medium stores the reagent that is lyophilized.

39. The storage medium of claim 37, wherein the reagent contacts the base substance through the patch contact surface and acquires liquidity due to the base substance.

40. The storage medium of claim 37, wherein the storage medium further comprises a sample contact surface that contacts the sample, and the providing of the activated reagent to the reaction area by the patch is providing the activated reagent to the reaction area through the sample contact surface of the storage medium.

41. An inspection module for inspecting a sample, wherein the inspection module comprises:
a storage medium for storing a reagent in a non-activated state, wherein the reagent is used to inspect whether the sample comprises a target substance;
a patch comprising a network structure in which multiple microcavities are formed and a base substance stored in the microcavities, and imparting an active condition to the reagent, wherein the patch accommodates the reagent put into an activated state upon contact with the storage medium;
and a plate container for containing a plate comprising a reaction area on which the sample is located and to which the reagent put into an activated state is provided.

42. The inspection module of claim 41, wherein the patch contacts the sample to provide the reagent put into an activated state to the reaction area.

43. The inspection module of claim 41, wherein the patch provides the reagent to the reaction area upon contact with the sample, while in contact with the storage medium.

44. The inspection module of claim 41, wherein the patch accommodates the reagent put into an activated state upon contact with the storage medium, while in contact with the sample, and provides the reagent to the reaction area.

45. The inspection module of claim 41, wherein the patch contacts the storage medium to activate the reagent and induces transfer of the activated reagent to the plate.

46. The inspection module of claim 41, wherein the storage medium provides the activated reagent to the reaction area upon contact with the sample, when the reagent is activated by contacting the storage medium with the patch.

47. The inspection module of claim 41, wherein the storage medium contacts the patch, while in contact with the sample.

48. The inspection module of claim 41, wherein the activated state of the reagent is a state in which fine particles comprised in the reagent have mobility.

49. The inspection module of claim 41, wherein the reagent comprises an antibody for detecting a target protein.

50. The inspection module of claim 41, wherein the sample is a biological sample for diagnosing a target disease.

51. An inspection module for inspecting a sample, the inspection module comprising:
a gel patch, the patch comprising a first main surface that receives a reagent, wherein the reagent is used to inspect whether a sample comprises a target substance, and a second main surface that contacts the sample to provide the reagent to the sample;
a storage medium which stores the reagent in a non-activated state, wherein the storage medium is disposed on the first main surface of the patch; and
a plate container comprising a reaction area on which the sample to be inspected is located, wherein the sample is provided to the inspection by contacting the patch, and containing a plate disposed on the second main surface of the patch, wherein the storage medium is moved toward the patch to transfer the reagent to the patch, the patch receives the reagent and activates the reagent, and the patch is moved toward the plate to provide the activated reagent to the reaction area.

52. The inspection module of claim 51, wherein the storage medium is spaced apart from the patch in a first direction by a predetermined distance, and the storage medium is moved in a second direction opposite to a first direction from the patch to transfer the reagent to the patch.

53. The inspection module of claim 51, wherein the inspection module is prepared as an inspection kit for inspecting the sample.
